(19) Europäisches Patentamt
European Patent Office
Office européen des brevets

(11) EP 4 311 559 A1

# (12) EUROPEAN PATENT APPLICATION

(43) Date of publication:
**31.01.2024 Bulletin 2024/05**

(21) Application number: **22382731.2**

(22) Date of filing: **29.07.2022**

(51) International Patent Classification (IPC):
***A61K 47/66*** *(2017.01)* ***A61K 47/69*** *(2017.01)*
***A61P 19/02*** *(2006.01)*

(52) Cooperative Patent Classification (CPC):
**A61K 47/6929; A61K 49/0002; A61K 49/006; A61K 49/04; A61K 49/06; A61K 51/00; A61P 19/02**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **4basebio UK Ltd**
  **Cambridge CB24 5QE (GB)**
- **4basebio, S.L.U.**
  **28049 Cantoblanco (Madrid) (ES)**

(72) Inventors:
- **WALKER, Amy**
  **Cambridge, CB24 5QE (GB)**
- **LANCKRIET, Heikki**
  **Cambridge, CB24 5QE (GB)**
- **PICHER, Ángel**
  **28049 Cantoblanco, Madrid (ES)**
- **HANGU, Ana Vanina**
  **Cambridge, CB24 5QE (GB)**
- **THOMPSON, William**
  **Cambridge, CB24 5QE (GB)**

(74) Representative: **Boult Wade Tennant LLP**
**Salisbury Square House**
**8 Salisbury Square**
**London EC4Y 8AP (GB)**

Remarks:
The complete document including Reference Table(s) and the Sequence Listing(s) can be downloaded from the EPO website

## (54) NANOPARTICLES AND PEPTIDES FOR THE DELIVERY OF CARGOS TO CHONDROCYTES

(57) Nanoparticles suitable for delivery of a cargo to a chondrocyte, and targeting peptides comprising a chondrocyte targeting sequence, are provided. Further provided are uses of the nanoparticles and targeting peptides, for example, in treating a joint or cartilage disease or disorder.

EP 4 311 559 A1

## Description

### TECHNICAL FIELD

The present invention relates to nanoparticles suitable for delivery of a cargo to chondrocytes. In addition, the present invention relates to targeting peptides comprising a chondrocyte targeting sequence. The present invention also relates to uses of the nanoparticles and targeting peptides, for example, in treating a joint disease or disorder.

### BACKGROUND

Articular cartilage is a smooth viscoelastic tissue designed to bear and distribute loads across the diarthrodial joints. This highly specialized tissue exhibits a unique mechanical behaviour and poor regenerative capacities. Chondrocytes in the articular cartilage proliferate and secrete extracellular matrix to maintain and sustain the cartilage. The cells themselves are separated from each other by cartilage matrix. They respond to outside stimuli and tissue damage, and are also responsible, either directly or indirectly for degenerative conditions, such as osteoarthritis, rheumatoid arthritis, Juvenile Idiopathic Arthritis (JIA), and Mucopolysaccharidosis I (MPS1).

Osteoarthritis is characterized by a progressive degradation of articular cartilage leading to loss of joint function. Although the molecular mechanisms regulating pathogenesis and progression of osteoarthritis are poorly understood, remarkably, some characteristics of this joint disease resemble chondrocyte differentiation processes during skeletal development by endochondral ossification (Dreier (2010) "Hypertrophic differentiation of chondrocytes in osteoarthritis: the developmental aspect of degenerative joint disorders"; Arthritis Research & Therapy; 12(5), 1-11). In healthy articular cartilage, chondrocytes resist proliferation and terminal differentiation. By contrast, chondrocytes in diseased cartilage progressively proliferate and develop hypertrophy. Moreover, vascularization and focal calcification of joint cartilage are initiated. Signalling molecules that regulate chondrocyte activities in both growth cartilage and permanent articular cartilage during osteoarthritis are thus interesting targets for disease-modifying osteoarthritis therapies.

In rheumatoid arthritis, multiple inflammatory mediators are present in the synovial joint. On the one hand, chondrocytes act as target cells of these inflammatory mediators, resulting in chondrocyte dysfunction (Tseng et al. (2020) "Dual Role of Chondrocytes in Rheumatoid Arthritis: The Chicken and the Egg"; International Journal of Molecular Sciences, 21(3), 1071). On the other hand, chondrocytes of rheumatoid arthritis also act as effector cells, exhibiting various alterations that directly or indirectly facilitate joint damage of rheumatoid arthritis. This dual action of chondrocytes in rheumatoid arthritis makes them an attractive target for rheumatoid arthritis therapies.

Mucopolysaccharidosis I (MPS1) is a rare autosomal recessive inherited disease, caused by deficiency of the enzyme $\alpha$-L-iduronidase, resulting in accumulation of the glycosaminoglycans (GAGs) dermatan and heparan sulfate in organs and tissues. The disease causes, amongst other things, articular chondrocyte apoptosis through accumulation of dermatan sulfate. Dermatan sulfate is structurally similar to lipopolysaccharide (LPS) and activates the TLR-4 signalling pathway, initiating the release of proinflammatory cytokines, which eventually leads to articular chondrocyte apoptosis (Hampe et al. (2020) "Mucopolysaccharidosis Type I: A Review of the Natural History and Molecular Pathology"; Cells, 9(8), 1838). Thus, targeting chondrocytes in patents with MPS1 could provide very useful in MPS-1 targeted therapies.

Adeno-associated viral vectors have been clinically adapted as an efficient tool for gene therapy in different osteoarticular disorders (Cucchiarini et al. "New trends in articular cartilage repair", 2015, Journal of experimental orthopaedics, 2(1), 1-8). Studies in rat have shown that transfection with adenoviral and lentiviral vectors can achieve efficiencies of up to 70 and 95%. However, despite all the advantages viral gene delivery can propose, these systems have been shown to induce an inflammatory response in joints, which can cause different side effects (Evans et al. (2006) "Gene therapeutic approaches - transfer in vivo"; Advanced drug delivery reviews, 58(2), 243-258). In addition, viral delivery vectors do not allow for repeated dosing which is required for treating certain diseases and disorders.

More generally although viruses as delivery agents are considered to have the advantages of high efficiency and high cell selectivity, they have several disadvantages including toxicity, risk of insertional mutagenesis, production of inflammatory responses, high likelihood of eliciting a host immune response and limited packaging capacity.

Non-viral vectors such as lipid-based nanoparticles provide a method of targeted delivery and controlled release of therapeutic agents such as drugs and nucleic acids to cells. Lipid-based nanoparticles (particularly nanoparticles comprising cationic or ionizable lipids) have been investigated as a non-viral vector for gene delivery.

Liposomes have a spherical lipid bilayer, mostly comprised of phospholipids with a hydrophilic head group and hydrophobic tail. The positively-charged head groups of cationic lipids can facilitate spontaneous electrostatic binding with negatively charged phosphate groups on DNA molecules, forming entropically favourable nanoparticles. The net charge of the nanoparticles is determined by the ratio between the free amine groups and the phosphate groups in the nanoparticle, which, in turn, affects the size, stability, and structure of the particles.

Felgner et al. ("Lipofection: a highly efficient, lipid-mediated DNA-transfection procedure" Proceedings of the National Academy of Sciences 84.21 (1987): 7413-7417) describes the application of cationic lipids as gene vectors. In

addition, there are now many commercially available, widely used lipids designed and synthesised specifically for transfection, with different structural aspects such as head group and hydrocarbon tail length. A neutral, 'helper' lipid such as DOPE (Dioleoyl L-α phosphatidyl ethanolamine) is now often formulated with cationic lipids because of its membrane stabilising properties, which are believed to aid endosomal escape. Cholesterol, an important component of biological cell membranes, can be added to increase circulation time of lipid nanoparticles by imparting stability to the complexes. Meanwhile, PEGylation of liposomal particles, (with poly(ethylene glycol)), can prevent aggregation of nanoparticles, with particles remaining small and discrete both outside and within cells, whilst conveying a stealth coating on the particles, enhancing serum stability.

WO 02/072616 A2 describes an exemplary non-viral transfection complex, which comprises: (a) a nucleic acid, (b) a lipid component, (c) a polycationic nucleic acid-binding component, and (d) a cell surface receptor binding component comprising a peptide that binds to human airway epithelial cells.

A need exists for an improved delivery system that does not suffer from the disadvantages of viral vectors and which allows for specific targeting of chondrocytes.

## DESCRIPTION

The invention provides a nanoparticle suitable for delivery of a cargo to a target cell. The invention provides a nanoparticle comprising (a) a cargo, (b) a lipid component, and (c) a targeting peptide. The targeting peptide may comprise a targeting sequence and a cargo-binding component. The nanoparticle is preferably a non-viral delivery system, such as a non-viral transfection complex. The nanoparticle is preferably a self-assembled nanoparticle. The invention preferably targets chondrocytes (i.e. cells responsible for cartilage formation). The cargo may be a nucleic acid. The cargo-binding component may be a nucleic-acid binding cationic component. The nucleic acid may have an enhanced resistance to nuclease (e.g. exonuclease) digestion. The cargo may comprise a cassette. The cassette may comprise a coding sequence. The coding sequence may encode a gene useful in treating a disease or disorder (e.g. a joint disease or disorder, such as osteoarthritis).

The present inventors have developed a nanoparticle (e.g. a non-viral transfection complex) suitable for use in treating different diseases or disorders. For example, the nanoparticle is suitable in treating a joint disease or disorder, such as osteoarthritis. The nanoparticle of the present invention has several advantages over viral delivery systems. Firstly, the nanoparticle of the present invention is less likely to elicit an immune response, which is particularly important as repeated doses may need to be administered. In addition, the nanoparticle of the present invention can be used to deliver much larger cargos to a cell (e.g. a chondrocyte).

The nanoparticles of the present invention provide further benefits in that they are cost-effective and simple to produce on a large scale.

The nanoparticles of the present invention may comprise a nucleic acid cargo (e.g. a DNA molecule) that has an enhanced resistance to nuclease (e.g. exonuclease) digestion, which results in a prolonged in vivo life of the cargo molecule.

Importantly, the nanoparticles of the present invention provide improved targeting to chondrocytes as compared to any other non-viral delivery systems.

The nanoparticles of the present invention preferentially bind to chondrocytes over other cell types.

The present inventors have also developed improved methods of producing nanoparticles in which the produced nanoparticles are more uniform as compared to nanoparticles produced by methods known in the art. This property of nanoparticles improve transfection efficiency which makes them particularly suitable for use in therapy.

### 1. Nanoparticles and non-viral transfection complexes

The invention provides a nanoparticle suitable for delivery of a cargo to a target cell. The nanoparticle comprises: (a) a cargo; (b) a lipid component; and (c) a targeting peptide. The targeting peptide may improve targeting of a nanoparticle to a desired location, for example, a chondrocyte.

The invention provides, a nanoparticle comprising:

(a) a cargo;
(b) a lipid component; and
(c) a targeting peptide comprising a chondrocyte targeting sequence.

The term "chondrocyte targeting sequence" refers to a sequence that has the ability to target, bind and/or interact with a chondrocyte. For example, the chondrocyte targeting sequence may target, bind and/or interact with a receptor on a chondrocyte, or a biologically active molecule present on the surface of the chondrocyte.

The chondrocyte targeting sequence may be a chondrocyte-specific targeting sequence. The term "specific"

refers to the ability to preferentially target, bind and/or interact with a given target, for example, a receptor on a chondrocyte, or a biologically active molecule present on the surface of the chondrocyte, over other targets. Preferential targeting, binding and/or interacting with may be assessed by measuring the binding affinity of a sequence for a target on a chondrocyte in comparison to a target on a different cell. Similarly, preferential targeting, binding and/or interacting with may be measured by calculating a dissociation rate (of a sequence from a target molecule). Preferential targeting may be assessed by comparing the amount of binding events for each one of the target cells. For example, the chondrocyte-specific targeting sequence may bind to a chondrocyte more often than any other cell type. The chondrocyte -specific targeting sequence may bind with higher affinity for a chondrocyte (or a receptor on a chondrocyte, or a biologically active molecule present on the surface of the chondrocyte) than any other cell (or receptor) type.

The nanoparticle comprising the chondrocyte-specific targeting sequence may deliver more cargo to the chondrocyte than to other cell types. For example, the nanoparticle comprising the chondrocyte-specific targeting sequence may deliver at least 5%, at least 10%, at least 20%, at least 30%, at least 40%, at least 50%, at least 60%, at least 70%, at least 80%, at least 90%, at least 100% more cargo to the chondrocyte than to other cell types. The nanoparticle comprising the chondrocyte-specific targeting sequence may deliver at least 2 times, 5 times, 10 times, at least 25, at least 50 times, at least 75 times, at least 100 times, at least 125 times, at least 150 times, at least 175 times, or at least 200 times more cargo to the chondrocyte than to other cell types.

The nanoparticle comprising a chondrocyte targeting sequence may provide higher selectivity for chondrocyte over other cell types. For example, the nanoparticle comprising a chondrocyte targeting sequence may provide at least 2 times, at least 5 times, at least 10 times, at least 25, at least 50 times, at least 75 times, at least 100 times, at least 125 times, at least 150 times, at least 175 times, or at least 200 times higher selectivity for chondrocyte over other cell types.

The expression "other cell types" is intended to mean at least one cell type other than the chondrocyte. For example, the expression "other cell types" may include at least 2, at least 3, at least 4, at least 5 cell types other than the chondrocyte. Preferably, "other cell types" comprise all cell types found in an animal (e.g. human) body other than the chondrocyte. Thus, the nanoparticle comprising a chondrocyte targeting sequence provides higher selectivity for chondrocyte over all other cell types in an animal (e.g. human) body. Exemplary other cell types include lung cells, blood cells, liver cells, and brain cells.

The nanoparticle is preferably a self-assembled nanoparticle. The nanoparticle may be a nanoparticle which is produced by a process in which pre-existing components (e.g. a lipid component, a cargo and a targeting peptide) form an organized structure as a consequence of specific, local interactions among the components themselves, without external direction.

The cargo, the lipid component and the targeting peptide may reversibly interact to form a self-assembled nanoparticle. The cargo, the lipid component and the targeting peptide may reversibly interact in the self-assembled nanoparticle through intermolecular forces. The cargo, the lipid component and the targeting peptide may reversibly interact in the self-assembled nanoparticle through non-covalent interactions. The cargo, the lipid component and the targeting peptide may reversibly interact in the self-assembled nanoparticle through hydrogen bonds, van der Waals, hydrophobic, and/or electrostatic interactions. The cargo, the lipid component and the targeting peptide may not be conjugated or linked by forces other than inter-molecular forces in the self-assembled nanoparticle.

The nanoparticle may be for delivery of a cargo to a chondrocyte.

The nanoparticle may be for delivery of any type of a cargo to a target cell. Preferably, the cargo has beneficial (e.g. therapeutic) effect on the target cell. The cargo may be a biomolecule. The cargo may be a small molecule. The cargo may be branched, linear, or spaced. The biomolecule may be a nucleic acid, a peptide, a polypeptide, or a protein. The biomolecule may be a fragment of a nucleic acid, a fragment of a peptide, a fragment of polypeptide, or a fragment of a protein.

The nucleic acid may be a DNA molecule or an RNA molecule. The DNA molecule may be a linear DNA molecule or circular DNA molecule. The nucleic acid may be single-stranded, double-stranded, or partially single-stranded and partially double-stranded. The nucleic acid may comprise one or more protected nucleotides (i.e. nucleotides resistant to nuclease (e.g. exonuclease) digestion) (e.g. phosphorothioated nucleotides).

The nucleic acid may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 nucleotides. Preferably, the nucleic acid comprises at least 500 nucleotides.

The nucleic acid may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the nucleic acid

comprises at least 500 base pairs.

The circular DNA molecule may be a plasmid DNA, a vector DNA, a cosmid, an isolated DNA, a bacterial artificial chromosome, a minicircle, or a mini intronic plasmid (MIP). The circular DNA may be an enzymatically produced circular DNA molecule. For example, (i) a circular DNA molecule obtained from recombinase reaction (e.g. Cre recombinase reaction) or (ii) a circular DNA molecule obtained from ligase reaction (e.g. using the golden gate assembly).

The linear DNA molecule may be a portion of a chromosome or a gene. The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides). The linear double-stranded DNA molecule may comprise a first adaptor molecule at a first end and a second adaptor molecule at a second end. The first adaptor molecule and the second adaptor may comprise one or more protected nucleotides (i.e. nucleotides resistant to nuclease (e.g. exonuclease) digestion). The presence of protected nucleotides may confer resistance to nuclease (e.g. exonuclease) digestion. The linear double-stranded DNA molecule may be a DNA molecule having a double-stranded portion comprising a double stranded linear adaptor comprising protected nucleotides (i.e. nucleotides resistant to nuclease (e.g. exonuclease) digestion) at a first end and a double stranded linear adaptor comprising protected nucleotides (i.e. nucleotides resistant to nuclease (e.g. exonuclease) digestion) at a second end.

The linear DNA molecule may be a closed linear DNA molecule. The closed linear DNA molecule may comprise a double-stranded DNA portion that is closed at a first end by a first single-stranded portion (i.e. it may comprise a first hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The linear DNA molecule may be a partially closed linear DNA molecule. The partially closed linear DNA molecule may comprise a double-stranded DNA portion that is closed at a first end and open at a second end. The partially closed linear DNA molecule may comprise a double-stranded DNA portion that is closed at a first end by a single-stranded portion (i.e. it may comprise a first hairpin at the first end) and open at a second end. The partially closed linear DNA molecule may comprise one or more nuclease-resistant nucleotides in an open-end region adjacent to the second end. The open-end region adjacent to the second end may be at the 3' end or 5' end of the molecule. The open-end region adjacent to the second end may comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 nucleotides located at the second end of the partially closed linear DNA molecule. This is to say that the open-end region adjacent to the second end may comprise any nucleotide between and including the end nucleotide of the second end and a nucleotide at location 2, 3, 4, 5, 6, 7, 8, 9, 10, 11, 12, 13, 14, 15, 16, 17, 18, 19, 20, 25, 30, 35, 40, 45, or 50 counting from the end nucleotide of the second end.

The partially closed linear DNA molecule may comprise a hairpin-loop at the 5' end or the 3' end. The partially closed linear DNA molecule may comprise a first adaptor molecule at a first end and a second adaptor molecule at a second end. The first adaptor molecule may comprise a hairpin and a second adaptor may comprise one or more protected nucleotides (i.e. nucleotides resistant to nuclease (e.g. exonuclease) digestion). The hairpin may confer resistance to nuclease (e.g. exonuclease) digestion. The presence of protected nucleotides may confer resistance to nuclease (e.g. exonuclease) digestion. The partially closed linear DNA molecule may be a DNA molecule having a double-stranded portion closed at a first end by ligation of a first adaptor (e.g. hairpin adaptor) to the first end and comprising at a second end a double stranded linear adaptor comprising protected nucleotides (i.e. nucleotides resistant to nuclease (e.g. exonuclease) digestion).

The RNA molecule may be a messenger RNA (mRNA), a transfer RNA, a ribosomal RNA, a small interfering RNA (siRNA), an antisense RNA (an antisense oligonucleotide), a small nuclear RNA (snRNA), a double-stranded RNA, a microRNA (miRNA), short hairpin RNA (shRNA), guide RNA (gRNA), self-amplifying RNA (samRNA), or circular RNA.

The RNA molecule may comprise at least 5, at least 10, at least 15, at least 20, at least 25, at least 30, at least 35, at least 40, at least 45, at least 50, at least 75, at least 100, at least 150, at least 200 nucleotides, at least 500 nucleotides, at least 1,000 nucleotides, at least 2,000 nucleotides, at least 5,000 nucleotides, at least 10,000 nucleotides, at least 15,000 nucleotides or at least 16,000 nucleotides. The RNA molecule may comprise 5-20,000, 6-19,000, 7-18,000, 8-17,000, 9-16,000, 10-15,000, 10-13,000, 15-10,000, 20-5,000, 20-1,000, 20-500, or 25-300 nucleotides. The RNA molecule may comprise one or more protected nucleotides (i.e. nucleotides resistant to nuclease (e.g. exonuclease)

digestion) (e.g. phosphorothioated nucleotides).

The RNA molecule may be an mRNA molecule comprising at least 5, at least 10, at least 15, at least 20, at least 25, at least 30, at least 35, at least 40, at least 45, at least 50, at least 75, at least 100, at least 150, or at least 200 nucleotides. The mRNA molecule may comprise 5-20,000, 6-19,000, 7-18,000, 8-17,000, 9-16,000, 10-15,000, 10-13,000, 15-10,000, 20-5,000, 20-1,000, 20-500, or 25-300 nucleotides. The mRNA molecule may comprise one or more protected nucleotides (i.e. nucleotides resistant to nuclease (e.g. exonuclease) digestion) (e.g. phosphorothioated nucleotides).

The RNA molecule may be a self-amplifying mRNA (samRNA) molecule comprising at least 3,000, at least 4,000, at least 5,000, at least 6,000, at least 7,000, at least 8,000, at least 9,000, at least 10,000, at least 11,000, at least 12,000, at least 13,000, at least 14,000, at least 15,000, at least 16,000, at least 17,000, at least 18,000, at least 19,000, or at least 20,000 nucleotides. The samRNA molecule may comprise 3,000-22,000, 5,000-21,000, 7,000-20,000, or 8,000-17,000 nucleotides. The samRNA molecule may comprise one or more protected nucleotides (i.e. nucleotides resistant to nuclease (e.g. exonuclease) digestion) (e.g. phosphorothioated nucleotides).

The siRNA may comprise a double-stranded portion of at least 17 base pairs, at least 18 base pairs or preferably at least 19 base pairs. The siRNA may comprise a double stranded portion of 17-30 base pairs, 18-27 base pairs, 19-24 base pairs or preferably 19-21 base pairs. The siRNA may comprise one or more protected nucleotides (i.e. nucleotides resistant to nuclease (e.g. exonuclease) digestion) (e.g. phosphorothioated nucleotides).

The miRNA may comprise at least 5, at least 10, at least 15, at least 20, at least 25, at least 30, at least 35, at least 40, at least 45, at least 50, at least 75, at least 100 nucleotides. The miRNA molecule may comprise 10-200, 12-150, 15-125, 17-100, 18-75, 20-75, 20-50, or 20-30 nucleotides.

The miRNA may comprise a double-stranded portion of at least 15 base pairs, at least 17 base pairs or preferably at least 20 base pairs. The miRNA may comprise a double stranded portion of 15-30 base pairs, 17-27 base pairs, 20-25 base pairs or preferably 21-23 base pairs. The miRNA may comprise one or more protected nucleotides (i.e. nucleotides resistant to nuclease (e.g. exonuclease) digestion) (e.g. phosphorothioated nucleotides).

The antisense RNA may comprise at least 18, at least 19, at least 20, at least 21, at least 22, or at least 23 nucleotides. The antisense RNA may comprise 18-24 nucleotides or preferably 19-23 nucleotides. The antisense RNA may comprise one or more protected nucleotides (i.e. nucleotides resistant to nuclease (e.g. exonuclease) digestion) (e.g. phosphorothioated nucleotides).

The nucleic acid cargo may encode a protein (or proteins) useful in treating a joint or cartilage disease or disorder. For example, the nucleic acid cargo may encode an IL-1Ra gene or a part thereof, an Interferon-β gene or a part thereof, an IL-10 gene or a part thereof, an IDUA gene or a part thereof, or a TGF-β gene or a part thereof. The nucleic acid cargo may encode a gene or a part thereof for an anti-inflammatory cytokine. The nucleic acid cargo may encode a one or more modified genes to inhibit pro-inflammatory cytokines, such as TNFα. For example, the nucleic acid cargo may encode TNFR:Fc gene or a part thereof. The nucleic acid cargo may comprise a gene encoding a protein useful in treating a joint disease or disorder. The nucleic acid cargo may comprise two or more genes encoding proteins useful in treating a joint disease or disorder. The joint disease or disorder may be MPS-1, rheumatoid arthritis, osteoarthritis, or Juvenile Idiopathic Arthritis (JIA).

The nucleic acid cargo may comprise one or more of the following genes or portions thereof: IL-1Ra, Interferon-β, IL-10, IDUA, TNFR:Fc or TGF-β .

The nucleic acid cargo may comprise an IL-1Ra gene, or portion(s) thereof, useful in treatment of rheumatoid arthritis.

The nucleic acid cargo may comprise an Interferon-β gene, or portion(s) thereof, useful in treatment of rheumatoid arthritis.

The nucleic acid cargo may comprise an IL-10 gene, or portion(s) thereof, useful in treatment of rheumatoid arthritis.

The nucleic acid cargo may comprise a gene (or portion(s) thereof) which inhibits TNFα, useful in treatment of rheumatoid arthritis.

The nucleic acid cargo may comprise a TNFR:Fc gene, or portion(s) thereof, useful in treatment of rheumatoid arthritis.

The nucleic acid cargo may comprise an IL-1Ra gene, or portion(s) thereof, useful in treatment of Juvenile Idiopathic Arthritis.

The nucleic acid cargo may comprise an Interferon-β gene, or portion(s) thereof, useful in treatment of Juvenile Idiopathic Arthritis.

The nucleic acid cargo may comprise an IL-10 gene, or portion(s) thereof, useful in treatment of Juvenile Idiopathic Arthritis.

The nucleic acid cargo may comprise a TNFR:Fc gene, or portion(s) thereof, useful in treatment of Juvenile Idiopathic Arthritis.

The nucleic acid cargo may comprise a gene (or portion(s) thereof) which inhibits TNFα, useful in treatment of

Juvenile Idiopathic Arthritis.

The nucleic acid cargo may comprise an IL-1Ra gene, or portion(s) thereof, useful in treatment of osteoarthritis.

The nucleic acid cargo may comprise a TGF-β gene, or portion(s) thereof, useful in treatment of osteoarthritis.

The nucleic acid cargo may comprise a IDUA gene, or portion(s) thereof, useful in treatment of MPS-1.

The protein may be a protein of a CRISPR system, for example, Cas9, Cas13, or Cpf1. The protein may be a therapeutic protein. That is to say that the therapeutic protein, once delivered to a target cell, elicits a therapeutic effect. The protein may be a base editing protein, or a prime editing protein.

The protein may be of at least 100 Da, at least 200 Da, at least 300 at least, at least 400 Da, at least 500 Da, at least 750 Da, at least 1 kDa, at least 5 kDa, at least 10 kDa, at least 25 kDa, at least 50 kDa, at least 75 kDa, at least 100 kDa, at least 125 kDa, at least 150 kDa, at least 160 kDa, or at least 175 kDa.

The small molecule may be an agonist (e.g. a receptor agonist), an antagonist (e.g. a receptor antagonist), an activator, or an inhibitor. The small molecule may be a chemical compound. The small molecule may be a drug of a chemical structure comprising 20-100 atoms. The small molecule may be a chemical compound of a molecular mass of less than 1 kDa. For example, the small molecule may be a PTEN inhibitor.

The nanoparticle may be a non-viral transfection complex. Thus, the invention provides a non-viral transfection complex comprising:

(a) a cargo
(b) a lipid component; and
(c) a targeting peptide comprising a chondrocyte targeting sequence.

Preferably, the cargo in the non-viral transfection complex is a nucleic acid. Thus, the non-viral transfection complex may comprise:

(a) a nucleic acid cargo;
(b) a lipid component; and
(c) a targeting peptide comprising a chondrocyte targeting sequence.

The nanoparticle (e.g. the non-viral transfection complex) may further comprise a cationic component, an anionic component, and/or a neutral component. The nanoparticle may comprise a cargo-binding component. The cargo-binding component may be a cargo-binding cationic component, a cargo-binding neutral component or a cargo-binding anionic component. The cationic component, the anionic component, and/or the neutral component may be used to establish a desired charge (i.e. a negative/positive ratio) of the nanoparticle. A specific charge (i.e. a Nitrogen/Phosphate ratio) may be required to facilitate or enhance cell transfection. Thus, the nanoparticle (or a non-viral transfection complex) may comprise:

(a) a cargo
(b) a lipid component;
(c) a targeting peptide comprising a chondrocyte targeting sequence; and
(d) a peptide comprising a cargo-binding component.

The cargo-binding component may be a biomolecule-binding component. The biomolecule-binding component may be a biomolecule-binding cationic component, a biomolecule-binding neutral component or a biomolecule-binding anionic component. The cargo-binding component may be a small molecule-binding component. The small molecule-binding component may be a small molecule-binding cationic component, a small molecule-binding neutral component or a small molecule-binding anionic component.

The cargo-binding component may be a nucleic acid-binding component. The nucleic acid-binding component may be a nucleic acid-binding cationic component or a nucleic acid-binding neutral component.

The nucleic acid-binding component may be a DNA-binding component. The DNA-binding component may be a DNA-binding cationic component or a DNA-binding neutral component. The nucleic acid-binding component may be a closed linear DNA-binding component. The closed linear DNA-binding component may be a closed linear DNA-binding cationic component or a closed linear DNA-binding neutral component. The nucleic acid-binding component may be a partially closed linear DNA-binding component. The partially closed linear DNA-binding component may be a partially closed linear DNA-binding cationic component or a partially closed linear DNA-binding neutral component. The nucleic acid-binding component may be a linear DNA-binding component. The linear DNA binding component may be a linear DNA-binding cationic component, or a linear DNA-binding neutral component. The nucleic acid-binding component may be an RNA-binding component. The RNA-binding component may be an RNA-binding cationic component, or an RNA-binding neutral component. The nucleic acid-binding component may be an antisense RNA-binding component. The

antisense RNA-binding component may be an antisense RNA-binding cationic component, or an antisense RNA-binding neutral component. The nucleic acid-binding component may be a siRNA-binding component. The siRNA-binding component may be a siRNA-binding cationic component, or a siRNA-binding neutral component. The nucleic acid-binding component may be an mRNA-binding component. The mRNA-binding component may be an mRNA-binding cationic component, or an mRNA-binding neutral component. The nucleic acid-binding component may be a transfer RNA-binding component. The transfer RNA-binding component may be a transfer RNA-binding cationic component, or a transfer RNA-binding neutral component. The nucleic acid-binding component may be a ribosomal RNA-binding component. The ribosomal RNA-binding component may be a ribosomal RNA-binding cationic component, or a ribosomal RNA-binding neutral component. The nucleic acid-binding component may be an snRNA-binding component. The snRNA-binding component may be an snRNA-binding cationic component, or an snRNA-binding neutral component. The nucleic acid-binding component may be a double-stranded RNA-binding component. The double-stranded RNA-binding component may be a double-stranded RNA-binding cationic component, or a double-stranded RNA-binding neutral component. The nucleic acid-binding component may be an miRNA-binding component. The miRNA-binding component may be an miRNA-binding cationic component, or an miRNA-binding neutral component. The nucleic acid-binding component may be a shRNA-binding component. The shRNA-binding component may be a shRNA-binding cationic component, or a shRNA-binding neutral component. The nucleic acid-binding component may be a gRNA-binding component. The gRNA-binding component may be a gRNA-binding cationic component, or a gRNA-binding neutral component. The nucleic acid-binding component may be a samRNA-binding component. The samRNA-binding component may be a samRNA-binding cationic component, or a samRNA-binding neutral component. The nucleic acid-binding component may be a circular RNA-binding component. The circular RNA-binding component may be a circular RNA-binding cationic component, or a circular RNA-binding neutral component.

The cargo-binding component may be a protein-binding component. The protein-binding component may be a protein-binding cationic component, a protein-binding neutral component or a protein-binding anionic component. The protein-binding component may be a Cas9-binding component or Cpf1-binding component. The Cas9 binding component may be a Cas9-binding cationic component, a Cas9-binding neutral component or a Cas9-binding anionic component. The Cpf1 binding component may be a Cpf1-binding cationic component, a Cpf1-binding neutral component or a Cpf1-binding anionic component.

The cargo-binding component may be a peptide-binding component. The peptide-binding component may be a peptide-binding cationic component, a peptide-binding neutral component or a peptide-binding anionic component.

The cargo-binding component may be a polypeptide-binding component. The polypeptide-binding component may be a polypeptide-binding cationic component, a polypeptide-binding neutral component or a polypeptide-binding anionic component.

If the cargo is a nucleic acid, the nanoparticle (e.g. the non-viral transfection complex) may comprise a nucleic acid-binding cationic component or a nucleic acid-binding neutral component. Thus, the nanoparticle (or a non-viral transfection complex) may comprise:

(a) a nucleic acid cargo;
(b) a lipid component;
(c) a targeting peptide comprising a chondrocyte targeting sequence; and
(d) a peptide comprising a nucleic acid-binding component.

Preferably, the nucleic acid-binding component is a nucleic acid-binding cationic component (e.g. DNA-binding cationic component).

The cargo-binding cationic component may be a cargo-binding polycationic component, The cargo-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the cargo-binding polycationic component comprises at least 16, at least 17 or at least 30 cationic monomers.

The cargo-binding cationic component may comprise a lysine, a histidine, or an arginine. The cargo-binding polycationic component may comprise a lysine, a histidine, or an arginine. The cargo-binding polycationic component may comprise an oligolysine (linear or branched), an oligohistidine (linear or branched) or an oligoarginine (linear or branched). For example, the cargo-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine

residues. Preferably, the cargo-binding polycationic component comprises at least 16, at least 17, or at least 30 lysine residues. More preferably still, the cargo-binding polycationic component comprises at least 17 lysine residues. The cargo-binding polycationic component may be linear or branched. For example, the cargo-binding linear polycationic component may comprise at least 17 lysine residues. The cargo-binding branched polycationic component may comprise at least 17 lysine residues. The cargo-binding polycationic component may comprise less than 10, or less than 9 lysine residues. The cargo-binding polycationic component may comprise 8 lysine residues.

The nanoparticle (or a non-viral transfection complex) may comprise:

(a) a nucleic acid cargo;
(b) a lipid component;
(c) a targeting peptide comprising a chondrocyte targeting sequence; and
(d) a peptide comprising a nucleic acid-binding cationic component.

The nanoparticle (or a non-viral transfection complex) may comprise:

(e) a nucleic acid cargo;
(f) a lipid component;
(g) a targeting peptide comprising a chondrocyte targeting sequence; and
(h) a peptide comprising a nucleic acid-binding polycationic component,

wherein the nucleic acid-binding polycationic component is oligolysine comprising at least 17 lysine resides.

The nanoparticle (or a non-viral transfection complex) may comprise:

(a) a nucleic acid cargo;
(b) a lipid component;
(c) a targeting peptide comprising a chondrocyte targeting sequence; and
(d) a peptide comprising a nucleic acid-binding polycationic component,

wherein the nucleic acid-binding polycationic component is oligolysine comprising at least 30 lysine resides.

The nanoparticle (or a non-viral transfection complex) may comprise:

(a) a nucleic acid cargo;
(b) a lipid component;
(c) a targeting peptide comprising a chondrocyte targeting sequence; and
(d) a peptide comprising a nucleic acid-binding polycationic component,

wherein the nucleic acid-binding polycationic component is oligolysine comprising at 8 lysine resides.

The nanoparticle (or a non-viral transfection complex) may comprise:

(a) a cargo, wherein the cargo is a nucleic acid;
(b) a lipid component;
(c) a targeting peptide comprising a chondrocyte targeting sequence; and
(d) a peptide comprising a nucleic acid-binding polycationic component,

wherein the nucleic acid-binding linear polycationic component is oligolysine comprising at least 17 lysine resides.

The nanoparticle (or a non-viral transfection complex) may comprise:

(a) a cargo, wherein the cargo is a nucleic acid;
(b) a lipid component;
(c) a targeting peptide comprising a chondrocyte targeting sequence; and
(d) a peptide comprising a nucleic acid-binding polycationic component,

wherein the nucleic acid-binding branched polycationic component is oligolysine comprising at least 30 lysine resides.

The cargo-binding anionic component maybe be a cargo-binding polyanionic component, The cargo-binding polyanionic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at

least 75, at least 80, at least 85, at least 90, at least 95, or at least 100 anionic monomers. The cargo-binding polyanionic component may be linear or branched. For example, the cargo-binding linear polyanionic component may comprise at least 17 monomers. The cargo-binding branched polyanionic component may comprise at least 17 monomers.

The cargo-binding neutral component maybe be a cargo-binding polyneutral component, The cargo-binding polyneutral component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 neutral monomers. The cargo-binding polyneutral component may be linear or branched. For example, the cargo-binding linear polyneutral component may comprise at least 17 monomers. The cargo-binding branched polyneutral component may comprise at least 17 monomers.

The nucleic acid-binding cationic component (e.g. the DNA-binding cationic component) may be a nucleic acid-binding polycationic component (e.g. a DNA-binding polycationic component). The nucleic acid-binding polycationic component may comprise a lysine. The nucleic acid-binding polycationic component may comprise an oligolysine. The nucleic acid-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the nucleic acid-binding polycationic component comprises at least 16, at least 17, or at least 30 lysine residues. More preferably still, the cargo-binding polycationic component comprises at least 17 lysine residues. The nucleic acid-binding polycationic component may be linear or branched. For example, the nucleic acid-binding linear polycationic component may comprise at least 17 lysine residues. The nucleic acid-binding branched polycationic component may comprise at least 17 lysine residues. The nucleic acid-binding polycationic component may comprise less than 10, or less than 9 lysine residues. The nucleic acid-binding polycationic component may comprise 8 lysine residues.

Thus, the nanoparticle (or a non-viral transfection complex) may comprise:

(a) a cargo, wherein the cargo is a nucleic acid;
(b) a lipid component;
(c) a targeting peptide comprising a chondrocyte targeting sequence; and
(d) a peptide comprising a nucleic acid-binding polycationic component (e.g. DNA-binding polycationic component).

The cargo-binding component may be located on the same sequence at the targeting sequence (e.g. chondrocyte targeting sequence). The cargo-binding component may be a part of the targeting peptide. Thus, the invention provides a nanoparticle (or a non-viral transfection complex) comprising:

(a) a cargo
(b) a lipid component; and
(c) a targeting peptide comprising a chondrocyte targeting sequence and a cargo-binding component.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding component (e.g. a DNA-binding polycationic component).

Preferably, the nanoparticle (e.g. the non-viral transfection complex) comprises:

(a) a nucleic acid cargo;
(b) a lipid component; and
(c) a targeting peptide comprising a chondrocyte targeting sequence and a nucleic acid-binding cationic component (e.g. a DNA-binding polycationic component).

The nanoparticle (e.g. the non-viral transfection complex) may comprise:

(a) a nucleic acid cargo;
(b) a lipid component; and

(c) a targeting peptide comprising a chondrocyte targeting sequence and a nucleic acid-binding polycationic component (e.g. a DNA-binding polycationic component).

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding polycationic component (e.g. a DNA-binding polycationic component),

wherein the nucleic acid-binding polycationic component comprises oligolysine, optionally wherein the oligolysine comprises at least 17 lysine residues.
The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding polycationic component (e.g. a DNA-binding polycationic component),

wherein the nucleic acid-binding polycationic component comprises oligolysine, optionally wherein the oligolysine comprises at least 30 lysine residues.
The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding polycationic component (e.g. a DNA-binding polycationic component),

wherein the nucleic acid-binding polycationic component comprises oligolysine, optionally wherein the oligolysine comprises 8 lysine residues.
The cargo may be a linear DNA molecule with enhanced resistance to nuclease (e.g. exonuclease digestion). Thus, the nanoparticle (e.g. the non-viral transfection complex) may comprise:

(a) a nucleic acid cargo, wherein the nucleic acid cargo is a closed linear DNA molecule;
(b) a lipid component; and
(c) a targeting peptide comprising a chondrocyte targeting sequence and a closed linear DNA-binding component (e.g. a closed linear DNA-binding polycationic component).

The cargo may be a linear DNA molecule with enhanced resistance to nuclease (e.g. exonuclease digestion). Thus, the nanoparticle (e.g. the non-viral transfection complex) may comprise:

(a) a nucleic acid cargo, wherein the nucleic acid cargo is a closed linear DNA molecule;
(b) a lipid component; and
(c) a targeting peptide comprising a chondrocyte targeting sequence and a closed linear DNA-binding component (e.g. a closed linear DNA-binding polycationic component);

wherein the closed linear DNA-binding component comprises oligolysine, optionally wherein the oligolysine comprises at least 17 lysine residues.
The cargo may be a linear DNA molecule with enhanced resistance to nuclease (e.g. exonuclease digestion). Thus, the nanoparticle (e.g. the non-viral transfection complex) may comprise:

(a) a nucleic acid cargo, wherein the nucleic acid cargo is a closed linear DNA molecule;
(b) a lipid component; and
(c) a targeting peptide comprising a chondrocyte targeting sequence and a closed linear DNA-binding component (e.g. a closed linear DNA-binding polycationic component);

wherein the closed linear DNA-binding component comprises oligolysine, optionally wherein the oligolysine comprises at least 30 lysine residues.

The cargo may be a linear DNA molecule with enhanced resistance to nuclease (e.g. exonuclease digestion). Thus, the nanoparticle (e.g. the non-viral transfection complex) may comprise:

(a) a nucleic acid cargo, wherein the nucleic acid cargo is a partially closed linear DNA molecule;
(b) a lipid component; and
(c) a targeting peptide comprising a chondrocyte targeting sequence and a partially closed linear DNA-binding component (e.g. a partially closed linear DNA-binding polycationic component).

The cargo may be a linear DNA molecule with enhanced resistance to nuclease (e.g. exonuclease digestion). Thus, the nanoparticle (e.g. the non-viral transfection complex) may comprise:

(a) a nucleic acid cargo, wherein the nucleic acid cargo is a partially closed linear DNA molecule;
(b) a lipid component; and
(c) a targeting peptide comprising a chondrocyte targeting sequence and a partially closed linear DNA-binding component (e.g. a partially closed linear DNA-binding polycationic component);

wherein the partially closed linear DNA-binding component comprises oligolysine, optionally wherein the oligolysine comprises at least 17 lysine residues.

The cargo may be a linear DNA molecule with enhanced resistance to nuclease (e.g. exonuclease digestion). Thus, the nanoparticle (e.g. the non-viral transfection complex) may comprise:

(a) a nucleic acid cargo, wherein the nucleic acid cargo is a partially closed linear DNA molecule;
(b) a lipid component; and
(c) a targeting peptide comprising a chondrocyte targeting sequence and a partially closed linear DNA-binding component (e.g. a partially closed linear DNA-binding polycationic component);

wherein the partially closed linear DNA-binding component comprises oligolysine, optionally wherein the oligolysine comprises at least 30 lysine residues.

The cargo may be an RNA molecule (e.g. mRNA, miRNA or samRNA). Thus, the nanoparticle (e.g. the non-viral transfection complex) may comprise:

(a) a nucleic acid cargo, wherein the nucleic acid cargo is an RNA molecule (e.g. mRNA, miRNA or samRNA);
(b) a lipid component; and
(c) a targeting peptide comprising a chondrocyte targeting sequence and an RNA-binding component (e.g. an RNA-binding polycationic component, such as an mRNA-binding polycationic component, an miRNA-binding polycationic component or a samRNA-binding polycationic component).

The cargo may be an RNA molecule (e.g. mRNA, miRNA or samRNA). Thus, the nanoparticle (e.g. the non-viral transfection complex) may comprise:

(a) a cargo, wherein the cargo is a nucleic acid, wherein the nucleic acid is an RNA molecule (e.g. mRNA, miRNA or samRNA);
(b) a lipid component; and
(c) a targeting peptide comprising a chondrocyte targeting sequence and an RNA-binding component (e.g. an RNA-binding polycationic component, such as an mRNA-binding polycationic component, an miRNA-binding polycationic component or a samRNA-binding polycationic component);

wherein the RNA-binding component comprises oligolysine, optionally wherein the oligolysine comprises at least 17 lysine residues.

The cargo may be an RNA molecule (e.g. mRNA, miRNA or samRNA). Thus, the nanoparticle (e.g. the non-viral transfection complex) may comprise:

(a) a cargo, wherein the cargo is a nucleic acid, wherein the nucleic acid is an RNA molecule (e.g. mRNA, miRNA or samRNA);
(b) a lipid component; and
(c) a targeting peptide comprising a chondrocyte targeting sequence and an RNA-binding component (e.g. an RNA-binding polycationic component, such as an mRNA-binding polycationic component, an miRNA-binding polycationic component or a samRNA-binding polycationic component);

wherein the RNA-binding component comprises oligolysine, optionally wherein the oligolysine comprises at least 30 lysine residues.

The nanoparticle (e.g. the non-viral transfection complex) may comprise:

(a) a nucleic acid cargo, wherein the nucleic acid cargo is a linear DNA molecule comprising one or more nuclease-resistant nucleotides (e.g. phosphorothioated nucleotides);
(b) a lipid component; and
(c) a targeting peptide comprising a chondrocyte targeting sequence and a linear DNA-binding component (e.g. a linear DNA-binding polycationic component).

The nanoparticle (e.g. the non-viral transfection complex) may comprise:

(a) a nucleic acid cargo, wherein the nucleic acid cargo is a linear DNA molecule comprising one or more nuclease-resistant nucleotides (e.g. phosphorothioated nucleotides);
(b) a lipid component; and
(c) a targeting peptide comprising a chondrocyte targeting sequence and a linear DNA-binding component (e.g. a linear DNA-binding polycationic component),

wherein the linear DNA-binding component comprises oligolysine, optionally wherein the oligolysine comprises at least 17 lysine residues.

The nanoparticle (e.g. the non-viral transfection complex) may comprise:

(a) a nucleic acid cargo, wherein the nucleic acid cargo is a linear DNA molecule comprising one or more nuclease-resistant nucleotides (e.g. phosphorothioated nucleotides);
(b) a lipid component; and
(c) a targeting peptide comprising a chondrocyte targeting sequence and a linear DNA-binding component (e.g. a linear DNA-binding polycationic component),

wherein the linear DNA-binding component comprises oligolysine, optionally wherein the oligolysine comprises at least 30 lysine residues.

The cargo-binding component may be linear or branched. The cargo-binding polycationic component may be linear or branched. The cargo-binding polyanionic component may be linear or branched. The cargo-binding polyneutral component may be linear or branched. For example, the cargo-binding polycationic component may comprise at least 16, at least 17, or at least 30 lysine residues in a linear chain. Alternatively, the cargo-binding polycationic component may comprise at least 16, at least 17, or at least 30 lysine residues in a branched chain. The cargo may be a nucleic acid-binding polycationic component. The nucleic acid-binding polycationic component may be linear or branched. For example, the nucleic acid-binding polycationic component (e.g. the DNA-binding polycationic component) may comprise at least 16, at least 17, or at least 30 lysine residues in a linear chain. Alternatively, the nucleic acid-binding polycationic component (e.g. the DNA-binding polycationic component) may comprise at least 16, at least 17, or at least 30 lysine residues in a branched chain. The nucleic acid-binding polycationic component may comprise less than 10, or less than 9 lysine residues in a linear or branched chain. The nucleic acid-binding polycationic component may comprise 8 lysine residues in a linear or branched chain.

The lipid component may be or may form a liposome. The lipid component may comprise a cationic lipid, an anionic lipid, or a neutral lipid. The lipid component may be or may form an anionic liposome, a cationic liposome, or a neutral liposome.

The liposome may comprise at least one lipid. The liposome may comprise at least one cationic lipid. The liposome may comprise at least one phospholipid. The liposome may comprise at least one cationic lipid and at least one phospholipid. The liposome may comprise at least one steroid lipid. The liposome may comprise at least one cationic lipid and at least one steroid lipid. The liposome may comprise at least one phospholipid and at least one steroid lipid. The liposome may comprise at least one cationic lipid, at least one phospholipid and at least one steroid lipid. The liposome may comprise at least one ionizable lipid. The liposome may comprise one or more of the ionizable lipids described in Table 1. The liposome may comprise at least one anionic lipid.

Table 1. Exemplary lipids which can be used in the nanoparticles described herein.

| Type | Function in LNPs | Example |
|---|---|---|
| Unsaturated | Enhance membrane disruption and payload release by increasing the tendency of bilayer lipids to form a nonbilayer phase. | 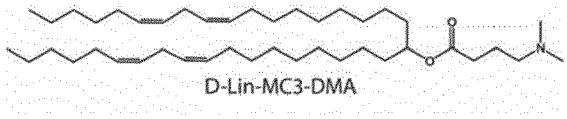 D-Lin-MC3-DMA |
| Multi-tail | Enhance endosomal disruption and RNA delivery by producing a cone-shaped LNP structure by increasing the cross-sectional area of the tail region. | 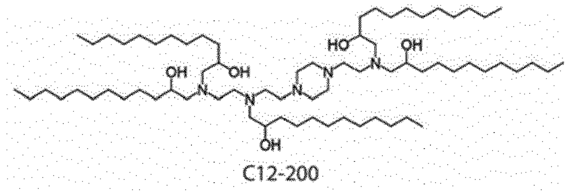 C12-200 |
| Polymeric | Enhance particle formation through hydrophobic aggregation. | 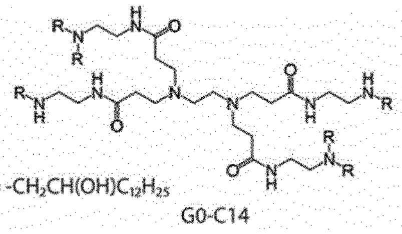 R = $-CH_2CH(OH)C_{12}H_{25}$ G0-C14 |
| Biodegradable | Reduce continual accumulation and toxicity after intracellular RNA delivery. | 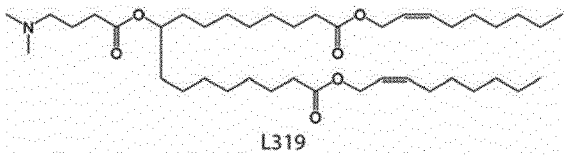 L319 |
| Branched-tail | Increase RNA delivery potency by enhancing endosomal escape and increasing the cross-sectional area of lipid tails. | 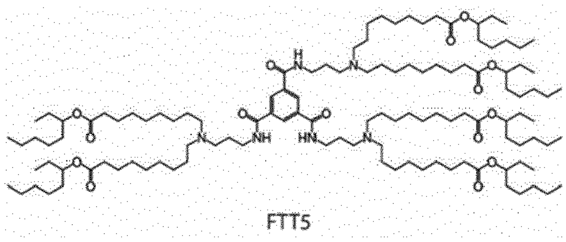 FTT5 |

The cationic lipid may be DTDTMA (ditetradecyl trimethyl ammonium), DOTMA (2,3-dioleyloxypropy1-1-trimentyl ammonium), or DHDTMA (dihexadecyl trimethyl ammonium). In addition to the cation, the cationic lipids may comprise a counter anion, for example, an inorganic counter ion, especially a pharmaceutically acceptable anion such as chloride or bromide. Preferably, the cationic lipid is DOTMA.

The lipid component may comprise a phospholipid. The term "phospholipid" refers to a lipid comprising a fatty acid chin and a phosphate group. Phospholipids are typically neutral molecules in that they do not have an overall charge, unlike a cationic lipid, which is positively charged. Phospholipids are typically zwitterionic molecules comprising both positive and negative charged components, but no overall charge. Thus, the neutral lipid may be a phospholipid. For example, the phospholipid may be DOPE (phosphatidylethanolamine or 1,2-dioleoyl-sn-glycero-3-phosphoethanolamine), or DOPC (phosphatidyl choline or 1,2-dioleoyl- sn-glycero-3-phosphoethanoltrimethylamine). Preferably, the phospholipid is DOPE.

The phospholipid may comprise a PEG moiety. The PEG moiety may have a molecular weight of from about 100 to about 10,000, optionally the PEG moiety has a molecular weight of from about 250 to about 7,500, optionally the PEG moiety has a molecular weight of from about 500 to about 5,000, optionally the PEG moiety has a molecular weight of from about 750 to about 4,000, optionally the PEG moiety has a molecular weight of from about 1,000 to about 3,000, optionally the PEG moiety has a molecular weight of approximately 2,000.

The lipid may be a PEGylated lipid e.g. DMG-PEG.

The ionizable lipid may be (6Z,9Z,28Z,31Z)-heptatriaconta-6,9,28,31-tetraen-19-yl-4-(dimethylamino)-butanoate (Dlin-MC3-DMA (MC3)), 2,2-dilinoleyl-4-(2- dimethylaminoethyl)-[1,3]-dioxolane (DLin-KC2-DMA (KC2)) or ALC-0315 ([(4-hydroxybutyl)azanediyl]di(hexane-6,1-diyl) bis(2-hexyldecanoate)).

The anionic lipid may be 1,2-Dioleoyl-sn-glycero-3-phosphoglycerol (DOPG).

The steroid lipid may be cholesterol. The liposome may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, at least 25%, at least 30% at least 35%, at least 40%, at least 45%, at least 50%, or at least 55% cholesterol (as defined by molar amount of cholesterol). That is to say that the liposome may comprise at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, at least 20% at least 21%, at least 22%, at least 23%, at least 24%, or at least 25% cholesterol and at least 99%, at least 98%, at least 97%, at least 96% at least 95%, at least 94%, at least 93%, at least 92%, at least 91%, at least 90%, at least 89%, at least 88%, at least 87%, at least 86%, at least 85%, at least 84%, at least 83%, at least 82%, at least 81%, at least 80% at least 79%, at least 78%, at least 77%, at least 76%, at least 75%, at least 70%, at least 65%, at least 60%, at least 55%, at least 50%, or at least 45% of other lipids in the liposome (as defined by molar ratio).

The molar ratio of at least one cationic lipid to at least one phospholipid in the nanoparticle (or non-viral transfection complex) may be 1:1, 1:2, 1:3, 1:4, 1:5, 2:1, 3:1, 4:1, or 5:1. Preferably, the molar ratio of at least one cationic lipid to at least one phospholipid in the nanoparticle (or non-viral transfection complex) is 1:1 or 2:1. For example, the molar ratio of DOTMA to DOPE in the nanoparticle (or non-viral transfection complex) may be 1:1. That is to say that the molar amount of DOTMA and DOPE in the nanoparticle is the same. The molar ratio of DOTMA to DOPE in the nanoparticle (or non-viral transfection complex) may be 2:1. That is to say that the molar amount of DOTMA is twice the molar amount of DOPE.

The nanoparticle may comprise an ionizable lipid and a cationic lipid. The nanoparticle may comprise an ionizable lipid and cholesterol. The nanoparticle may comprise an ionizable lipid and a PEG lipid. The nanoparticle may comprise a cationic lipid and cholesterol. The nanoparticle may comprise a cationic lipid and a PEG lipid. The nanoparticle may comprise an ionizable lipid and a phospholipid lipid. The nanoparticle may comprise an ionizable lipid, a phospholipid and a cationic lipid. The nanoparticle may further comprise cholesterol. The nanoparticle may comprise an ionizable lipid, a phospholipid and cholesterol. The nanoparticle may comprise a cationic lipid, a phospholipid and cholesterol. The nanoparticle may comprise an ionizable lipid, a phospholipid, cholesterol and a PEG lipid. The nanoparticle may comprise a cationic lipid, a phospholipid, cholesterol and a PEG lipid. The nanoparticle may comprise an ionizable lipid, a phospholipid, cholesterol and a cationic lipid. For example, the nanoparticle may comprise ALC-0315, DMG-PEG, cholesterol and DOTMA. The nanoparticle may comprise ALC-0315, DMG-PEG, cholesterol and DOPE. The nanoparticle may comprise ALC-0315, DMG-PEG, cholesterol, DOPE and DOTMA. The nanoparticle may comprise DOTMA, DMG-PEG, cholesterol and DOPE.

The mass ratio of targeting peptide to cargo (e.g. nucleic acid) in the nanoparticle may be between 1.0-5.0 (targeting peptide) to 0.6-1.5 (cargo). For example, the mass ratio of targeting peptide to cargo may be about 1.5 to about 1, about 2 to about 1, about 2.15 to about 1, about 2.5 to about 1, about 2.7 to about 1, about 3 to about 1, about 3.2 to about 1, about 4 to about 1, about 4.5 to about 1, or about 5 to about 1 [peptide: cargo].

The molar ratio of targeting peptide to cargo (e.g. nucleic acid) in the nanoparticle may be at least 50:1, at least 100:1, at least 150:1, at least 200:1, at least 250:1 at least 300:1, at least 350:1, at least 400:1, at least 450:1. at least 500:1, at least 550:1 at least 600:1, at least 650:1, at least 700:1, at least 750:1 at least 800:1, at least 850:1, at least 900:1, at least 950:1 at least 1000:1, at least 1050:1, at least 1100:1, at least 1150:1 at least 1200:1, at least 1250:1, or at least 1300:1.

The targeting peptide may comprise at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40 amino acids (e.g. from a targeting sequence and/or a cargo-binding component). The amount of positively charged amino acids in the targeting peptide may be at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31.

If the cargo is a nucleic acid molecule (e.g. DNA molecule), the targeting peptide may comprise a nucleic-acid binding cationic component.

The molar ratio of the targeting peptide to the nucleic acid molecule in the nanoparticle may be at least 350:1 or at least 650:1 and the N/P ratio of the nanoparticle may be about 4.

The molar ratio of the targeting peptide to the nucleic acid molecule in the nanoparticle may be at least 500:1 or at least 900:1 and the N/P ratio of the nanoparticle may be about 6.

The molar ratio of the targeting peptide to the nucleic acid molecule in the nanoparticle may be at least 750:1 or at least 1400:1 and the N/P ratio of the nanoparticle may be about 8.

The targeting peptide may comprise at least 30 (e.g. 31) positively charged amino acids. In such cases, the peptide : cargo (e.g. nucleic acid) molar ratio may be between 300;1 and 850:1.

The targeting peptide may comprise at least 15 (e.g. 17) positively charged amino acids. In such cases, the peptide : cargo (e.g. nucleic acid) molar ratio may be between 600;1 and 1500:1.

The specific ratio of targeting peptide to cargo (e.g. nucleic acid) allows for an effective and stable formulation of the nanoparticle.

The molar ratio of DNA molecule: lipid: targeting peptide in the nanoparticle may be between 0.6-1.5 (DNA molecule) to between 1000-6000 (lipid) to between 500-1500 (peptide).

The targeting sequence may be a chondrocyte targeting sequence.

The targeting sequence may comprise at least 3, at least 4, at least 5, at least 6, at least 7, at least 8, at least 9, at least 10, at least 11, at least 12, at least 13, at least 14 at least 15, at least 16, at least 17 or at least 18, at least 19, or at least 20 amino acids. The targeting sequence may comprise 2-35, 3-30, 4-25, 4-23, 4-20, 5-22, 6-21, 5-17, 6-15, 7-14, or 7-20 amino acids. Preferably, the targeting sequence comprises 7-14 amino acids or 7-20 amino acids. For example, the targeting sequence may comprise 7, 12, or 20 amino acids.

The targeting sequence (e.g. chondrocyte targeting sequence) may comprise at least 3, at least 4, at least 5 or at least 6 contiguous amino acids of SEQ ID NO: 1 or a variant thereof comprising one or more conservative amino acid substitutions. The targeting sequence (e.g. the chondrocyte targeting sequence) may be SEQ ID NO: 1 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 1. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 1.

The targeting sequence (e.g. chondrocyte targeting sequence) may comprise at least 3, at least 4, at least 5 or at least 6 contiguous amino acids of SEQ ID NO: 2 or a variant thereof comprising one or more conservative amino acid substitutions. The targeting sequence (e.g. the chondrocyte targeting sequence) may be SEQ ID NO: 2 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 2. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 2.

The targeting sequence (e.g. the chondrocyte targeting sequence) may comprise at least 4, at least 5, at least 6, at least 7, at least 8, at least 9, at least 10 or at least 11 contiguous amino acids of SEQ ID NO: 3 or a variant thereof comprising one or more conservative amino acid substitutions. The targeting sequence (e.g. the chondrocyte targeting sequence) may be SEQ ID NO: 3 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 3. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 3.

The targeting sequence (e.g. the chondrocyte targeting sequence) may comprise at least 4, at least 5, at least 6, at least 7, at least 8, at least 9, at least 10 or at least 11 contiguous amino acids of SEQ ID NO: 4 or a variant thereof comprising one or more conservative amino acid substitutions. The targeting sequence (e.g. the chondrocyte targeting sequence) may be SEQ ID NO: 4 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 4. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 4.

The targeting sequence (e.g. the chondrocyte targeting sequence) may comprise at least 4, at least 5, at least 6, at least 7, at least 8, at least 9, at least 10 or at least 11 contiguous amino acids of SEQ ID NO: 5 or a variant thereof comprising one or more conservative amino acid substitutions. The targeting sequence (e.g. the chondrocyte targeting sequence) may be SEQ ID NO: 5 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least

94%, at least 95%, at least 96%, at least 97%, at least 98%, at least 99% or at least 100% similarity to SEQ ID NO: 5. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 5.

The targeting sequence may be any sequence listed in Table 2 or a variant thereof comprising one or more conservative amino acid substitutions.

Table 2. Exemplary targeting sequences.

| SEQ ID NO | Sequence | Peptide name |
|---|---|---|
| 1 | STLHQKL | HC1 |
| 2 | SSLHQKL | HC2 |
| 3 | RLDPTSYLRTFW | HC3 |
| 4 | HDSQLEALIKFM | HC4 |
| 5 | DWRVIIPPRPSA | HC5 |

Thus, the invention provides a nanoparticle (or a non-viral transfection complex) comprising:

(a) a cargo
(b) a lipid component; and
(c) a targeting peptide comprising a chondrocyte targeting sequence and cargo-binding component, wherein the chondrocyte targeting sequence is SEQ ID NO: 1 or a variant thereof comprising one or more conservative amino acid substitutions.

The invention also provides a nanoparticle (or a non-viral transfection complex) comprising:

(a) a cargo
(b) a lipid component; and
(c) a targeting peptide comprising a chondrocyte targeting sequence and cargo-binding component, wherein the chondrocyte targeting sequence is SEQ ID NO: 2 or a variant thereof comprising one or more conservative amino acid substitutions.

The invention also provides a nanoparticle (or a non-viral transfection complex) comprising:

(a) a cargo
(b) a lipid component; and
(c) a targeting peptide comprising a chondrocyte targeting sequence and cargo-binding component, wherein the chondrocyte targeting sequence is SEQ ID NO: 3 or a variant thereof comprising one or more conservative amino acid substitutions.

The invention also provides a nanoparticle (or a non-viral transfection complex) comprising:

(a) a cargo
(b) a lipid component; and
(c) a targeting peptide comprising a chondrocyte targeting sequence and cargo-binding component, wherein the chondrocyte targeting sequence is SEQ ID NO: 4 or a variant thereof comprising one or more conservative amino acid substitutions.

The invention also provides a nanoparticle (or a non-viral transfection complex) comprising:

(a) a cargo
(b) a lipid component; and
(c) a targeting peptide comprising a chondrocyte targeting sequence and cargo-binding component, wherein the chondrocyte targeting sequence is SEQ ID NO: 5 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding component, wherein the chondrocyte targeting sequence is SEQ ID NO: 1 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding component, wherein the chondrocyte targeting sequence is SEQ ID NO: 2 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding component, wherein the chondrocyte targeting sequence is SEQ ID NO: 3 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding component, wherein the chondrocyte targeting sequence is SEQ ID NO: 4 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding component, wherein the chondrocyte targeting sequence is SEQ ID NO: 5 or a variant thereof comprising one or more conservative amino acid substitutions.

Preferably, the nanoparticle (e.g. the non-viral transfection complex) comprises:

(a) a nucleic acid cargo;
(b) a lipid component; and
(c) a targeting peptide comprising a chondrocyte targeting sequence and a nucleic acid-binding cationic component, wherein the chondrocyte targeting sequence is SEQ ID NO: 1 or a variant thereof comprising one or more conservative amino acid substitutions.

Preferably, the nanoparticle (e.g. the non-viral transfection complex) comprises:

(a) a nucleic acid cargo;
(b) a lipid component; and
(c) a targeting peptide comprising a chondrocyte targeting sequence and a nucleic acid-binding cationic component, wherein the chondrocyte targeting sequence is SEQ ID NO: 2 or a variant thereof comprising one or more conservative amino acid substitutions.

Preferably, the nanoparticle (e.g. the non-viral transfection complex) comprises:

(a) a nucleic acid cargo;
(b) a lipid component; and
(c) a targeting peptide comprising a chondrocyte targeting sequence and a nucleic acid-binding cationic component, wherein the chondrocyte targeting sequence is SEQ ID NO: 3 or a variant thereof comprising one or more conservative amino acid substitutions.

Preferably, the nanoparticle (e.g. the non-viral transfection complex) comprises:

(a) a nucleic acid cargo;
(b) a lipid component; and
(c) a targeting peptide comprising a chondrocyte targeting sequence and a nucleic acid-binding cationic component, wherein the chondrocyte targeting sequence is SEQ ID NO: 4 or a variant thereof comprising one or more conservative amino acid substitutions.

Preferably, the nanoparticle (e.g. the non-viral transfection complex) comprises:

(a) a nucleic acid cargo;
(b) a lipid component; and
(c) a targeting peptide comprising a chondrocyte targeting sequence and a nucleic acid-binding cationic component, wherein the chondrocyte targeting sequence is SEQ ID NO: 5 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding polycationic component, wherein the chondrocyte targeting sequence is SEQ ID NO: 1 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding polycationic component, wherein the chondrocyte targeting sequence is SEQ ID NO: 2 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding polycationic component, wherein the chondrocyte targeting sequence is SEQ ID NO: 3 or a variant thereof comprising one or more conservative amino acid substitutions.

The nanoparticle (e.g. the non-viral transfection complex) may comprise:

(a) a nucleic acid cargo;
(b) a lipid component; and
(c) a targeting peptide comprising a chondrocyte targeting sequence and a nucleic acid-binding polycationic component, wherein the chondrocyte targeting sequence is SEQ ID NO: 4 or a variant thereof comprising one or more conservative amino acid substitutions.

The nanoparticle (e.g. the non-viral transfection complex) may comprise:

(a) a nucleic acid cargo;
(b) a lipid component; and

(c) a targeting peptide comprising a chondrocyte targeting sequence and a nucleic acid-binding polycationic component, wherein the chondrocyte targeting sequence is SEQ ID NO: 5 or a variant thereof comprising one or more conservative amino acid substitutions.

The nanoparticle (e.g. the non-viral transfection complex) may comprise:

(a) a nucleic acid cargo, wherein the nucleic acid cargo is a closed linear DNA molecule;
(b) a lipid component; and
(c) a targeting peptide comprising a chondrocyte targeting sequence and a closed linear DNA-binding component (e.g. a closed linear DNA-binding polycationic component), wherein the chondrocyte targeting sequence is SEQ ID NO: 1, SEQ ID NO: 2, SEQ ID NO: 3, SEQ ID NO: 4, or SEQ ID NO: 5, or a variant thereof comprising one or more conservative amino acid substitutions.

The nanoparticle (e.g. the non-viral transfection complex) may comprise:

(a) a nucleic acid cargo, wherein the nucleic acid cargo is a linear DNA molecule comprising one or more nuclease-resistant nucleotides (e.g. phosphorothioated nucleotides);
(b) a lipid component; and
(c) a targeting peptide comprising a chondrocyte targeting sequence and a linear DNA-binding component (e.g. a linear DNA-binding polycationic component), wherein the chondrocyte targeting sequence is SEQ ID NO: 1, SEQ ID NO: 2, SEQ ID NO: 3, SEQ ID NO: 4, or SEQ ID NO: 5, or a variant thereof comprising one or more conservative amino acid substitutions.

The nanoparticle (e.g. the non-viral transfection complex) may comprise:

(a) a nucleic acid cargo, wherein the nucleic acid cargo is a partially closed linear DNA molecule, optionally comprising one or more nuclease-resistant nucleotides (e.g. phosphorothioated nucleotides);
(b) a lipid component; and
(c) a targeting peptide comprising a chondrocyte targeting sequence and a partially closed linear DNA-binding component (e.g. a partially closed linear DNA-binding polycationic component), wherein the chondrocyte targeting sequence is SEQ ID NO: 1, SEQ ID NO: 2, SEQ ID NO: 3, SEQ ID NO: 4, or SEQ ID NO: 5, or a variant thereof comprising one or more conservative amino acid substitutions.

The nanoparticle (e.g. the non-viral transfection complex) may comprise:

(a) a cargo, wherein the cargo is a nucleic acid, wherein the nucleic acid is an RNA molecule;
(b) a lipid component; and
(c) a targeting peptide comprising a chondrocyte targeting sequence and an RNA-binding component (e.g. an RNA-binding polycationic component), wherein the chondrocyte targeting sequence is SEQ ID NO: 1, SEQ ID NO: 2, SEQ ID NO: 3, SEQ ID NO: 4, or SEQ ID NO: 5, or a variant thereof comprising one or more conservative amino acid substitutions.

The nanoparticle (e.g. the non-viral transfection complex) may comprise:

(a) a nucleic acid cargo, wherein the nucleic acid cargo is a miRNA molecule;
(b) a lipid component; and
(c) a targeting peptide comprising a chondrocyte targeting sequence and a miRNA-binding component (e.g. a miRNA-binding polycationic component), wherein the chondrocyte targeting sequence is SEQ ID NO: 1, SEQ ID NO: 2, SEQ ID NO: 3, SEQ ID NO: 4, or SEQ ID NO: 5, or a variant thereof comprising one or more conservative amino acid substitutions.

The targeting peptide may comprise at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40. The targeting sequence (e.g. the chondrocyte targeting sequence) may comprise 4-100, 5-70, 6-50, 7-40, 7-15 amino acids.

The targeting peptide may comprise at least 4, at least 5, at least 6, at least 7, or at least 8 contiguous amino acids of SEQ ID NO: 6 or a variant thereof comprising one or more conservative amino acid substitutions. The targeting peptide may be SEQ ID NO: 6 or a variant thereof comprising one or more conservative amino acid substitutions. The

targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 6. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 6.

The targeting peptide may comprise at least 6, at least 7, or at least 8, at least 9, at least 10 contiguous amino acids of SEQ ID NO: 7 or a variant thereof comprising one or more conservative amino acid substitutions. The targeting peptide may be SEQ ID NO: 7 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 7. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 7.

The targeting peptide may comprise at least 8, at least 9, at least 10, at least 11, at least 12, at least 13, or at least 14 contiguous amino acids of SEQ ID NO: 8 or a variant thereof comprising one or more conservative amino acid substitutions. The targeting peptide may be SEQ ID NO: 8 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 8. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 8.

The targeting peptide may comprise at least 18, at least 19, at least 20, at least 21, at least 22, at least 23, or at least 24 contiguous amino acids of SEQ ID NO: 9 or a variant thereof comprising one or more conservative amino acid substitutions. The targeting peptide may be SEQ ID NO: 9 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 9. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 9.

The targeting peptide may comprise at least 18, at least 19, at least 20, at least 21, at least 22, at least 23, at least 24, at least 25, or at least 26 contiguous amino acids of SEQ ID NO: 10 or a variant thereof comprising one or more conservative amino acid substitutions. The targeting peptide may be SEQ ID NO: 10 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 10. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 10.

The targeting peptide may comprise at least 20, at least 21, at least 22, at least 23, at least 24, at least 25, at least 26, at least 27, at least 28, at least 29, or at least 30 contiguous amino acids of SEQ ID NO: 11 or a variant thereof comprising one or more conservative amino acid substitutions. The targeting peptide may be SEQ ID NO: 11 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 11. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 11.

The targeting peptide may comprise at least 4, at least 5, at least 6, at least 7, or at least 8 contiguous amino acids of SEQ ID NO: 12 or a variant thereof comprising one or more conservative amino acid substitutions. The targeting peptide may be SEQ ID NO: 12 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 12. The targeting peptide may comprise a sequence comprising at least 60%, at least 70%, at least 75%, at least 80%, at least 85%, at least 90%, at least 91%, at least 92%,

at least 93%, at least 94%, at least 95%, at least 96%, at least 97%, at least 98%, at least 99% or at least 100% identity to SEQ ID NO: 12.

The targeting peptide may comprise at least 6, at least 7, or at least 8, at least 9, at least 10 contiguous amino acids of SEQ ID NO: 13 or a variant thereof comprising one or more conservative amino acid substitutions. The targeting peptide may be SEQ ID NO: 13 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 13. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 13.

The targeting peptide may comprise at least 8, at least 9, at least 10, at least 11, at least 12, at least 13, or at least 14 contiguous amino acids of SEQ ID NO: 14 or a variant thereof comprising one or more conservative amino acid substitutions. The targeting peptide may be SEQ ID NO: 14 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 14. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 14.

The targeting peptide may comprise at least 18, at least 19, at least 20, at least 21, at least 22, at least 23, or at least 24 contiguous amino acids of SEQ ID NO: 15 or a variant thereof comprising one or more conservative amino acid substitutions. The targeting peptide may be SEQ ID NO: 15 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 15. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 15.

The targeting peptide may comprise at least 18, at least 19, at least 20, at least 21, at least 22, at least 23, at least 24, at least 25, or at least 26 contiguous amino acids of SEQ ID NO: 16 or a variant thereof comprising one or more conservative amino acid substitutions. The targeting peptide may be SEQ ID NO: 16 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 16. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 16.

The targeting peptide may comprise at least 20, at least 21, at least 22, at least 23, at least 24, at least 25, at least 26, at least 27, at least 28, at least 29, or at least 30 contiguous amino acids of SEQ ID NO: 17 or a variant thereof comprising one or more conservative amino acid substitutions. The targeting peptide may be SEQ ID NO: 17 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 17. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 17.

The targeting peptide may comprise at least 9, at least 10, at least 11, at least 12, or at least 13 contiguous amino acids of SEQ ID NO: 18 or a variant thereof comprising one or more conservative amino acid substitutions. The targeting peptide may be SEQ ID NO: 18 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 18. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 18.

The targeting peptide may comprise at least 11, at least 12, or at least 13, at least 14, at least 15 contiguous amino acids of SEQ ID NO: 19 or a variant thereof comprising one or more conservative amino acid substitutions. The

targeting peptide may be SEQ ID NO: 19 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 19. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 19.

The targeting peptide may comprise at least 13, at least 14, at least 15, at least 16, at least 17, at least 18, or at least 19 contiguous amino acids of SEQ ID NO: 20 or a variant thereof comprising one or more conservative amino acid substitutions. The targeting peptide may be SEQ ID NO: 20 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 20. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 20.

The targeting peptide may comprise at least 23, at least 24, at least 25, at least 26, at least 27, at least 28, or at least 29 contiguous amino acids of SEQ ID NO: 21 or a variant thereof comprising one or more conservative amino acid substitutions. The targeting peptide may be SEQ ID NO: 21 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 21. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 21.

The targeting peptide may comprise at least 23, at least 24, at least 25, at least 26, at least 27, at least 28, at least 29, at least 30, or at least 31 contiguous amino acids of SEQ ID NO: 22 or a variant thereof comprising one or more conservative amino acid substitutions. The targeting peptide may be SEQ ID NO: 22 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 22. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 122.

The targeting peptide may comprise at least 25, at least 26, at least 27, at least 28, at least 29, at least 30, at least 31, at least 32, at least 33, at least 34, or at least 35 contiguous amino acids of SEQ ID NO: 23 or a variant thereof comprising one or more conservative amino acid substitutions. The targeting peptide may be SEQ ID NO: 23 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 23. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 23.

The targeting peptide may comprise at least 9, at least 10, at least 11, at least 12, or at least 13 contiguous amino acids of SEQ ID NO: 24 or a variant thereof comprising one or more conservative amino acid substitutions. The targeting peptide may be SEQ ID NO: 24 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 24. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 24.

The targeting peptide may comprise at least 11, at least 12, or at least 13, at least 14, at least 15 contiguous amino acids of SEQ ID NO: 25 or a variant thereof comprising one or more conservative amino acid substitutions. The targeting peptide may be SEQ ID NO: 25 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 25. The targeting peptide may comprise

a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 25.

The targeting peptide may comprise at least 13, at least 14, at least 15, at least 16, at least 17, at least 18, or at least 19 contiguous amino acids of SEQ ID NO: 26 or a variant thereof comprising one or more conservative amino acid substitutions. The targeting peptide may be SEQ ID NO: 26 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 26. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 26.

The targeting peptide may comprise at least 23, at least 24, at least 25, at least 26, at least 27, at least 28, or at least 29 contiguous amino acids of SEQ ID NO: 27 or a variant thereof comprising one or more conservative amino acid substitutions. The targeting peptide may be SEQ ID NO: 27 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 27. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 27.

The targeting peptide may comprise at least 23, at least 24, at least 25, at least 26, at least 27, at least 28, at least 29, at least 30, or at least 31 contiguous amino acids of SEQ ID NO: 28 or a variant thereof comprising one or more conservative amino acid substitutions. The targeting peptide may be SEQ ID NO: 28 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 28. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 28.

The targeting peptide may comprise at least 25, at least 26, at least 27, at least 28, at least 29, at least 30, at least 31, at least 32, at least 33, at least 34, or at least 35 contiguous amino acids of SEQ ID NO: 29 or a variant thereof comprising one or more conservative amino acid substitutions. The targeting peptide may be SEQ ID NO: 29 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 29. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 29.

The targeting peptide may comprise at least 9, at least 10, at least 11, at least 12, or at least 13 contiguous amino acids of SEQ ID NO: 30 or a variant thereof comprising one or more conservative amino acid substitutions. The targeting peptide may be SEQ ID NO: 30 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 30. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 30.

The targeting peptide may comprise at least 11, at least 12, or at least 13, at least 14, at least 15 contiguous amino acids of SEQ ID NO: 31 or a variant thereof comprising one or more conservative amino acid substitutions. The targeting peptide may be SEQ ID NO: 31 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 31. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 31.

The targeting peptide may comprise at least 13, at least 14, at least 15, at least 16, at least 17, at least 18, or

at least 19 contiguous amino acids of SEQ ID NO: 32 or a variant thereof comprising one or more conservative amino acid substitutions. The targeting peptide may be SEQ ID NO: 32 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 32. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 32.

The targeting peptide may comprise at least 13, at least 14, at least 15, at least 16, or at least 17 contiguous amino acids of SEQ ID NO: 33 or a variant thereof comprising one or more conservative amino acid substitutions. The targeting peptide may be SEQ ID NO: 33 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 33. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 33.

The targeting peptide may comprise at least 23, at least 24, at least 25, at least 26, at least 27, at least 28, or at least 29 contiguous amino acids of SEQ ID NO: 34 or a variant thereof comprising one or more conservative amino acid substitutions. The targeting peptide may be SEQ ID NO: 34 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 34. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 34.

The targeting peptide may comprise at least 23, at least 24, at least 25, at least 26, at least 27, at least 28, at least 29, at least 30, or at least 31 contiguous amino acids of SEQ ID NO: 35 or a variant thereof comprising one or more conservative amino acid substitutions. The targeting peptide may be SEQ ID NO: 35 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 35. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 35.

The targeting peptide may comprise at least 25, at least 26, at least 27, at least 28, at least 29, at least 30, at least 31, at least 32, or at least 33 contiguous amino acids of SEQ ID NO: 36 or a variant thereof comprising one or more conservative amino acid substitutions. The targeting peptide may be SEQ ID NO: 36 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 36. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 36.

The targeting peptide may comprise at least 25, at least 26, at least 27, at least 28, at least 29, at least 30, at least 31, at least 32, at least 33, at least 34, or at least 35 contiguous amino acids of SEQ ID NO: 37 or a variant thereof comprising one or more conservative amino acid substitutions. The targeting peptide may be SEQ ID NO: 37 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 37. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 37.

The polylysine chain in SEQ ID NOs: 9-11, 15-17, 21-23, 27-29 and 34-37 may be a polylysine chain of two or more lysine residues. For example, the polylysine chain may be of 8, 16 or 32 lysine residues.

The position of the "GA" spacer in SEQ ID Nos: 8, 11, 14, 17, 20, 23, 26, 29, 32, and 37 can be before or after the "RVRR" linker residues. The "GA" spacer may be substituted with a "GG" spacer of an "AA" spacer in any of the above sequences.

By "sequence identity" or "sequence similarity" is meant that the identity or similarity, respectively, between two or more amino acid sequences, or two or more nucleotide sequences, is expressed in terms of the identity or similarity between the sequences. Sequence identity can be measured in terms of "percentage (%) identity," in which a higher percentage indicates greater identity shared between the sequences. Sequence similarity can be measured in terms of percentage similarity (which takes into account conservative amino acid substitutions); the higher the percentage, the more similarity shared between the sequences.

The peptides described herein may comprise conservative amino acid substitutions at one or more amino acid residues, e.g. at essential or non-essential amino acid residues. A "conservative amino acid substitution" is one in which the amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagines, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

The targeting peptide may comprise any one of the sequences of Table 3.

Table 3. Exemplary targeting peptides.

| SE Q ID NO | Sequence | Peptide name |
|---|---|---|
| 6 | GASTLHQKL | |
| 7 | GACSTLHQKLC | |
| 8 | GARVRRCSTLHQKLC | |
| 9 | KKKKKKKKKKKKKKKGASTLHQKL | HC1 L-N |
| 10 | KKKKKKKKKKKKKKKGACSTLHQKLC | HC1 C-N |
| 11 | KKKKKKKKKKKKKKKGARVRRCSTLHQKLC | HC1 C-C |
| 12 | GASSLHQKL | |
| 13 | GACSSLHQKLC | |
| 14 | GARVRRCSSLHQKLC | |
| 15 | KKKKKKKKKKKKKKKGASSLHQKL | HC2 L-N |
| 16 | KKKKKKKKKKKKKKKGACSSLHQKLC | HC2 C-N |
| 17 | KKKKKKKKKKKKKKKGARVRRCSSLHQKLC | HC2 C-C |
| 18 | GARLDPTSYLRTFW | |
| 19 | GACRLDPTSYLRTFWC | |
| 20 | GARVRRCRLDPTSYLRTFWC | |
| 21 | KKKKKKKKKKKKKKKGARLDPTSYLRTFW | HC3 L-N |
| 22 | KKKKKKKKKKKKKKKGACRLDPTSYLRTFWC | HC3 C-N |
| 23 | KKKKKKKKKKKKKKKGARVRRCRLDPTSYLRTFWC | HC3 C-C |
| 24 | GAHDSQLEALIKFM | |
| 25 | GACHDSQLEALIKFMC | |
| 26 | RVRRGACHDSQLEALIKFMC | |
| 27 | KKKKKKKKKKKKKKKGAHDSQLEALIKFM | HC4 L-N |
| 28 | KKKKKKKKKKKKKKKGACHDSQLEALIKFMC | HC4 C-N |
| 29 | KKKKKKKKKKKKKKKRVRRGACHDSQLEALIKFMC | HC4 C-C |
| 30 | GADWRVIIPPRPSA | |
| 31 | GACDWRVIIPPRPSAC | |
| 32 | RVRRGACDWRVIIPPRPSAC | |

(continued)

| SE Q ID NO | Sequence | Peptide name |
|---|---|---|
| 33 | RVRRCDWRVIIPPRPSAC | |
| 34 | KKKKKKKKKKKKKKKKGADWRVIIPPRPSA | HC5 L-N |
| 35 | KKKKKKKKKKKKKKKKGACDWRVIIPPRPSAC | HC5 C-N |
| 36 | KKKKKKKKKKKKKKKKRVRRCDWRVIIPPRPSAC | HC5 C-C |
| 37 | KKKKKKKKKKKKKKKKRVRRGACDWRVIIPPRPSAC | |

The targeting peptide may comprise a cyclic region, a branched region, and/or a linear region.

The targeting peptide comprising the cyclic region may be formed by the provision of at least two cysteine residues in the peptide, thus enabling the formation of a disulphide bond. Thus, the targeting peptide may comprise two or more cysteine residues that are capable of forming one or more disulphide bond(s). Preferably, the two or more cysteine residues flank the targeting sequence. For example, if the targeting sequence is STLKQKL (SEQ ID NO: 1), the targeting peptide may comprise a sequence: CSTLKQKLC (SEQ ID NO: 38).

The targeting peptide may comprise a linker. The linker may be cleavable or non-cleavable.

The linker may comprise at least 1, at least 2, at least 3, at least 4, at least 5, at least 6, at least 7, at least 8, at least 9, at least 10, at least 11 or at least 12 amino acids. The linker may comprise 2-10 amino acids or 4-8 amino acids. The amino acids may be naturally occurring or non-naturally occurring. They may have L- or D- configuration. The amino acids may be the same or different. The use of multiple lysine residues (or other cationic amino acids suitable for use in the cargo-binding polycationic component) should generally be avoided in the linker as oligo-lysine sequences have activity as a cargo-binding polycationic component.

The linker may comprise a cleavable portion that is susceptible to cleavage within a cell. The linker that comprises a cleavable portion that is susceptible to cleavage within a cell may be susceptible to cleavage within the endosome, lysosome, and/or cytoplasm of a cell. The expression "susceptible to cleavage" refers to a linker that is susceptible to cleavage over a timescale during which the remaining elements of the targeting peptide are intact. Thus, the linker may be cleaved more rapidly than the cellular peptide-degradation pathways take effect. The cleavable portion may comprise from 3 to 6 amino acids, for example 4 amino acids. The linker may include the amino acid sequence RVRR (SEQ ID NO: 39) as a cleavable portion. The amino acid sequence RVRR is susceptible to enzymatic cleavage by the endosomal protease furin. The cleavable portion of the linker may be attached to a cargo-binding component. The cleavable portion of the linker may be cleavable by a protease. The protease may be cathepsin (e.g. serine, cysteine, aspartic-type), furin, a lysosomal protease or an endosomal protease.

The targeting peptide may comprise a spacer. The spacer may be either a peptide, that is to say, it comprises amino acid residues, or a polyethyleneglycol group, or a mixture of the two. The amino acids may be naturally occurring or non-naturally occurring. They may have L- or D-configuration. The spacer may have one or more amino acids. The spacer may comprise at least 1, at least 2, at least 3, at least 4, at least 5, at least 6, at least 7, at least 8, at least 9, at least 10, at least 11 or at least 12 amino acids.

The spacer may comprise 1-7 amino acids, preferably 2-5 amino acids. The amino acids may be the same or different. The spacer may comprise the dipeptide glycine-glycine (GG), glycine-alanine (GA) or alanine-alanine (AA). The spacer may comprise a hydrophobic spacer. The spacer may include the amino acid sequence XSX in which X is c-aminocaproic acid (c-Ahx) also known as 6-aminohexanoic acid, a synthetic, i.e. non-naturally occurring, amino acid. Aminocaproic acid functions as a hydrophobic spacer. The spacer may comprise GG, GA, AA, XSXGG, XSXGA or XSXAA. Preferably, the spacer comprises GA or GG. The spacer may be located at the end of the linker in a targeting peptide. The spacer may be attached to a targeting sequence or a targeting sequence flanked by cysteine residues. Preferably, the spacer links the linker and the targeting sequence (which is optionally flanked by the cysteine residues).

The targeting peptide may have a structure: A-B-C-D, wherein component A is a cargo-binding component, component B is a linker, component C is a spacer and component D is a targeting sequence (optionally flanked by the cysteine residues). The targeting peptide may have a structure: A-C-B-D, wherein component A is a cargo-binding component, component B is a linker, component C is a spacer and component D is a targeting sequence (optionally flanked by the cysteine residues).

The targeting peptide may have a structure: A-B-D, wherein component A is a cargo-binding component, component B is a linker and component D is a targeting sequence (optionally flanked by the cysteine residues).

The targeting peptide may have a structure: A-D, wherein component A is a cargo-binding component and component D is a targeting sequence (optionally flanked by the cysteine residues).

The nanoparticle (e.g. the non-viral transfection complex) may have a particle size of less than 500 nm, for

example less than 250 nm, less than 100 nm, less than 85, less than 75 nm, less than 65 nm, less than 50 nm, or less than 40 nm. In a population or a library of particles there will be some variation in particle size but the above criteria will be taken as met if at least 70%, at least 80% or at least 90% of the particles are of less than 500 nm, for example less than 250nm, less than 100 nm, less than 75 nm, or less than 65 nm. Preferably, in a population or a library of particles, at least 80% of the particles are less than 500 nm, for example less than 250nm, less than 100 nm, less than 85 nm, less than 75 nm, less than 65 nm, less than 50 nm, or less than 40 nm. Preferably, the nanoparticle, is a self-assembled nanoparticle. In a population or a library of self-assembled nanoparticles (e.g. non-viral transfection complexes), the size of the particles may be lower than the size of particles which were produced by methods other than self-assembly methods (e.g. methods in which the lipid component is conjugated to a targeting sequence before encapsulating of a cargo). For example, the size of the self-assembled nanoparticles may be lower by at least 5%, at least 10%, at least 15%, at least 20%, at least 25%, at least 30%, at least 35% or at least 40% than the size of particles which were produced by methods other than self-assembly methods. Preferably, the self-assembly of the nanoparticle is performed using a microfluidics device.

In a population or a library of nanoparticles described herein, each nanoparticle may have substantially the same size as at least 9, at least 99, at least 999, at least 9,999, at least 99,999 other nanoparticles in the library.

Thus, in a population or a library of nanoparticles (e.g. non-viral transfection complexes) of the present invention, the nanoparticles may be monodisperse or substantially monodisperse. The nanoparticles (e.g. the non-viral transfection complex) may have a polydispersity index (PDI) of less than 0.4, less than 0.3, less than 0.2, or less than 0.15. The nanoparticles (e.g. the non-viral transfection complex) may have a polydispersity index similar or equal to the polydispersity index of empty liposomes (control).

In a population or a library of self-assembled nanoparticles (e.g. non-viral transfection complexes), a polydispersity index may be lower than the polydispersity index of a population or a library of nanoparticles which were produced by methods other than self-assembly methods (e.g. methods in which the lipid component is conjugated to a targeting sequence before encapsulating of a cargo). The polydispersity index of a population or a library of self-assembled nanoparticles may be lower by at least 1%, at least 5%, at least 10%, at least 15%, at least 20%, at least 25%, at least 30%, at least 35%, or at least 40% than the polydispersity index of a population or a library of nanoparticles which were produced by methods other than self-assembly methods.

The polydispersity index is a measure of the heterogeneity of a sample based on size. Polydispersity can occur due to size distribution in a sample or agglomeration or aggregation of the sample during isolation or analysis. The skilled person would know different ways of determining the polydispersity index. For example, the polydispersity index can be obtained from instruments that use dynamic light scattering (DLS) or determined from electron micrographs. In general, the polydispersity index values of less than 0.3 are more common to monodisperse or substantially monodisperse samples, while values of above 0.7 are common to a broad size (e.g. polydisperse) distribution of particles.

The inventors of the present application have discovered that the generation of monodisperse or substantially monodisperse nanoparticles of the present invention is facilitated by a specific ratio of charges between a targeting peptide, a lipid component and a cargo.

Thus, the invention provides a library comprising two or more nanoparticles described herein. A library may comprise 3 or more, 4 or more, 5 or more, 6 or more, 7 or more, 8 or more 9 or more 10 or more, 20 or more, 30 or more, 40 or more, 50 or more, 60 or more, 70 or more, 80 or more, 90 or more, 100 or more, 1000 or more, 10,000 or more, 100,000 or more nanoparticles described herein. The polydispersity index (PDI) of the nanoparticles in the library may be less than about 0.2, for example between 0.2 and 0.1, preferably between 0.16 and 0.12. The polydispersity index (PDI) of the nanoparticles in the library may be between 0.15 and 0.13.

The nanoparticle (e.g. the non-viral transfection complex) may have a Nitrogen/Phosphate (N/P) molar ratio of 2.0-13.0, 2.5-10.0, 3.0-9.0, or 4.0-5.0. The nanoparticle (e.g. the non-viral transfection complex) may have a N/P ratio of about 4.5, about 4.0, about 4.2, about 4.5, about 4.7, about 5.2, about 5.5, about 5.7 about 6.5, about 7.5, or about 8.0. Preferably, the nanoparticle has a N/P ratio of 3.0-9.0.

The N/P ratio is calculated from the molar amount of each free amine group(s) (N) in the components of the nanoparticle to the phosphate groups (P) in the components of the nanoparticle. For example, the free amine group(s) may come from the targeting peptide and the lipid component and the phosphate group(s) may come from the phosphate groups in the cargo (e.g. DNA molecule) (P). The N/P ratio is typically driven by the mass of the targeting peptide. A N/P ratio of 1, for example, consists of 1 amine group to 1 phosphate and is conventionally expressed as N/P = 1. Similarly, a ratio N/P = 5 refers to the ratio between 5 amine groups to 1 phosphate group.

For example, for nanoparticles consisting of lipid, peptide and mRNA components, the N/P ratio is calculated as followed:

$$\frac{N}{P} ratio = \frac{(Moles_{lip} \times N_{lip)} + (Moles_{pep} \times N_{pep})}{(Moles_{mRNA} \times P_{mRNA})}$$

For three-component nanoparticles, the mass of each component to formulate can be calculated from the desired mass of nucleic acid to be encapsulated and the desired charge ratio of Peptide/mRNA and Lipid/mRNA.

First, P must be calculated:

$$Moles_{mRNA} = \left(\frac{Mass_{mRNA}}{MW_{mRNA}}\right)$$

$$P = Moles_{mRNA} \times Number\ of\ bases_{mRNA}$$

The number of moles required of each lipid and peptide in the final formulation can then be calculated as followed:

$$Moles_{pep} = \frac{\left(Charge\ ratio\,\frac{Peptide}{mRNA} \times P\right)}{N_{pep}}$$

$$Moles_{lip} = \frac{\left(Charge\ ratio\,\frac{Lipid}{mRNA} \times P\right)}{N_{lip}}$$

Where $N_{pep}$ is equal to the number of positively charged amino acids in the peptide sequence (Lysine, Histidine and Arginine), and $N_{lip}$ is equal to the number of free amine groups in the cationic lipid component.

Therefore, the mass of peptide or lipid to formulate in the final nanoparticle formulation can be calculated as follows:

$$Mass = Moles \times Molecular\ Weight$$

Finally, the N/P ratio of the final nanoparticle equation can be calculated using the formulation above.

The term "about" as used herein for numerical parameters refers to a value within 10% of the underlying parameter (i.e. plus or minus 10%). For example, a charge ratio of "about 4.5" can include N/P ratios between 4.1 - 5.0, including charge ratios 4.1 and 5.0.

The nanoparticle (e.g. the non-viral delivery complex) may deliver the cargo to a chondrocyte with a transfection efficiency of at least 5%, at least 10%, at least 15%, at least 20%, at least 25%, at least 30%, at least 35%, at least 40%, at least 50%, at least 52%, at least 55%, at least 60%, at least 65%, at least 70%, at least 75%, at least 80%, at least 85%, at least 90%, at least 95%, or at least 98%. Preferably, the transfection efficiency is at least 15%.

The nanoparticle of the present invention facilitates delivery of a cargo to the target cell. The improved delivery is determined, for example, by determining the transfection efficiency (i.e. the percentage of cells transfected from cells non-transfected). The nanoparticle (e.g. the non-viral delivery complex) comprising a chondrocyte targeting sequence may deliver the cargo to a chondrocyte with a transfection efficiency of at least 5%, 10%, 15%, 20%, 25%, 30%, 35%, 40%, 45% or 50%. The nanoparticle (e.g. the non-viral delivery complex) comprising a chondrocyte targeting sequence may deliver the cargo to a chondrocyte with a transfection efficiency which is at least 1 time, at least 2 times, at least 3 times, at least 4 times, at least 5 times, at least 6 times, at least 7 times, at least 8 times, at least 9 times, at least 10 times, at least 12 times, at least 14 times, at least 16 times, at least 18 times, at least 20 times, at least 22 times, at least 24 times, at least 26 times, at least 28 times, at least 30 times, at least 32 times, at least 34 times, at least 36 times, at least 38 times, at least 40 times, at least 45 times, at least 50 times, at least 55 times, at least 60 times, or at least 70 times higher than the transfection efficiency of a nanoparticle without chondrocyte targeting sequence (i.e. a nanoparticle comprising a lipid component and a cargo) or with a non-chondrocyte targeting sequence (e.g. a non- chondrocyte specific targeting sequence). Preferably, the transfection efficiency of a nanoparticle comprising a chondrocyte targeting sequence is at least 20 times higher than the transfection efficiency of a nanoparticle without a chondrocyte targeting sequence. Preferably, the transfection efficiency of a nanoparticle comprising a chondrocyte targeting sequence is at least 20 times higher than the transfection efficiency of a nanoparticle with a non-chondrocyte targeting sequence.

The skilled person is aware of different ways to determine the transfection efficiency. For example, if the cargo is a nucleic acid, the transfection efficiency may be determined by measuring or detecting the level of expression of genes encoded on the nucleic acid. For example, the nucleic acid may encode green fluorescent protein (GFP), which,

once expressed, may be detected to determine the transfection efficiency.

The presence of a chondrocyte targeting sequence improves self-assembly of a nanoparticle of the present invention. For example, the presence of a chondrocyte targeting sequence may improve the encapsulation efficiency of a cargo by a lipid component. This ensures that the method for producing nanoparticles of the present invention is very efficient. The encapsulation efficiency may be at least 75%, at least 80%, at least 90% or at least 95%. That is to say that at least 75%, at least 80%, at least 90% or at least 95% of the starting cargo is encapsulated in a lipid component. The presence of a targeting peptide may improve the encapsulation efficiency of a cargo by at least at least 10%, at least 15%, at least 20%, at least 25%, at least 30%, at least 35%, at least 40%, at least 50%, at least 52%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, at least 160%, at least 170%, at least 180%, at least 190%, at least 200%, at least 220%, at least 240%, at least 260%, at least 280%, or at least 300% as compared to the encapsulation efficiency without the presence of a targeting peptide. Preferably, the encapsulation efficiency is improved by at least 50%. The skilled person is aware of different ways of measuring the encapsulation efficiency.

The nanoparticles described herein may be administered to a patient by various methods of administration, such as by intra-articular administration, or by IV.

**2. Targeting peptides**

The invention provides a targeting peptide comprising a targeting sequence.

Preferably, the targeting sequence is a chondrocyte targeting sequence. The targeting peptide may be suitable for use in the nanoparticle (e.g. the non-viral transfection complex) described herein. The targeting peptide may comprise a cargo-binding component.

The invention provides a targeting peptide comprising:

(a) a chondrocyte targeting sequence; and
(b) a cargo-binding component.

The targeting sequence (e.g. the chondrocyte targeting sequence) may comprise at least 3, at least 4, at least 5, at least 6, at least 7, at least 8, at least 9, at least 10 amino acids, at least 11, at least 12, at least 13, or at least 14 amino acids. The targeting sequence (e.g. the chondrocyte targeting sequence) may comprise 3-30, 4-20, 5-17, 6-15 or 7-12 amino acids. Preferably, the targeting sequence comprises 7-12 amino acids. For example, the targeting sequence may comprise 7 or 12 amino acids.

The targeting sequence (e.g. chondrocyte targeting sequence) may comprise at least 3, at least 4, at least 5 or at least 6 contiguous amino acids of SEQ ID NO: 1 or a variant thereof comprising one or more conservative amino acid substitutions. The targeting sequence (e.g. the chondrocyte targeting sequence) may be SEQ ID NO: 1 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 1. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 1.

The targeting sequence (e.g. chondrocyte targeting sequence) may comprise at least 3, at least 4, at least 5 or at least 6 contiguous amino acids of SEQ ID NO: 2 or a variant thereof comprising one or more conservative amino acid substitutions. The targeting sequence (e.g. the chondrocyte targeting sequence) may be SEQ ID NO: 2 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 2. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 2.

The targeting sequence (e.g. the chondrocyte targeting sequence) may comprise at least 4, at least 5, at least 6, at least 7, at least 8, at least 9, at least 10 or at least 11 contiguous amino acids of SEQ ID NO: 3 or a variant thereof comprising one or more conservative amino acid substitutions. The targeting sequence (e.g. the chondrocyte targeting sequence) may be SEQ ID NO: 3 or a variant thereof comprising one or more conservative amino acid substitutions. The targeting sequence (e.g. the chondrocyte targeting sequence) may comprise a sequence comprising at least 60%,

at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 3. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 3.

The targeting sequence (e.g. the chondrocyte targeting sequence) may comprise at least 4, at least 5, at least 6, at least 7, at least 8, at least 9, at least 10 or at least 11 contiguous amino acids of SEQ ID NO: 4 or a variant thereof comprising one or more conservative amino acid substitutions. The targeting sequence (e.g. the chondrocyte targeting sequence) may be SEQ ID NO: 4 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 4. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 4.

The targeting sequence (e.g. the chondrocyte targeting sequence) may comprise at least 4, at least 5, at least 6, at least 7, at least 8, at least 9, at least 10 or at least 11 contiguous amino acids of SEQ ID NO: 5 or a variant thereof comprising one or more conservative amino acid substitutions. The targeting sequence (e.g. the chondrocyte targeting sequence) may be SEQ ID NO: 5 or a variant thereof comprising one or more conservative amino acid substitutions. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 5. The targeting sequence (e.g. the chondrocyte targeting sequence) may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 5.

Thus, the invention provides a targeting peptide comprising a chondrocyte targeting sequence and cargo-binding component, wherein the chondrocyte targeting sequence is SEQ ID NO: 1 or a variant thereof comprising one or more conservative amino acid substitutions.

The invention also provides a targeting peptide comprising a chondrocyte targeting sequence and cargo-binding component, wherein the chondrocyte targeting sequence is SEQ ID NO: 2 or a variant thereof comprising one or more conservative amino acid substitutions.

The invention provides a targeting peptide comprising a chondrocyte targeting sequence and cargo-binding component, wherein the chondrocyte targeting sequence is SEQ ID NO: 3 or a variant thereof comprising one or more conservative amino acid substitutions.

The invention provides a targeting peptide comprising a chondrocyte targeting sequence and cargo-binding component, wherein the chondrocyte targeting sequence is SEQ ID NO: 4 or a variant thereof comprising one or more conservative amino acid substitutions.

The invention provides a targeting peptide comprising a chondrocyte targeting sequence and cargo-binding component, wherein the chondrocyte targeting sequence is SEQ ID NO: 5 or a variant thereof comprising one or more conservative amino acid substitutions.

The targeting peptide may comprise a chondrocyte targeting sequence and a nucleic acid-binding component, wherein the chondrocyte targeting sequence is SEQ ID NO: 1 or a variant thereof comprising one or more conservative amino acid substitutions.

The targeting peptide may comprise a chondrocyte targeting sequence and a nucleic acid-binding component, wherein the chondrocyte targeting sequence is SEQ ID NO: 2 or a variant thereof comprising one or more conservative amino acid substitutions.

The targeting peptide may comprise a chondrocyte targeting sequence and a nucleic acid-binding component, wherein the chondrocyte targeting sequence is SEQ ID NO: 3 or a variant thereof comprising one or more conservative amino acid substitutions.

The targeting peptide may comprise a chondrocyte targeting sequence and a nucleic acid-binding component, wherein the chondrocyte targeting sequence is SEQ ID NO: 4 or a variant thereof comprising one or more conservative amino acid substitutions.

The targeting peptide may comprise a chondrocyte targeting sequence and a nucleic acid-binding component, wherein the chondrocyte targeting sequence is SEQ ID NO: 5 or a variant thereof comprising one or more conservative amino acid substitutions.

The targeting peptide may comprise a chondrocyte targeting sequence and a nucleic acid-binding cationic component, wherein the chondrocyte targeting sequence is SEQ ID NO: 1 or a variant thereof comprising one or more conservative amino acid substitutions.

The targeting peptide may comprise a chondrocyte targeting sequence and a nucleic acid-binding cationic component, wherein the chondrocyte targeting sequence is SEQ ID NO: 2 or a variant thereof comprising one or more conservative amino acid substitutions.

The targeting peptide may comprise a chondrocyte targeting sequence and a nucleic acid-binding cationic component, wherein the chondrocyte targeting sequence is SEQ ID NO: 3 or a variant thereof comprising one or more conservative amino acid substitutions.

The targeting peptide may comprise a chondrocyte targeting sequence and a nucleic acid-binding cationic component, wherein the chondrocyte targeting sequence is SEQ ID NO: 4 or a variant thereof comprising one or more conservative amino acid substitutions.

The targeting peptide may comprise a chondrocyte targeting sequence and a nucleic acid-binding cationic component, wherein the chondrocyte targeting sequence is SEQ ID NO: 5 or a variant thereof comprising one or more conservative amino acid substitutions.

The targeting peptide may comprise a chondrocyte targeting sequence and a nucleic acid-binding polycationic component, wherein the chondrocyte targeting sequence is SEQ ID NO: 1 or a variant thereof comprising one or more conservative amino acid substitutions.

The targeting peptide may comprise a chondrocyte targeting sequence and a nucleic acid-binding polycationic component, wherein the chondrocyte targeting sequence is SEQ ID NO: 2 or a variant thereof comprising one or more conservative amino acid substitutions.

The targeting peptide may comprise a chondrocyte targeting sequence and a nucleic acid-binding polycationic component, wherein the chondrocyte targeting sequence is SEQ ID NO: 3 or a variant thereof comprising one or more conservative amino acid substitutions.

The targeting peptide may comprise a chondrocyte targeting sequence and a nucleic acid-binding polycationic component, wherein the chondrocyte targeting sequence is SEQ ID NO: 4 or a variant thereof comprising one or more conservative amino acid substitutions.

The targeting peptide may comprise a chondrocyte targeting sequence and a nucleic acid-binding polycationic component, wherein the chondrocyte targeting sequence is SEQ ID NO: 5 or a variant thereof comprising one or more conservative amino acid substitutions.

The targeting peptide may comprise a chondrocyte targeting sequence and a closed linear DNA-binding component (e.g. a closed linear DNA-binding polycationic component), wherein the chondrocyte targeting sequence is SEQ ID NO: 1, SEQ ID NO: 2, SEQ ID NO: 3, SEQ ID NO: 4, or SEQ ID NO: 5, or a variant thereof comprising one or more conservative amino acid substitutions.

The targeting peptide may comprise a chondrocyte targeting sequence and a linear DNA-binding component (e.g. a linear DNA-binding polycationic component), wherein the chondrocyte targeting sequence is SEQ ID NO: 1, SEQ ID NO: 2, SEQ ID NO: 3, SEQ ID NO: 4, or SEQ ID NO: 5 or a variant thereof comprising one or more conservative amino acid substitutions.

The targeting peptide may comprise a chondrocyte targeting sequence and a partially closed linear DNA-binding component (e.g. a partially closed linear DNA-binding polycationic component), wherein the chondrocyte targeting sequence is SEQ ID NO: 1, SEQ ID NO: 2, SEQ ID NO: 3, SEQ ID NO: 4, or SEQ ID NO: 5 or a variant thereof comprising one or more conservative amino acid substitutions.

The targeting peptide may comprise at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40 amino acids. The targeting sequence (e.g. the chondrocyte targeting sequence) may comprise 4-100, 5-70, 6-50, 7-40, 7-15 amino acids.

The targeting peptide may comprise at least 4, at least 5, at least 6, at least 7, or at least 8 contiguous amino acids of SEQ ID NO: 6 or a variant thereof comprising one or more conservative amino acid substitutions. The targeting peptide may be SEQ ID NO: 6 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 6. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 6.

The targeting peptide may comprise at least 6, at least 7, or at least 8, at least 9, at least 10 contiguous amino acids of SEQ ID NO: 7 or a variant thereof comprising one or more conservative amino acid substitutions. The targeting peptide may be SEQ ID NO: 7 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 7. The targeting peptide may comprise a sequence

comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 7.

The targeting peptide may comprise at least 8, at least 9, at least 10, at least 11, at least 12, at least 13, or at least 14 contiguous amino acids of SEQ ID NO: 8 or a variant thereof comprising one or more conservative amino acid substitutions. The targeting peptide may be SEQ ID NO: 8 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 8. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 8.

The targeting peptide may comprise at least 18, at least 19, at least 20, at least 21, at least 22, at least 23, or at least 24 contiguous amino acids of SEQ ID NO: 9 or a variant thereof comprising one or more conservative amino acid substitutions. The targeting peptide may be SEQ ID NO: 9 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 9. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 9.

The targeting peptide may comprise at least 18, at least 19, at least 20, at least 21, at least 22, at least 23, at least 24, at least 25, or at least 26 contiguous amino acids of SEQ ID NO: 10 or a variant thereof comprising one or more conservative amino acid substitutions. The targeting peptide may be SEQ ID NO: 10 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 10. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 10.

The targeting peptide may comprise at least 20, at least 21, at least 22, at least 23, at least 24, at least 25, at least 26, at least 27, at least 28, at least 29, or at least 30 contiguous amino acids of SEQ ID NO: 11 or a variant thereof comprising one or more conservative amino acid substitutions. The targeting peptide may be SEQ ID NO: 11 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 11. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 11.

The targeting peptide may comprise at least 4, at least 5, at least 6, at least 7, or at least 8 contiguous amino acids of SEQ ID NO: 12 or a variant thereof comprising one or more conservative amino acid substitutions. The targeting peptide may be SEQ ID NO: 12 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 12. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 12.

The targeting peptide may comprise at least 6, at least 7, or at least 8, at least 9, at least 10 contiguous amino acids of SEQ ID NO: 13 or a variant thereof comprising one or more conservative amino acid substitutions. The targeting peptide may be SEQ ID NO: 13 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 13. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 13.

The targeting peptide may comprise at least 8, at least 9, at least 10, at least 11, at least 12, at least 13, or at

least 14 contiguous amino acids of SEQ ID NO: 14 or a variant thereof comprising one or more conservative amino acid substitutions. The targeting peptide may be SEQ ID NO: 14 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 14. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 14.

The targeting peptide may comprise at least 18, at least 19, at least 20, at least 21, at least 22, at least 23, or at least 24 contiguous amino acids of SEQ ID NO: 15 or a variant thereof comprising one or more conservative amino acid substitutions. The targeting peptide may be SEQ ID NO: 15 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 15. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 15.

The targeting peptide may comprise at least 18, at least 19, at least 20, at least 21, at least 22, at least 23, at least 24, at least 25, or at least 26 contiguous amino acids of SEQ ID NO: 16 or a variant thereof comprising one or more conservative amino acid substitutions. The targeting peptide may be SEQ ID NO: 16 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 16. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 16.

The targeting peptide may comprise at least 20, at least 21, at least 22, at least 23, at least 24, at least 25, at least 26, at least 27, at least 28, at least 29, or at least 30 contiguous amino acids of SEQ ID NO: 17 or a variant thereof comprising one or more conservative amino acid substitutions. The targeting peptide may be SEQ ID NO: 17 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 17. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 17.

The targeting peptide may comprise at least 9, at least 10, at least 11, at least 12, or at least 13 contiguous amino acids of SEQ ID NO: 18 or a variant thereof comprising one or more conservative amino acid substitutions. The targeting peptide may be SEQ ID NO: 18 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 18. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 18.

The targeting peptide may comprise at least 11, at least 12, or at least 13, at least 14, at least 15 contiguous amino acids of SEQ ID NO: 19 or a variant thereof comprising one or more conservative amino acid substitutions. The targeting peptide may be SEQ ID NO: 19 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 19. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 19.

The targeting peptide may comprise at least 13, at least 14, at least 15, at least 16, at least 17, at least 18, or at least 19 contiguous amino acids of SEQ ID NO: 20 or a variant thereof comprising one or more conservative amino acid substitutions. The targeting peptide may be SEQ ID NO: 20 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%,

at least 96%, at least 97%, at least 98%, at least 99% or at least 100% similarity to SEQ ID NO: 20. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 20.

The targeting peptide may comprise at least 23, at least 24, at least 25, at least 26, at least 27, at least 28, or at least 29 contiguous amino acids of SEQ ID NO: 21 or a variant thereof comprising one or more conservative amino acid substitutions. The targeting peptide may be SEQ ID NO: 21 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 21. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 21.

The targeting peptide may comprise at least 23, at least 24, at least 25, at least 26, at least 27, at least 28, at least 29, at least 30, or at least 31 contiguous amino acids of SEQ ID NO: 22 or a variant thereof comprising one or more conservative amino acid substitutions. The targeting peptide may be SEQ ID NO: 22 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 22. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 122.

The targeting peptide may comprise at least 25, at least 26, at least 27, at least 28, at least 29, at least 30, at least 31, at least 32, at least 33, at least 34, or at least 35 contiguous amino acids of SEQ ID NO: 23 or a variant thereof comprising one or more conservative amino acid substitutions. The targeting peptide may be SEQ ID NO: 23 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 23. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 23.

The targeting peptide may comprise at least 9, at least 10, at least 11, at least 12, or at least 13 contiguous amino acids of SEQ ID NO: 24 or a variant thereof comprising one or more conservative amino acid substitutions. The targeting peptide may be SEQ ID NO: 24 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 24. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 24.

The targeting peptide may comprise at least 11, at least 12, or at least 13, at least 14, at least 15 contiguous amino acids of SEQ ID NO: 25 or a variant thereof comprising one or more conservative amino acid substitutions. The targeting peptide may be SEQ ID NO: 25 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 25. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 25.

The targeting peptide may comprise at least 13, at least 14, at least 15, at least 16, at least 17, at least 18, or at least 19 contiguous amino acids of SEQ ID NO: 26 or a variant thereof comprising one or more conservative amino acid substitutions. The targeting peptide may be SEQ ID NO: 26 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 26. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 26.

The targeting peptide may comprise at least 23, at least 24, at least 25, at least 26, at least 27, at least 28, or at least 29 contiguous amino acids of SEQ ID NO: 27 or a variant thereof comprising one or more conservative amino acid substitutions. The targeting peptide may be SEQ ID NO: 27 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 27. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 27.

The targeting peptide may comprise at least 23, at least 24, at least 25, at least 26, at least 27, at least 28, at least 29, at least 30, or at least 31 contiguous amino acids of SEQ ID NO: 28 or a variant thereof comprising one or more conservative amino acid substitutions. The targeting peptide may be SEQ ID NO: 28 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 28. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 28.

The targeting peptide may comprise at least 25, at least 26, at least 27, at least 28, at least 29, at least 30, at least 31, at least 32, at least 33, at least 34, or at least 35 contiguous amino acids of SEQ ID NO: 29 or a variant thereof comprising one or more conservative amino acid substitutions. The targeting peptide may be SEQ ID NO: 29 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 29. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 29.

The targeting peptide may comprise at least 9, at least 10, at least 11, at least 12, or at least 13 contiguous amino acids of SEQ ID NO: 30 or a variant thereof comprising one or more conservative amino acid substitutions. The targeting peptide may be SEQ ID NO: 30 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 30. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 30.

The targeting peptide may comprise at least 11, at least 12, or at least 13, at least 14, at least 15 contiguous amino acids of SEQ ID NO: 31 or a variant thereof comprising one or more conservative amino acid substitutions. The targeting peptide may be SEQ ID NO: 31 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 31. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 31.

The targeting peptide may comprise at least 13, at least 14, at least 15, at least 16, at least 17, at least 18, or at least 19 contiguous amino acids of SEQ ID NO: 32 or a variant thereof comprising one or more conservative amino acid substitutions. The targeting peptide may be SEQ ID NO: 32 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 32. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 32.

The targeting peptide may comprise at least 13, at least 14, at least 15, at least 16, or at least 17 contiguous amino acids of SEQ ID NO: 33 or a variant thereof comprising one or more conservative amino acid substitutions. The targeting peptide may be SEQ ID NO: 33 or a variant thereof comprising one or more conservative amino acid substitutions. The targeting peptide may comprise a sequence comprising at least 60%, at least 70%, at least 75%, at least

80%, at least 85%, at least 90%, at least 91%, at least 92%, at least 93%, at least 94%, at least 95%, at least 96%, at least 97%, at least 98%, at least 99% or at least 100% similarity to SEQ ID NO: 33. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 33.

The targeting peptide may comprise at least 23, at least 24, at least 25, at least 26, at least 27, at least 28, or at least 29 contiguous amino acids of SEQ ID NO: 34 or a variant thereof comprising one or more conservative amino acid substitutions. The targeting peptide may be SEQ ID NO: 34 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 34. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 34.

The targeting peptide may comprise at least 23, at least 24, at least 25, at least 26, at least 27, at least 28, at least 29, at least 30, or at least 31 contiguous amino acids of SEQ ID NO: 35 or a variant thereof comprising one or more conservative amino acid substitutions. The targeting peptide may be SEQ ID NO: 35 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 35. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 35.

The targeting peptide may comprise at least 25, at least 26, at least 27, at least 28, at least 29, at least 30, at least 31, at least 32, or at least 33 contiguous amino acids of SEQ ID NO: 36 or a variant thereof comprising one or more conservative amino acid substitutions. The targeting peptide may be SEQ ID NO: 36 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 36. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 36.

The targeting peptide may comprise at least 25, at least 26, at least 27, at least 28, at least 29, at least 30, at least 31, at least 32, at least 33, at least 34, or at least 35 contiguous amino acids of SEQ ID NO: 37 or a variant thereof comprising one or more conservative amino acid substitutions. The targeting peptide may be SEQ ID NO: 37 or a variant thereof comprising one or more conservative amino acid substitutions. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% similarity to SEQ ID NO: 37. The targeting peptide may comprise a sequence comprising at least 60%, at least 70%, at least 75%, at least 80%, at least 85%, at least 90%, at least 91%, at least 92%, at least 93%, at least 94%, at least 95%, at least 96%, at least 97%, at least 98%, at least 99% or at least 100% identity to SEQ ID NO: 37.

The polylysine chain in SEQ ID NOs: 9-11, 15-17, 21-23, 27-29 and 34-37 may be a polylysine chain of two or more lysine residues. For example, the polylysine chain may be of 8, 16 or 32 lysine residues.

The position of the "GA" spacer in SEQ ID Nos: 8, 11, 14, 17, 20, 23, 26, 29, 32, and 37 can be before or after the "RVRR" linker residues. The "GA" spacer may be substituted with a "GG" spacer of an "AA" spacer in any of the above sequences.

By "sequence identity" or "sequence similarity" is meant that the identity or similarity, respectively, between two or more amino acid sequences, or two or more nucleotide sequences, is expressed in terms of the identity or similarity between the sequences. Sequence identity can be measured in terms of "percentage (%) identity," in which a higher percentage indicates greater identity shared between the sequences. Sequence similarity can be measured in terms of percentage similarity (which takes into account conservative amino acid substitutions); the higher the percentage, the more similarity shared between the sequences.

The targeting peptides described herein may comprise conservative amino acid substitutions at one or more amino acid residues, e.g. at essential or non-essential amino acid residues. A "conservative amino acid substitution" is one in which the amino acid residue is replaced with an amino acid residue having a similar side chain. Families of amino acid residues having similar side chains have been defined in the art, including basic side chains (e.g., lysine, arginine, histidine), acidic side chains (e.g., aspartic acid, glutamic acid), uncharged polar side chains (e.g., glycine, asparagines, glutamine, serine, threonine, tyrosine, cysteine), nonpolar side chains (e.g., alanine, valine, leucine, isoleucine, proline,

phenylalanine, methionine, tryptophan), beta-branched side chains (e.g., threonine, valine, isoleucine) and aromatic side chains (e.g., tyrosine, phenylalanine, tryptophan, histidine).

The targeting peptide may comprise a cyclic region, a branched region, and/or a linear region.

The targeting peptide comprising the cyclic region may be formed by the provision of at least two cysteine residues in the peptide, thus enabling the formation of a disulphide bond. Thus, the targeting peptide may comprise two or more cysteine residues that are capable of forming one or more disulphide bond(s). Preferably, the two or more cysteine residues flank the targeting sequence. For example, if the targeting sequence is SEQ ID NO: 1, the targeting peptide may comprise a sequence of SEQ ID NO: 38.

The targeting peptide may comprise a linker. The linker may be cleavable or non-cleavable.

The invention provides a targeting peptide comprising:

(a) a chondrocyte targeting sequence; and
(b) a linker.

The invention provides a targeting peptide comprising:

(a) a chondrocyte targeting sequence;
(b) a linker; and
(c) a cargo-binding component.

The linker may comprise at least 1, at least 2, at least 3, at least 4, at least 5, at least 6, at least 7, at least 8, at least 9, at least 10, at least 11 or at least 12 amino acids. The linker may comprise 2-10 amino acids or 4-8 amino acids. The amino acids may be naturally occurring or non-naturally occurring. They may have L- or D- configuration. The amino acids may be the same or different. The use of multiple lysine residues (or other cationic amino acids suitable for use in the cargo-binding polycationic component) should generally be avoided in the linker as oligo-lysine sequences have activity as a cargo-binding polycationic component.

The linker may comprise a cleavable portion that is susceptible to cleavage within a cell. The linker that comprises a cleavable portion that is susceptible to cleavage within a cell may be susceptible to cleavage within the endosome, lysosome, and/or cytoplasm of a cell. The expression "susceptible to cleavage" refers to a linker that is susceptible to cleavage over a timescale during which the remaining elements on the targeting peptide are intact. Thus, the linker may be cleaved more rapidly than the cellular peptide-degradation pathways take effect. The cleavable portion may comprise from 3 to 6 amino acids, for example 4 amino acids. The linker may include the amino acid sequence RVRR (SEQ ID NO: 39) as a cleavable portion. The amino acid sequence RVRR is susceptible to enzymatic cleavage by the endosomal protease furin. The cleavable portion of the linker may be attached to a cargo-binding component. The cleavable portion of the linker may be cleavable by a protease. The protease may be cathepsin (e.g. serine, cysteine, aspartic-type), furin, a lysosomal protease or an endosomal protease.

The targeting peptide may comprise a spacer. The spacer may be either a peptide, that is to say, it comprises amino acid residues, or a polyethyleneglycol group, or a mixture of the two. The amino acids may be naturally occurring or non-naturally occurring. They may have L- or D-configuration. The spacer may have one or more amino acids. The spacer may comprise at least 1, at least 2, at least 3, at least 4, at least 5, at least 6, at least 7, at least 8, at least 9, at least 10, at least 11 or at least 12 amino acids.

The spacer may comprise 1-7 amino acids, preferably 2-5 amino acids. The amino acids may be the same or different. The spacer may comprise the dipeptide glycine-glycine (GG), glycine-alanine (GA) or alanine-alanine (AA). The spacer may comprise a hydrophobic spacer. The spacer may include the amino acid sequence XSX in which X is c-aminocaproic acid (c-Ahx) also known as 6-aminohexanoic acid, a synthetic, i.e. non-naturally occurring, amino acid. Aminocaproic acid functions as a hydrophobic spacer. The spacer may comprise GG, GA, AA, XSXGG, XSXGA or XSXAA. Preferably, the spacer comprises GA or GG. The spacer may be located at the end of the linker in a targeting peptide. The spacer may be attached to a targeting sequence or a targeting sequence flanked by cysteine residues. Preferably, the spacer links the linker and the targeting sequence (which is optionally flanked by the cysteine residues).

Thus, the invention provides a targeting peptide comprising:

(a) a chondrocyte targeting sequence;
(b) a linker; and
(c) a spacer.

Thus, the invention provides a targeting peptide comprising:

(a) a chondrocyte targeting sequence;

(b) a linker; and
(c) a cargo-binding component.

The invention provides a targeting peptide comprising:

(a) a chondrocyte targeting sequence;
(b) a linker
(c) a spacer; and
(d) a cargo-binding component.

The targeting peptide may have a structure: A-B-C-D, wherein component A is a cargo-binding component, component B is a linker, component C is a spacer and component D is a targeting sequence (optionally flanked by the cysteine residues). The targeting peptide may have a structure: A-C-B-D, wherein component A is a cargo-binding component, component B is a linker, component C is a spacer and component D is a targeting sequence (optionally flanked by the cysteine residues).

The targeting peptide may have a structure: A-B-D, wherein component A is a cargo-binding component, component B is a linker and component D is a targeting sequence (optionally flanked by the cysteine residues).

The targeting peptide may have a structure: A-D, wherein component A is a cargo-binding component and component D is a targeting sequence (optionally flanked by the cysteine residues).

The cargo-binding component may be a cargo-binding cationic component, a cargo-binding neutral component or a cargo-binding anionic component. The cationic component, the anionic component, and/or the neutral component may be used to establish a desired charge (i.e. a negative/positive ratio) of the nanoparticle. A specific charge (i.e. a negative/positive ratio) may be required to facilitate or enhance cell transfection.

The cargo-binding component may be a biomolecule-binding component. The biomolecule-binding component may be a biomolecule-binding cationic component, a biomolecule-binding neutral component or a biomolecule-binding anionic component. The cargo-binding component may be a small molecule-binding component. The small molecule-binding component may be a small molecule-binding cationic component, a small molecule-binding neutral component or a small molecule-binding anionic component.

The cargo-binding component may be a nucleic acid-binding component. The nucleic acid-binding component may be a nucleic acid-binding cationic component, or a nucleic acid-binding neutral component.

The nucleic acid-binding component may be a DNA-binding component. The DNA-binding component may be a DNA-binding cationic component or a DNA-binding neutral component. The nucleic acid-binding component may be a closed linear DNA-binding component. The closed linear DNA-binding component may be a closed linear DNA-binding cationic component or a closed linear DNA-binding neutral component. The nucleic acid-binding component may be a partially closed linear DNA-binding component. The partially closed linear DNA-binding component may be a partially closed linear DNA-binding cationic component or a partially closed linear DNA-binding neutral component. The nucleic acid-binding component may be an RNA-binding component. The RNA-binding component may be an RNA-binding cationic component, or an RNA-binding neutral component. The nucleic acid-binding component may be an antisense RNA-binding component. The antisense RNA-binding component may be an antisense RNA-binding cationic component, or an antisense RNA-binding neutral component. The nucleic acid-binding component may be a siRNA-binding component. The siRNA-binding component may be a siRNA-binding cationic component, or a siRNA-binding neutral component. The nucleic acid-binding component may be an mRNA-binding component. The mRNA-binding component may be an mRNA-binding cationic component, or an mRNA-binding neutral component. The nucleic acid-binding component may be a transfer RNA-binding component. The transfer RNA-binding component may be a transfer RNA-binding cationic component, or a transfer RNA-binding neutral component. The nucleic acid-binding component may be a ribosomal RNA-binding component. The ribosomal RNA-binding component may be a ribosomal RNA-binding cationic component, or a ribosomal RNA-binding neutral component. The nucleic acid-binding component may be an snRNA-binding component. The snRNA-binding component may be an snRNA-binding cationic component, or an snRNA-binding neutral component. The nucleic acid-binding component may be a double-stranded RNA-binding component. The double-stranded RNA-binding component may be a double-stranded RNA-binding cationic component, or a double-stranded RNA-binding neutral component. The nucleic acid-binding component may be an miRNA-binding component. The miRNA-binding component may be an miRNA-binding cationic component, or an miRNA-binding neutral component. The nucleic acid-binding component may be a shRNA-binding component. The shRNA-binding component may be a shRNA-binding cationic component, or a shRNA-binding neutral component. The nucleic acid-binding component may be a gRNA-binding component. The gRNA-binding component may be a gRNA-binding cationic component, or a gRNA-binding neutral component. The nucleic acid-binding component may be a samRNA-binding component. The samRNA-binding component may be a samRNA-binding cationic component, or a samRNA-binding neutral component. The nucleic acid-binding component may be a circular RNA-binding component. The circular RNA-binding component may

be a circular RNA-binding cationic component, or a circular RNA-binding neutral component.

The cargo-binding component may be a protein-binding component. The protein-binding component may be a protein-binding cationic component, a protein-binding neutral component or a protein-binding anionic component. The protein-binding component may be a Cas9-binding component. The Cas9 binding component may be a Cas9-binding cationic component, a Cas9-binding neutral component or a Cas9-binding anionic component.

The cargo-binding component may be a peptide-binding component. The peptide-binding component may be a peptide-binding cationic component, a peptide-binding neutral component or a peptide-binding anionic component.

The cargo-binding component may be a polypeptide-binding component. The polypeptide-binding component may be a polypeptide-binding cationic component, a polypeptide-binding neutral component or a polypeptide-binding anionic component.

If the cargo is a nucleic acid, the targeting peptide may comprise a nucleic acid-binding cationic component or a nucleic acid- binding neutral component. Thus, the targeting peptide may comprise:

(a) a chondrocyte targeting sequence; and
(b) a nucleic acid-binding component.

Preferably, the nucleic acid-binding component is a nucleic acid-binding cationic component (e.g. DNA-binding cationic component).

The cargo-binding cationic component may be a cargo-binding polycationic component, The cargo-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 cationic monomers. Preferably, the cargo-binding polycationic component comprises at least 16 or at least 30 cationic monomers.

The cargo-binding cationic component may comprise a lysine, a histidine, or an arginine. The cargo-binding polycationic component may comprise a lysine, a histidine, or an arginine. The cargo-binding polycationic component may comprise an oligolysine (linear or branched), an oligohistidine (linear or branched) or an oligoarginine (linear or branched). For example, the cargo-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the cargo-binding polycationic component comprises at least 16, at least 17 or at least 30 lysine residues. More preferably still, the cargo-binding polycationic component comprises at least 17 lysine residues. The cargo-binding polycationic component may be linear or branched. For example, the cargo-binding linear polycationic component may comprise at least 17 lysine residues. The cargo-binding branched polycationic component may comprise at least 17 lysine residues. The cargo-binding polycationic component may comprise less than 10, or less than 9 lysine residues. The cargo-binding polycationic component may comprise 8 lysine residues.

The cargo-binding anionic component maybe be a cargo-binding polyanionic component, The cargo-binding polyanionic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 anionic monomers. The cargo-binding polyanionic component may be linear or branched. For example, the cargo-binding linear polyanionic component may comprise at least 17 monomers. The cargo-binding branched polyanionic component may comprise at least 17 monomers.

The cargo-binding neutral component maybe be a cargo-binding polyneutral component, The cargo-binding polyneutral component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 neutral monomers. The cargo-binding polyneutral component may be linear or branched. For example, the cargo-binding linear polyneutral component may comprise at least 17 monomers. The cargo-binding branched polyneutral component may comprise at least 17 monomers.

The nucleic acid-binding cationic component (e.g. the DNA-binding cationic component) may be a nucleic acid-binding polycationic component (e.g. a DNA-binding polycationic component). The nucleic acid-binding polycationic component may comprise a lysine. The nucleic acid-binding polycationic component may comprise an oligolysine. The

nucleic acid-binding polycationic component may comprise at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 32, at least 34, at least 36, at least 38, at least 40, at least 45, at least 50, at least 55, at least 60, at least 65, at least 70, at least 75, at least 80, at least 85, at least 90, at least 95, or at least 100 lysine residues. Preferably, the nucleic acid-binding polycationic component comprises at least 16, at least 17, or at least 30 lysine residues. More preferably still, the cargo-binding polycationic component comprises at least 17 lysine residues. The nucleic acid-binding polycationic component may be linear or branched. For example, the nucleic acid-binding linear polycationic component may comprise at least 17 lysine residues. The nucleic acid-binding branched polycationic component may comprise at least 17 lysine residues. The nucleic acid-binding polycationic component may comprise less than 10 or less than 9 lysine residues. The nucleic acid-binding polycationic component may comprise 8 lysine residues.

The cargo-binding component may be linear or branched. The cargo-binding polycationic component may be linear or branched. The cargo-binding polyanionic component may be linear or branched. The cargo-binding polyneutral component may be linear or branched. For example, the cargo-binding polycationic component may comprise at least 16, at least 17, or at least 30 lysine residues in a linear chain. Alternatively, the cargo-binding polycationic component may comprise at least 16, at least 17, or at least 30 lysine residues in a branched chain. The cargo may be a nucleic acid-binding polycationic component. The nucleic acid-binding polycationic component may be linear or branched. For example, the nucleic acid-binding polycationic component (e.g. the DNA-binding polycationic component) may comprise at least 16, at least 17, or at least 30 lysine residues in a linear chain. Alternatively, the nucleic acid-binding polycationic component (e.g. the DNA-binding polycationic component) may comprise at least 16, at least 17, or at least 30 lysine residues in a branched chain.

Thus, the targeting peptide may comprise a chondrocyte targeting sequence and a nucleic acid-binding component. The targeting peptide may comprise a chondrocyte targeting sequence and a nucleic acid-binding cationic component. The targeting peptide may comprise a chondrocyte targeting sequence and a nucleic acid-binding polycationic component (e.g. DNA-binding polycationic component). The targeting peptide may comprise a chondrocyte targeting sequence and a closed linear DNA-binding component (e.g. a closed linear DNA-binding polycationic component). The targeting peptide may comprise a chondrocyte targeting sequence and a linear DNA-binding component (e.g. a linear DNA-binding polycationic component). The targeting peptide may comprise a chondrocyte targeting sequence and a partially closed linear DNA-binding component (e.g. a partially closed linear DNA-binding polycationic component).

The targeting peptide may comprise:

(a) a chondrocyte targeting sequence;
(b) a spacer; and
(c) a nucleic acid-binding component.

The targeting peptide may comprise a chondrocyte targeting sequence, a spacer, and a nucleic acid-binding cationic component. The targeting peptide may comprise a chondrocyte targeting sequence, a spacer, and a nucleic acid-binding polycationic component. The targeting peptide may comprise a chondrocyte targeting sequence, a spacer, and a closed linear DNA-binding component (e.g. a closed linear DNA-binding polycationic component). The targeting peptide may comprise a chondrocyte targeting sequence, a spacer, and a linear DNA-binding component (e.g. a linear DNA-binding polycationic component). The targeting peptide may comprise a chondrocyte targeting sequence, a spacer, and a partially closed linear DNA-binding component (e.g. a partially closed linear DNA-binding polycationic component).

The targeting peptide may comprise:

(a) a chondrocyte targeting sequence;
(b) a linker; and
(c) a nucleic acid-binding component.

The targeting peptide may comprise a chondrocyte targeting sequence, a linker, and a nucleic acid-binding cationic component. The targeting peptide may comprise a chondrocyte targeting sequence, a linker, and a nucleic acid-binding polycationic component. The targeting peptide may comprise a chondrocyte targeting sequence, a linker, and a closed linear DNA-binding component (e.g. a closed linear DNA-binding polycationic component). The targeting peptide may comprise a chondrocyte targeting sequence, a linker, and a linear DNA-binding component (e.g. a linear DNA-binding polycationic component). The targeting peptide may comprise a chondrocyte targeting sequence, a linker, and a partially closed linear DNA-binding component (e.g. a partially closed linear DNA-binding polycationic component).

The targeting peptide may comprise:

(a) a chondrocyte targeting sequence;

(b) a linker
(c) a spacer; and
(d) a nucleic acid-binding component.

The targeting peptide may comprise a chondrocyte targeting sequence, a linker, a spacer, and a nucleic acid-binding cationic component. The targeting peptide may comprise a chondrocyte targeting sequence, a linker, a spacer, and a nucleic acid-binding polycationic component. The targeting peptide may comprise a chondrocyte targeting sequence, a linker, a spacer, and a closed linear DNA-binding component (e.g. a closed linear DNA-binding polycationic component). The targeting peptide may comprise a chondrocyte targeting sequence, a linker, a spacer, and a linear DNA-binding component (e.g. a linear DNA-binding polycationic component). The targeting peptide may comprise a chondrocyte targeting sequence, a linker, a spacer, and a partially closed linear DNA-binding component (e.g. a partially closed linear DNA-binding polycationic component).

Preferably, the chondrocyte targeting sequence is SEQ ID NO: 1, SEQ ID NO: 2, SEQ ID NO: 3, SEQ ID NO: 4, or SEQ ID NO: 5. Thus, the targeting peptide may comprise:

(a) a chondrocyte targeting sequence, wherein the chondrocyte targeting sequence is: SEQ ID NO: 1, SEQ ID NO: 2, SEQ ID NO: 3, SEQ ID NO: 4, or SEQ ID NO: 5; and
(b) a nucleic acid-binding component (e.g. a DNA-binding polycationic component).

The targeting peptide may comprise:

(a) a chondrocyte targeting sequence, wherein the chondrocyte targeting sequence is: SEQ ID NO: 1, SEQ ID NO: 2, SEQ ID NO: 3, SEQ ID NO: 4, or SEQ ID NO: 5;
(b) a spacer; and
(c) a nucleic acid-binding component (e.g. a DNA-binding polycationic component).

The targeting peptide may comprise:

(a) a chondrocyte targeting sequence, wherein the chondrocyte targeting sequence is: SEQ ID NO: 1, SEQ ID NO: 2, SEQ ID NO: 3, SEQ ID NO: 4, or SEQ ID NO: 5;
(b) a linker; and
(c) a nucleic acid-binding component (e.g. a DNA-binding polycationic component).

The targeting peptide may comprise:

(a) a chondrocyte targeting sequence, wherein the chondrocyte targeting sequence is: SEQ ID NO: 1, SEQ ID NO: 2, SEQ ID NO: 3, SEQ ID NO: 4, or SEQ ID NO: 5;
(b) a linker;
(c) a spacer; and
(d) a nucleic acid-binding component (e.g. a DNA-binding polycationic component).

The targeting peptide may comprise:

(a) a chondrocyte targeting sequence, wherein the chondrocyte targeting sequence is: SEQ ID NO: 1, SEQ ID NO: 2, SEQ ID NO: 3, SEQ ID NO: 4, or SEQ ID NO: 5; and
(b) a spacer.

The targeting peptide may comprise:

(a) a chondrocyte targeting sequence, wherein the chondrocyte targeting sequence is: SEQ ID NO: 1, SEQ ID NO: 2, SEQ ID NO: 3, SEQ ID NO: 4, or SEQ ID NO: 5; and
(b) a linker.

The targeting peptide may comprise:

(a) a chondrocyte targeting sequence, wherein the chondrocyte targeting sequence is: SEQ ID NO: 1, SEQ ID NO: 2, SEQ ID NO: 3, SEQ ID NO: 4, or SEQ ID NO: 5;
(b) a linker; and

(c) a spacer.

### 3. Nucleic acid cargos

The cargo suitable for use in the nanoparticle (e.g. non-viral transfection complex) may be any biomolecule or a small molecule. For example, the cargo may be a nucleic acid, a peptide, a polypeptide, or a protein. The cargo may be a fragment of a nucleic acid, a peptide, a polypeptide, or a protein.

The nucleic acid may be a DNA molecule or an RNA molecule. The DNA molecule may be a linear DNA molecule or circular DNA molecule. The nucleic acid may be single-stranded, double-stranded, or partially single-stranded and partially double-stranded.

The nucleic acid may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 nucleotides. Preferably, the nucleic acid comprises at least 500 nucleotides.

The nucleic acid may comprise at least 5, at least 10, at least 15, at least 20, at least 25, at least 30, at least 40, at least 45, at least 50, at least 100, at least 200, at least 300, at least 400, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the nucleic acid comprises at least 500 base pairs.

The circular DNA molecule may be a plasmid DNA, a vector DNA, a cosmid, an isolated DNA, a bacterial artificial chromosome, a minicircle, or a mini intronic plasmid (MIP). The circular DNA may be an enzymatically produced circular DNA molecule. For example, (i) a circular DNA molecule obtained from recombinase reaction (e.g. Cre recombinase reaction), or (ii) a circular DNA molecule obtained from ligase reaction (e.g. using the golden gate assembly).

The linear DNA molecule may be a portion of a chromosome or a gene. The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides). The linear double-stranded DNA molecule may comprise a first adaptor molecule at a first end and a second adaptor molecule at a second end. The first adaptor molecule and the second adaptor may comprise one or more protected nucleotides (i.e. nucleotides resistant to nuclease (e.g. exonuclease) digestion). The presence of protected nucleotides may confer resistance to nuclease (e.g. exonuclease) digestion. The linear double-stranded DNA molecule may be a DNA molecule having a double-stranded portion comprising a double stranded linear adaptor comprising protected nucleotides (i.e. nucleotides resistant to nuclease (e.g. exonuclease) digestion) at a first end and a double stranded linear adaptor comprising protected nucleotides (i.e. nucleotides resistant to nuclease (e.g. exonuclease) digestion) at a second end.

The linear DNA molecule may be a closed linear DNA molecule. The closed linear DNA molecule may comprise a double-stranded DNA portion that is closed at a first end by a first single-stranded portion (i.e. it may comprise a first hairpin at the first end) and closed at a second end by a second single-stranded portion (i.e. it may comprise a second hairpin at the second end). The closed DNA molecule may be a covalently-closed linear DNA molecule. The covalently-closed linear DNA molecule may comprise a first adaptor molecule at a first end and a second adaptor molecule at a second end. The first adaptor molecule and the second adaptor molecule may each comprise a hairpin. The hairpin may confer resistance to nuclease (e.g. exonuclease) digestion. The closed linear DNA molecule may comprise one or more protected nucleotides (i.e. nucleotides resistant to nuclease (e.g. exonuclease) digestion). The closed linear DNA molecule may be (i) a DNA molecule processed with TelN protelomerase; or (ii) a DNA molecule having a double-stranded portion closed at a first end by ligation of a first adaptor to the first end and closed at a second end by the ligation of a second adaptor to the second end.

The linear DNA molecule be a partially closed linear DNA molecule. The partially closed linear DNA molecule may comprise a double-stranded DNA portion that is closed at a first end and open at a second end. The partially closed linear DNA molecule may comprise a double-stranded DNA portion that is closed at a first end by a single-stranded portion (i.e. it may comprise a first hairpin at the first end) and open at a second end. The partially closed linear DNA molecule may comprise one or more nuclease-resistant nucleotides in an open-end region adjacent to the second end. The open-end region adjacent to the second end may be at the 3' end or 5' end of the molecule. The open-end region adjacent to the second end may comprise at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, or at least 50 nucleotides located at the second end of the partially closed linear DNA molecule. This is to say that the open-end region adjacent to the

second end may comprise any nucleotide between and including the end nucleotide of the second end and a nucleotide at location 2, 3, 4, 5, 6, 7, 8, 9, 10, 11, 12, 13, 14, 15, 16, 17, 18, 19, 20, 25, 30, 35, 40, 45, or 50 counting from the end nucleotide of the second end.

The open-end region adjacent to the second end may comprise a sense strand and an antisense strand. The open-end region adjacent to the second end may comprise one or more nuclease-resistant nucleotides in the sense strand or the antisense strand. The open-end region adjacent to the second end may comprise one or more nuclease-resistant nucleotides in both the sense and antisense strand. The open-end region adjacent to the second end may comprise two or more, three or more, four or more, or five or more nuclease-resistant nucleotides in both the sense and antisense strand. Preferably, the open-end region adjacent to the second end comprises five nuclease-resistant nucleotides in both the sense and antisense strand.

The partially closed linear DNA molecule may comprise a hairpin-loop at the 5' end or the 3' end. The partially closed linear DNA molecule may comprise a first adaptor molecule at a first end and a second adaptor molecule at a second end. The first adaptor molecule may comprise a hairpin and a second adaptor may comprise one or more protected nucleotides (i.e. nucleotides resistant to nuclease (e.g. exonuclease) digestion). The hairpin may confer resistance to nuclease (e.g. exonuclease) digestion. The presence of protected nucleotides may confer resistance to nuclease (e.g. exonuclease) digestion. The partially closed linear DNA molecule may be a DNA molecule having a double-stranded portion closed at a first end by ligation of a first adaptor (e.g. hairpin adaptor) to the first end and comprising at a second end a double stranded linear adaptor comprising protected nucleotides (i.e. nucleotides resistant to nuclease (e.g. exonuclease) digestion).

The partially closed linear DNA molecule may comprise (i) a cassette, wherein the cassette comprises a sense strand and an antisense strand; and (ii) one or more nuclease-resistant nucleotides in an open-end region of the partially closed linear DNA molecule, wherein the open-end region is 5' of the sense strand of the cassette. The partially closed linear DNA molecule may comprise (i) a cassette, wherein the cassette comprises a sense strand and an antisense strand; and (ii) one or more nuclease-resistant nucleotides in an open-end region of the partially closed linear DNA molecule, wherein the open-end region is 5' of the antisense strand of the cassette. The partially closed linear DNA molecule may comprise (i) a cassette, wherein the cassette comprises a sense strand and an antisense strand; and (ii) one or more nuclease-resistant nucleotides in an open-end region of the partially closed linear DNA molecule, wherein the open-end region is 3' of the sense strand of the cassette. The partially closed linear DNA molecule may comprise (i) a cassette, wherein the cassette comprises a sense strand and an antisense strand; and (ii) one or more nuclease-resistant nucleotides in an open-end region of the partially closed linear DNA molecule, wherein the open-end region is 3' of the antisense strand of the cassette.

The partially closed linear DNA molecule may comprise (i) a cassette, wherein the cassette comprises a sense strand and an antisense strand; (ii) one or more nuclease-resistant nucleotides in an open-end region of the partially closed linear DNA molecule, wherein the open-end region is 5' of the sense strand of the cassette; and (iii) one or more nuclease-resistant nucleotides in an open-end region of the partially closed linear DNA molecule, wherein the open-end region is 3' of the antisense strand of the cassette.

The partially closed linear DNA molecule may comprise (i) a cassette, wherein the cassette comprises a sense strand and an antisense strand; (ii) one or more nuclease-resistant nucleotides in an open-end region of the partially closed linear DNA, wherein the open-end region is 3' of the sense strand of the cassette; and (iii) one or more nuclease-resistant nucleotides in an open-end region of the partially closed linear DNA molecule, wherein the open-end region is 5' of the antisense strand of the cassette.

The closed linear DNA molecule has particular utility as a therapeutic agent (i.e. DNA therapeutic) which can be used to express a gene product in vivo. This is because its closed structure (e.g. covalently closed structure) prevents attack by enzymes such as exonucleases, leading to enhanced stability and longevity of gene expression as compared to "open" DNA molecules with exposed DNA ends. Linear double-stranded open-ended cassettes have been demonstrated to be inefficient with respect to gene expression when introduced into host tissue. This has been attributed to cassette instability due to the action of exonucleases in the extracellular space.

Sequestering DNA ends inside closed structures also has other advantages. The DNA ends are prevented from integrating with genomic DNA and so closed linear DNA molecules offer improved safety. In addition, the closed linear structure reduces concatamerisation of DNA molecules inside host cells and thus expression levels of the gene product can be regulated in a more sensitive manner.

The linear DNA molecule (e.g. the linear double-stranded DNA molecule, the closed linear DNA or the partially closed linear DNA molecule) may comprise a cassette. Thus, the linear DNA molecule (e.g. the linear double-stranded DNA molecule, the closed linear DNA or the partially closed linear DNA molecule) may comprise a sense strand and an antisense strand, wherein the linear DNA molecule comprises a single cassette and one or more protected (e.g. phosphorothioated) nucleotides at internal positions in each strand (optionally wherein the linear DNA molecule comprises one or more protected (e.g. phosphorothioated) nucleotides at internal positions in each strand outside of the cassette).

The term "single cassette" as used herein in the context of a linear DNA molecule is intended to encompass a

linear DNA molecule that does not comprise or consist of a plurality of cassettes. That is to say that the linear DNA molecule comprises only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The linear DNA molecule (e.g. the linear double-stranded DNA molecule, the closed linear DNA or the partially closed linear DNA molecule) may comprise a cassette. The cassette may comprise a coding sequence. The coding sequence may encode a gene of interest, for example a gene encoding a protein. The cassette may comprise at least a portion of a promoter and a coding sequence. The cassette may comprise a promoter and a coding sequence. The cassette may comprise a promoter, a coding sequence, a ribosomal binding site and a translational termination sequence. The cassette may additionally comprise sequences aiding protein expression, such as a cap-independent translation element. The cassette may comprise (or encode) a repair template (or editing template). The repair template (or editing template) may be for use in CRISPR-Cas mediated homology directed repair (HDR). The cassette may encode CRISPR guide RNA. The cassette may be a mammalian expression cassette. The promoter may be a CMV promoter. The cassette may further comprise an enhancer. The cassette may further comprise a reporter gene, such as an eGFP reporter gene or a luciferase reporter gene. The cassette may further comprise a homopolymeric sequence, such as a polyA, poly C, polyT or polyG sequence. The homopolymeric sequence may be between 3-200 nucleotides in length. The homopolymeric sequence may be used to facilitate purification of the cassette, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, 20, 30, 40, 50, 60, 70, 80, 90, 100, 110, 120, 130, 140, 150, 160, 170, 180, 190, or 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The double-stranded linear DNA molecule may comprise a spacer region. The spacer region may comprise at least 10, at least 20, at least 30, at least 40, at least 50, at least 60, at least 70, at least 80, at least 90, at least 100, at least 125, at least 150, at least 175, or at least 200 base pairs.

The closed linear DNA molecule may comprise a spacer region. The spacer region may comprise at least 10, at least 20, at least 30, at least 40, at least 50, at least 60, at least 70, at least 80, at least 90, at least 100, at least 125, at least 150, at least 175, or at least 200 base pairs.

The partially closed linear DNA molecule may comprise a spacer region. The spacer region may comprise at least 10, at least 20, at least 30, at least 40, at least 50, at least 60, at least 70, at least 80, at least 90, at least 100, at least 125, at least 150, at least 175, or at least 200 base pairs.

The double-stranded linear DNA molecule may comprise an inverted terminal repeat sequence.

The closed linear DNA molecule may comprise an inverted terminal repeat sequence.

The partially closed linear DNA molecule may comprise an inverted terminal repeat sequence.

The double-stranded linear DNA molecule may comprise at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the double-stranded linear DNA molecule comprises at least 500 base pairs.

The closed linear DNA molecule may comprise at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the closed linear DNA molecule comprises at least 500 base pairs.

The partially closed linear DNA molecule may comprise at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, at least 15,000, at least 20,000, at least 25,000, at least 30,000, at least 35,000, at least 40,000, at least 45,000 or at least 50,000 base pairs. Preferably, the partially closed linear DNA molecule comprises at least 500 base pairs.

The closed linear DNA molecule may comprise one or more protected nucleotides (i.e. nucleotides resistant to nuclease (e.g. exonuclease) digestion). The closed linear DNA molecule may comprise one or more of protected nucleotides (i.e. nucleotides resistant to nuclease (e.g. exonuclease) in each strand. For example, the closed linear DNA

molecule may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) in each strand.

The linear DNA molecule may be a linear double-stranded DNA molecule. The linear double-stranded DNA molecule may comprise one or more protected nucleotides (i.e. nucleotides resistant to nuclease (e.g. exonuclease) digestion) (e.g. phosphorothioated nucleotides). The protected nucleotides may be located at the 3' and/or 5' end and/or 3' and/or 5' end region of the linear double-stranded DNA molecule. Preferably, the protected nucleotides are located at the 3' and 5' end and at the 3' and 5' region of the linear double-stranded DNA molecule. The 3' region comprises at least 20 3'-end nucleotides of the linear DNA molecule. The 3' region comprises less than 30 3'-end nucleotides of the linear DNA molecule. The 5' region comprises no more than 30 5'-end nucleotides of the linear DNA molecule. That is to say that the nucleotides which are the last and/or the first nucleotides in the linear DNA molecule may be protected nucleotides. In other words, the linear DNA molecule may comprise a "cap" of protected nucleotides, which protects the ends of the linear DNA molecule from nuclease (e.g. exonuclease) digestion.

The partially closed linear DNA molecule may comprise one or more protected nucleotides (i.e. nucleotides resistant to nuclease (e.g. exonuclease) digestion). The partially closed linear DNA molecule may comprise one or more of protected nucleotides (i.e. nucleotides resistant to nuclease (e.g. exonuclease) in each strand. For example, the partially closed linear DNA molecule may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, at least or 500 protected nucleotides (e.g. phosphorothioated nucleotides) in each strand.

The RNA molecule may be a messenger RNA (mRNA), a transfer RNA, a ribosomal RNA, a small interfering RNA (siRNA), an antisense RNA (an antisense oligonucleotide), a small nuclear RNA (snRNA), a double-stranded RNA, a microRNA (miRNA), short hairpin RNA (shRNA), guide RNA (gRNA), self-amplifying mRNA (samRNA), or circular RNA.

The RNA molecule may comprise at least 5, at least 10, at least 15, at least 20, at least 25, at least 30, at least 35, at least 40, at least 45, at least 50, at least 75, at least 100, at least 150, or at least 200 nucleotides, at least 500 nucleotides, at least 1,000 nucleotides, at least 2,000 nucleotides, at least 5,000 nucleotides, at least 10,000 nucleotides, at least 15,000 nucleotides or at least 16,000 nucleotides. The RNA molecule may comprise 5-20,000, 6-19,000, 7-18,000, 8-17,000, 9-16,000, 10-15,000, 10-13,000, 15-10,000, 20-5,000, 20-1,000, 20-500, or 25-300 nucleotides. The RNA molecule may comprise one or more protected nucleotides (i.e. nucleotides resistant to nuclease (e.g. exonuclease) digestion) (e.g. phosphorothioated nucleotides).

The RNA molecule may be an mRNA molecule comprising at least 5, at least 10, at least 15, at least 20, at least 25, at least 30, at least 35, at least 40, at least 45, at least 50, at least 75, at least 100, at least 150, or at least 200 nucleotides. The mRNA molecule may comprise 5-20,000, 6-19,000, 7-18,000, 8-17,000, 9-16,000, 10-13,000, 10-15,000, 15-10,000, 20-5,000, 20-1,000, 20-500, or 25-300 nucleotides. The mRNA molecule may comprise one or more protected nucleotides (i.e. nucleotides resistant to nuclease (e.g. exonuclease) digestion) (e.g. phosphorothioated nucleotides).

The RNA molecule may be a self-amplifying mRNA (samRNA) molecule comprising at least 3,000, at least 4,000, at least 5,000, at least 6,000, at least 7,000, at least 8,000, at least 9,000, at least 10,000, at least 11,000, at least 12,000, at least 13,000, at least 14,000, at least 15,000, at least 16,000, at least 17,000, at least 18,000, at least 19,000, or at least 20,000 nucleotides. The samRNA molecule may comprise 3,000-22,000, 5,000-21,000, 7,000-20,000, or 8,000-17,000 nucleotides. The samRNA molecule may comprise one or more protected nucleotides (i.e. nucleotides resistant to nuclease (e.g. exonuclease) digestion) (e.g. phosphorothioated nucleotides).

The siRNA may comprise a double-stranded portion of at least 17 base pairs, at least 18 base pairs or preferably at least 19 base pairs. The siRNA may comprise a double stranded portion of 17-30 base pairs, 18-27 base pairs, 19-24 base pairs or preferably 19-21 base pairs. The siRNA may comprise one or more protected nucleotides (i.e. nucleotides resistant to nuclease (e.g. exonuclease) digestion) (e.g. phosphorothioated nucleotides).

The miRNA may comprise at least 5, at least 10, at least 15, at least 20, at least 25, at least 30, at least 35, at least 40, at least 45, at least 50, at least 75, at least 100 nucleotides. The miRNA molecule may comprise 10-200, 12-150, 15-125, 17-100, 18-75, 20-75, 20-50, or 20-30 nucleotides.

The miRNA may comprise a double-stranded portion of at least 15 base pairs, at least 17 base pairs or preferably at least 20 base pairs. The miRNA may comprise a double stranded portion of 15-30 base pairs, 17-27 base pairs, 20-25 base pairs or preferably 21-23 base pairs. The miRNA may comprise one or more protected nucleotides (i.e. nucleotides resistant to nuclease (e.g. exonuclease) digestion) (e.g. phosphorothioated nucleotides).

The antisense RNA may comprise at least 18, at least 19, at least 20, at least 21, at least 22, or at least 23 nucleotides. The antisense RNA may comprise 18-24 nucleotides or preferably 19-23 nucleotides. The antisense RNA may comprise one or more protected nucleotides (i.e. nucleotides resistant to nuclease (e.g. exonuclease) digestion) (e.g. phosphorothioated nucleotides).

The protected nucleotides (i.e. nucleotides resistant to nuclease (e.g. exonuclease) digestion) may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The protected nucleotides (i.e. nucleotides resistant to nuclease (e.g. exonuclease) digestion) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The linear double-stranded DNA molecule may comprise one or more protected nucleotides (e.g. phosphorothioated nucleotides) at internal positions in each strand (i.e. the sense and antisense strands). Similarly, the RNA molecule may comprise one or more protected nucleotides (e.g. phosphorothioated nucleotides) at internal positions in the RNA strand or strands (if double-stranded RNA).

The internal positions may be any positions in the linear double-stranded DNA molecule or the RNA molecule other than the last nucleotide on the 3'-end and the 5'-end of the sense and/or antisense strands. The internal positions may be located at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA molecule or the strand of the RNA molecule. The internal positions may be located at least 7 nucleotides away from the 3'-end and/or the 5'-end of each strand of the linear double-stranded DNA molecule or the strand of the RNA molecule. Preferably, the internal positions are located at least 10 nucleotides away from the 3'-end and/or the 5'-end of each strand of the linear double-stranded DNA molecule or the strand of the RNA molecule. In the linear double-stranded DNA molecule, the internal positions may be located at least 7 nucleotides away from the 3'-end and the 5'-end of the sense strand. Preferably, in the linear double-stranded DNA molecule, the internal positions are located at least 10 nucleotides away from the 3'-end and the 5'-end of the sense strand. In the linear double-stranded DNA molecule, the internal positions may be located at least 7 nucleotides away from the 3'-end and the 5'-end of the antisense strand. Preferably, in the linear double-stranded DNA molecule, the internal positions may be located at least 10 nucleotides away from the 3'-end and the 5'-end of the antisense strand. In the linear double-stranded DNA molecule, the internal positions may be located at least 7 nucleotides away from the 3'-end of each strand. Preferably, in the linear double-stranded DNA molecule, the internal positions may be located at least 10 nucleotides away from the 3'-end of each strand. In the linear double-stranded DNA molecule, the internal positions may be located at least 7 nucleotides away from the 5'-end of each strand. Preferably, in the linear double-stranded DNA molecule, the internal positions may be located at least 10 nucleotides away from the 5'-end of each strand.

The linear double-stranded DNA molecule may comprise a cassette. The location of the protected (e.g. phosphorothioated) nucleotides in the linear double-stranded DNA molecule may be such that the cassette is protected from nuclease (e.g. exonuclease III) digestion. Thus, the one or more phosphorothioated nucleotides at the internal positions in each strand may be selected from:

(a) a 5'-end nucleotide of the cassette;
(b) a 3'-end nucleotide of the cassette; and
(c) one or more nucleotides outside of the cassette

The term "*5'-end nucleotide of the cassette"* as used herein is intended to encompass the 5'-end nucleotide of each strand of the cassette. Thus, in the linear double-stranded DNA molecule, the cassette typically comprises a 5'-end nucleotide of the sense strand and a 5'-end nucleotide of the antisense strand.

The term *"3'-end nucleotide of the cassette"* as used herein is intended to encompass the 3'-end nucleotide of each strand of the cassette. Thus, in the linear double-stranded DNA molecule, the cassette typically comprises a 3'-end nucleotide of the sense strand and a 3'-end nucleotide of the antisense strand.

The term *"outside of the cassette"* as used herein is intended to encompass any nucleotides which are not part of the cassette. This includes any nucleotides that are comprised in the linear double-stranded DNA molecule and which also do not form part of the cassette. The term *"N nucleotides outside of the cassette"* or *"N nucleotides away from the cassette"* is intended to describe nucleotides that are located N nucleotides from the end of the cassette towards the end of the linear double-stranded DNA molecule. For example, the term *"2 nucleotides outside of the cassette"* in the context of internal positions of nucleotides is meant to describe a nucleotide that is outside of the cassette and that is two nucleotides away from the last nucleotide of the cassette. For example, in the sequence: 5'-AAAAAACATAAAA (SEQ ID NO: 37), wherein the cassette starts from nucleotide "T" (in the 5' to 3' direction), the term *"2 nucleotides outside of the cassette"* refers to the "C" nucleotide. Thus, the term *"at least 2 nucleotides outside of the cassette"* or *"at least 2 nucleotides away from the cassette"* is meant to describe nucleotides that are outside of the cassette and that are at least two nucleotides away from the last nucleotide of the cassette. In the example above, nucleotides *"at least 2 nucleotides away from the cassette"* would be any nucleotides selected from 5'-AAAAAAC. Similarly, the term *"at least*

*2 nucleotides away from the 5'-end of the cassette"* is meant to describe nucleotides that are outside of the cassette and that are at least two nucleotides away from the last nucleotide at the 5'-end of the cassette. In the example above, the last nucleotide at the 5'-end of the cassette is "T", and nucleotides *"at least 2 nucleotides away from the 5'-end of the cassette"* would be any nucleotides selected from 5'-AAAAAAC.

The internal positions may not be located between the second and penultimate nucleotide of the cassette. The internal positions may be any position in the linear double-stranded DNA molecule other than the last nucleotide on the 3'-end and the 5'-end of the sense and antisense strands and other than nucleotides located between the second and penultimate nucleotide of the cassette. The internal positions may be located outside of the cassette and at least 2, 3, 4, 5, 6, 7, 8, 9, 10, 12, 14, 16, 18, 20, 30, 40, 50, 75, or 100 nucleotides away from the 3'-end and/or the 5'-end of each strand of the linear double-stranded DNA molecule and/or at least 2, 3, 4, 5, 6, 7, 8, 9, 10, 12, 14, 16, 18, 20, 30, 40, 50, 75, or 100 nucleotides away (i.e. outside of the cassette) from the 3'-end and/or the 5'-end of each strand of the cassette. Preferably, the internal positions is located outside of the cassette and at least 6, at least 8, or at least 10 nucleotides away from the 3'-end and/or the 5'-end of each strand of the linear double-stranded DNA molecule and/or at least 6, at least 8, or at least 10 nucleotides away from the 3'-end and/or the 5'-end of each strand of the cassette (i.e. at least 6, at least 8, or at least 10 nucleotides outside of the cassette). Preferably, the cassette does not comprise a phosphorothioated nucleotide at either one of the positions 1-2, 1-3, 1-4, 1-5, 1-6, 1-7, 1-8, 1-9, 1-10, 1-12, 1-14, 1-16, 1-18, or 1-20 of the sense and/or the antisense strand away from the 3'-end and/or the 5'-end of the cassette (i.e. outside of the cassette). Preferably, the internal positions in each strand are located at least 6 nucleotides away from the end of the linear double-stranded DNA molecule and are not located between the second and penultimate nucleotide of the cassette. Preferably, the internal positions in each strand are located at least 10 nucleotides away from the end of the linear double-stranded DNA molecule and are not located between the second and penultimate nucleotide of the cassette. Preferably, the internal positions in each strand are located at least 6 nucleotides away from the end of the linear double-stranded DNA molecule and at least 6 nucleotides away from the end of the cassette (i.e. at least 6 nucleotides outside of the cassette). Preferably, the internal positions in each strand are located at least 10 nucleotides away from the end of the linear double-stranded DNA molecule and at least 10 nucleotides away from the end of the cassette (i.e. at least 10 nucleotides outside of the cassette). The linear double-stranded DNA molecule may comprise a first phosphorothioated nucleotide at an internal position which is a position other than the last nucleotide on the 3'-end and the 5'-end of the sense and antisense strands as long as this position is located at least 2, 3, 4, 5, 6, 7, 8, 9, 10, 12, 14, 16, 18, 20, 30, 40, or 50 nucleotides outside of the cassette. For example, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are not located between the second and penultimate nucleotide of the cassette. Preferably, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located outside of the cassette. The linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located at least 2, 3, 4, 5, 6, 7, 8, 9, 10, 12, 14, 16, 18, 20, 30, 40, or 50 nucleotides outside of the cassette. Preferably, the linear double-stranded DNA molecule may comprise a first phosphorothioated nucleotide which is at least the 6th, 8th or 10th nucleotide counting from the 3'-end and/or the 5'-end of the sense and/or the antisense strand as long as these positions are located at least 6, 8, or 10 nucleotides outside of the cassette.

The linear double-stranded DNA molecule may comprise at least 2, 3, 4, 5, 6, 7, 8, 9 or 10 protected (e.g. phosphorothioated) nucleotides at internal positions in each strand. Preferably, the linear double-stranded DNA molecule comprises at least 2 protected (e.g. phosphorothioated) nucleotides at internal positions in each strand.

The location of phosphorothioated nucleotides in the sense strand of the linear double-stranded DNA molecule may be such that the one or more phosphorothioated nucleotides are located upstream of the cassette (i.e. towards the 5'-end of the sense strand of the DNA molecule).

In the sense strand of the linear double-stranded DNA molecule:

(a) one of the at least two phosphorothioated nucleotides may be the 5'-end nucleotide of the cassette; and/or
(b) one of the at least two phosphorothioated nucleotides may be in a first region of the sense strand, wherein the first region of the sense strand is 5' of the cassette.

The location of phosphorothioated nucleotides in the sense strand of the linear double-stranded DNA molecule may be such that the one or more phosphorothioated nucleotides are located downstream of the cassette (i.e. towards the 3'-end of the sense strand of the DNA molecule).

In the sense strand of the linear double-stranded DNA molecule:

(a) one of the at least two phosphorothioated nucleotides may be the 3'-end nucleotide of the cassette; and/or

(b) one of the at least two phosphorothioated nucleotides may be in a second region of the sense strand, wherein the second region of the sense strand is 3' of the cassette.

The location of phosphorothioated nucleotides in the antisense strand of the linear double-stranded DNA molecule may be such that the one or more phosphorothioated nucleotides are located upstream of the cassette (i.e. towards the 5'-end of the antisense strand of the DNA molecule).

In the antisense strand of the linear double-stranded DNA molecule:

(a) one of the at least two phosphorothioated nucleotides may be the 5'-end nucleotide of the cassette; and/or
(b) one of the at least two phosphorothioated nucleotides may be in a first region of the antisense strand, wherein the first region of the antisense strand is 5' of the cassette.

The location of phosphorothioated nucleotides in the antisense strand of the linear double-stranded DNA molecule may be such that the one or more phosphorothioated nucleotides are located downstream of the cassette (i.e. towards the 3'-end of the antisense strand of the DNA molecule).

In the antisense strand of the linear double-stranded DNA molecule:

(a) one of the at least two phosphorothioated nucleotides may be the 3'-end nucleotide of the cassette; and/or
(b) one of the at least two phosphorothioated nucleotides may be in a second region of the antisense strand, wherein the second region of the antisense strand is 3' of the cassette.

The location of phosphorothioated nucleotides in both the sense and the antisense strand of the linear double-stranded DNA molecule may be such that at least one phosphorothioated nucleotide is located downstream of the cassette and at least one phosphorothioated nucleotide is located upstream of the cassette in each strand.

In the linear double-stranded DNA molecule:

(a) in the sense strand one of the at least two phosphorothioated nucleotides may be the 5'-end nucleotide of the cassette, and/or one of the at least two phosphorothioated nucleotides may be in a first region of the sense strand, wherein the first region of the sense strand is 5' of the cassette;
(b) in the sense strand one of the at least two phosphorothioated nucleotides may be the 3'-end nucleotide of the cassette, and/or one of the at least two phosphorothioated nucleotides may be in a second region of the sense strand, wherein the second region of the sense strand is 3' of the cassette;
(c) in the antisense strand one of the at least two phosphorothioated nucleotides may be the 5'-end nucleotide of the cassette, and/or one of the at least two phosphorothioated nucleotides may be in a first region of the antisense strand, wherein the first region of the antisense strand is 5' of the cassette; and
(d) in the antisense strand one of the at least two phosphorothioated nucleotides may be the 3'-end nucleotide of the cassette, and/or one of the at least two phosphorothioated nucleotides may be in a second region of the antisense strand, wherein the second region of the antisense strand is 3' of the cassette.

The term *"first region of the sense strand*" as used herein is intended to encompass the part of the sense strand of the linear double-stranded DNA molecule that is between the 5'-end of the linear double-stranded DNA molecule and the first 5' nucleotide of the cassette in the sense strand. For example, in the sequence of the sense strand: 5'-AAAAAACATAAAA-3' (SEQ ID NO: 40), wherein the cassette starts from nucleotide "T" in the 5'-3' direction, the term *"first region of the sense strand*" refers to the 5'-AAAAAACA-3' region.

The term *"first region of the antisense strand*" is intended to encompass the part of the antisense strand of the linear double-stranded DNA molecule that is between the 5'-end of the linear double-stranded DNA molecule and the first 5' nucleotide of the cassette in the antisense strand. For example, in the sequence of the antisense strand: 5'-AAAAAACATAAAA-3' (SEQ ID NO: 40), wherein the cassette starts from nucleotide "T" in the 5'-3' direction, the term *"first region of the antisense strand*" refers to the 5'-AAAAAACA-3' region.

The term *"second region of the sense strand*" is intended to encompass the part of the sense strand of the linear double-stranded DNA molecule that is between the 3'-end of the linear double-stranded DNA molecule and the first 3' nucleotide of the cassette in the sense strand. For example, in the sequence of the sense strand: 5'-AAAAAACATAAAA-3' (SEQ ID NO: 40), wherein the cassette starts from nucleotide "T" in the 3'-5' direction, the term *"second region of the sense strand*" refers to the 5'-AAAA-3' region.

The term *"second region of the antisense strand*" is intended to encompass the part of the antisense strand of the linear double-stranded DNA molecule that is between the 3'-end of the linear double-stranded DNA molecule and the first 3' nucleotide of the cassette in the antisense strand. For example, in the sequence of the antisense strand: 5'-AAAAAACATAAAA-3' (SEQ ID NO: 40), wherein the cassette starts from nucleotide "T" in the 3'-5' direction, the term

*"second region of the antisense strand*" refers to the 5'-AAAA-3' region.

The linear double-stranded DNA molecule may comprise a plurality of the phosphorothioated nucleotides upstream (i.e. towards the 5'-end of the sense strand of the DNA molecule) of the cassette. For example, the linear double-stranded DNA molecule may comprise at least 2, 3, 4, 5, 6, 7, 8, 9 or 10 phosphorothioated nucleotides upstream of the cassette. Preferably, at least 2 phosphorothioated nucleotides upstream of the cassette. Thus, the location of phosphorothioated nucleotides in the sense strand of the linear double-stranded DNA molecule may be such that at least two phosphorothioated nucleotides are located upstream of the cassette.

In the sense strand of the linear double-stranded DNA molecule:

(a) one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or

(b) the at least two phosphorothioated nucleotides may be in a first region of the sense strand, wherein the first region of the sense strand is 5' of the cassette.

The linear double-stranded DNA molecule may comprise a plurality of the phosphorothioated nucleotides downstream (i.e. towards the 3-end of the DNA molecule) of the cassette. For example, the linear double-stranded DNA molecule may comprise at least 2, 3, 4, 5, 6, 7, 8, 9 or 10 phosphorothioated nucleotides downstream of the cassette, preferably at least 2 phosphorothioated nucleotides downstream of the cassette. Thus, the location of phosphorothioated nucleotides in the sense strand of the linear double-stranded DNA molecule may be such that at least two phosphorothioated nucleotides are located downstream of the cassette.

In the sense strand of the linear double-stranded DNA molecule:

(a) one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or

(b) the at least two phosphorothioated nucleotides may be in a first region of the sense strand, wherein the first region of the sense strand is 3' of the cassette.

The location of phosphorothioated nucleotides in the antisense strand of the linear double-stranded DNA molecule may be such that at least two phosphorothioated nucleotides are located upstream of the cassette (i.e. towards the 5'-end of the antisense strand of the DNA molecule).

In the antisense strand of the linear double-stranded DNA molecule:

(a) one of the at least two phosphorothioated nucleotides is the 5'-end nucleotide of the cassette and one of the at least two phosphorothioated nucleotides is in a first region of the antisense strand, wherein the first region of the antisense strand is 5' of the cassette; or

(b) the at least two phosphorothioated nucleotides are in a first region of the antisense strand, wherein the first region of the antisense strand is 5' of the cassette.

The location of phosphorothioated nucleotides in the antisense strand of the linear double-stranded DNA molecule may be such that at least two phosphorothioated nucleotides are located downstream of the cassette (i.e. towards the 3'-end of the antisense strand of the DNA molecule).

In the antisense strand of the linear double-stranded DNA molecule:

(a) one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or

(b) the least two phosphorothioated nucleotides may be in a second region of the antisense strand, wherein the second region of the antisense strand is 3' of the cassette.

Each strand of the linear double-stranded DNA molecule may comprise a plurality of phosphorothioated nucleotides. For example, the linear double-stranded DNA molecule may comprise at least 2, 3, 4, 5, 6, 7, 8, 9 or 10 phosphorothioated nucleotides upstream and downstream of the cassette. The location of phosphorothioated nucleotides in both the sense and the antisense strand of the linear double-stranded DNA molecule may be such that the at least two phosphorothioated nucleotides are located downstream of the cassette and at least two phosphorothioated nucleotides are located upstream of the cassette in each strand.

In the linear double-stranded DNA molecule:

(a) in the sense strand:

i. one of the at least two phosphorothioated nucleotides may be the 5'-end nucleotide of the cassette and one of the at least two phosphorothioated nucleotides may be in a first region of the sense strand, wherein the first region of the sense strand is 5' of the cassette; or
ii. the at least two phosphorothioated nucleotides may be in a first region of the sense strand, wherein the first region of the sense strand is 5' of the cassette;

(b) in the sense strand:

i. one of the at least two phosphorothioated nucleotides may be the 3'-end nucleotide of the cassette and one of the at least two phosphorothioated nucleotides may be in a second region of the sense strand, wherein the second region of the sense strand is 3' of the cassette; or
ii. the at least two phosphorothioated nucleotides may be in a second region of the sense strand, wherein the second region of the sense strand is 3' of the cassette;

(c) in the antisense strand:

i. one of the at least two phosphorothioated nucleotides may be the 5'-end nucleotide of the cassette and one of the at least two phosphorothioated nucleotides may be in a first region of the antisense strand, wherein the first region of the antisense strand is 5' of the cassette; or
ii. the at least two phosphorothioated nucleotides may be in a first region of the antisense strand, wherein the first region of the antisense strand is 5' of the cassette; and

(d) in the antisense strand:

i. one of the at least two phosphorothioated nucleotides may be the 3'-end nucleotide of the cassette and one of the at least two phosphorothioated nucleotides may be in a second region of the antisense strand, wherein the second region of the antisense strand is 3' of the cassette; or
ii. the at least two phosphorothioated nucleotides may be in a second region of the antisense strand, wherein the second region of the antisense strand is 3' of the cassette.

The linear double-stranded DNA molecule, the partially closed linear DNA molecule or the closed linear DNA molecule may be resistant to nuclease digestion or may have improved or enhanced resistance to nuclease digestion. The linear double-stranded DNA molecule, the partially closed linear DNA molecule or the closed linear DNA molecule may be resistant to exonuclease digestion or have improved or enhanced resistance to exonuclease digestion. The linear double-stranded DNA molecule, the partially closed linear DNA molecule or the closed linear DNA molecule may be resistant, or have improved or enhanced resistance, to digestion by exonucleases that cleave the 3'-end nucleotides (e.g. exonuclease III) and/or exonucleases that cleave the 5'-end nucleotides (e.g. exonuclease VIII). The terms "improved" or "enhanced" in the context of resistance to enzyme digestion refer to higher resistance to enzyme digestion when compared to a DNA molecule not produced by the methods described herein. For example, for the linear double-stranded DNA molecule, when compared to a molecule that does not comprise protected nucleotides, such as phosphorothioated nucleotides.

## 4. Pharmaceutical compositions

The invention provides a pharmaceutical composition comprising a nanoparticle (e.g. a non-viral transfection complex) described herein. The invention provides a pharmaceutical composition comprising a nanoparticle (e.g. a non-viral transfection complex) described herein and a pharmaceutically suitable carrier or excipient. The invention provides a pharmaceutical composition comprising a targeting peptide described herein and a pharmaceutically suitable carrier or excipient.
The invention provides a pharmaceutical composition comprising a nanoparticle (e.g. a non-viral transfection complex), which comprises:

(a) a cargo;
(b) a lipid component; and
(c) a targeting peptide comprising a chondrocyte targeting sequence.

The invention provides a pharmaceutical composition comprising:

i. a nanoparticle (e.g. a non-viral transfection complex), which comprises:

(a) a cargo;
(b) a lipid component; and
(c) a targeting peptide comprising a chondrocyte targeting sequence; and

ii. a pharmaceutically suitable carrier.

The pharmaceutical composition may comprise:

i. a nanoparticle (e.g. a non-viral transfection complex), which comprises:

(a) a cargo
(b) a lipid component; and
(c) a targeting peptide comprising a chondrocyte targeting sequence and a cargo-binding component; and

ii. a pharmaceutically suitable carrier.

The pharmaceutical composition may comprise:

i. a nanoparticle (e.g. the non-viral transfection complex), which comprises:

(a) a cargo, wherein the cargo is a nucleic acid;
(b) a lipid component; and
(c) a targeting peptide comprising a chondrocyte targeting sequence and a nucleic acid-binding polycationic component (e.g. DNA-binding polycationic component); and

ii. a pharmaceutically suitable carrier.

The pharmaceutical composition may comprise:

i. a nanoparticle (e.g. the non-viral transfection complex), which comprises:

(a) a cargo, wherein the cargo is a nucleic acid, wherein the nucleic acid is a closed linear DNA molecule;
(b) a lipid component; and
(c) a targeting peptide comprising a chondrocyte targeting sequence and a closed linear DNA-binding component (e.g. a closed linear DNA-binding polycationic component); and

ii. a pharmaceutically suitable carrier.

The pharmaceutical composition may comprise:

i. a nanoparticle (e.g. the non-viral transfection complex) may comprise:

(a) a cargo, wherein the cargo is a nucleic acid, wherein the nucleic acid is a linear DNA molecule comprising one or more nuclease-resistant nucleotides (e.g. phosphorothioated nucleotides);
(b) a lipid component; and
(c) a targeting peptide comprising a chondrocyte targeting sequence and a linear DNA-binding component (e.g. a linear DNA-binding polycationic component); and

ii. a pharmaceutically suitable carrier.

The pharmaceutical composition may comprise:

i. a nanoparticle (e.g. the non-viral transfection complex), which comprises:

(a) a cargo, wherein the cargo is a nucleic acid, wherein the nucleic acid is a partially closed linear DNA molecule comprising a double-stranded DNA portion that is closed at a first end and open at a second end, wherein the partially closed linear DNA molecule comprises one or more nuclease-resistant nucleotides in an open end region adjacent to the second end;
(b) a lipid component; and
(c) a targeting peptide comprising a chondrocyte targeting sequence and a closed linear DNA-binding component (e.g. a closed linear DNA-binding polycationic component); and

ii. a pharmaceutically suitable carrier.

The pharmaceutical composition may comprise:

i. a targeting peptide comprising:

(a) a chondrocyte targeting sequence; and
(b) a cargo-binding component; and

ii. a pharmaceutically suitable carrier.

The pharmaceutical composition may comprise:

i. a targeting peptide comprising:

(a) a chondrocyte targeting sequence
(b) a spacer; and
(c) a cargo-binding component; and

ii. a pharmaceutically suitable carrier.

The pharmaceutical composition may comprise:

i. a targeting peptide comprising:

(a) a chondrocyte targeting sequence;
(b) a linker; and
(c) a cargo-binding component; and

ii. a pharmaceutically suitable carrier.

The pharmaceutical composition may comprise:

i. a targeting peptide comprising:

(a) a chondrocyte targeting sequence;
(b) a linker;
(c) a spacer; and
(d) a cargo-binding component; and

ii. a pharmaceutically suitable carrier.

In all embodiments described herein, the presence of a pharmaceutically suitable carrier is optional. The nanoparticle of the invention may function as a pharmaceutically suitable carrier.
The chondrocyte targeting sequence may be any of the chondrocyte targeting sequences described herein. Preferably, the chondrocyte targeting sequence is SEQ ID NO: 1, SEQ ID NO: 2, SEQ ID NO: 3, SEQ ID NO: 4, or SEQ ID NO: 5, or a variant thereof comprising one or more conservative amino acid substitutions.
The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation

may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally, transdermally, intramuscularly, intra-articularly, by IV or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

The pharmaceutical composition may be administered by injection (e.g. intravenous or intramuscular injection). The pharmaceutical composition may be formulated as an emulsion consisting of a sterile, pyrogen-free preparation or a lipid complex preparation.

The pharmaceutical composition may be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

The pharmaceutical composition may be administered by intra-articular injection (i.e. into the joint). Such methods are routinely used in the art, for example, for the administration of hydrocortisone into the joint.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

In another mode of administration, the pharmaceutical composition can be administered in situ, via a catheter or pump. A catheter or pump can, for example, direct the composition into the target location.

The parenteral carrier system (e.g. intravenous carrier system or intramuscular carrier system) may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing

agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

## 5. Uses and applications

### General therapeutic and diagnostic uses

The invention provides the nanoparticle described herein for use in therapy.

The invention provides the targeting peptide described herein for use in therapy.

The invention also provides the pharmaceutical composition described herein for use in therapy.

The invention provides a cargo for use in therapy, wherein the cargo is administered to a subject as part of a nanoparticle described herein. Preferably, the cargo is nucleic acid described herein.

The nanoparticle may comprise a cargo (e.g. a nucleic acid cargo) which encodes a sequence of a therapeutic protein, a part of a vaccine, or an element of a genetic engineering mechanism, which is used to treat a disease or

infection in a subject.

The nanoparticle described herein, the targeting peptide described herein, the pharmaceutical composition described herein, or the cargo described herein may be used (or for use) in the treatment of a joint or cartilage disease or disorder. The joint or cartilage disease or disorder may be MPS-1, rheumatoid arthritis, osteoarthritis, or Juvenile Idiopathic Arthritis (JIA).

The nanoparticle described herein, the targeting peptide described herein, the pharmaceutical composition described herein, or the cargo described herein, may be used (or for use) in the treatment of a disease or disorder manifested by an overexpression, decreased expression or abnormal expression of one or more of the following genes IL-1Ra, Interferon-β, IL-10, IDUA, TNFR:Fc or TGF-β.The nanoparticle or pharmaceutical composition may comprise a nucleic acid cargo comprising the corresponding gene(s) or portion(s) thereof. The cargo may be a nucleic acid cargo comprising the corresponding gene(s) or portion(s) thereof.

The nanoparticle described herein, the targeting peptide described herein, the pharmaceutical composition described herein, or the cargo described herein may be used (or for use) in eliminating, alleviating or ameliorating symptoms of a joint or cartilage disease or disorder.

The nanoparticle described herein, the targeting peptide described herein, the pharmaceutical composition described herein, or the cargo described herein may be used (or for use) as a medicament. The invention provides the nanoparticle described herein, the targeting peptide described herein, the pharmaceutical composition described herein, or the cargo described herein for use in the manufacture of a medicament for the prophylaxis of a condition caused in a subject by a defect and/or deficiency in a gene.

The invention provides a method for treatment of a disease or disorder caused in a subject by defect and/or deficiency in a gene, the method comprising administering the nanoparticle described herein, the targeting peptide described herein, the pharmaceutical composition described herein, or the cargo described herein to the subject. Preferably, the amount of the nanoparticle, the targeting peptide, the pharmaceutical composition, or the cargo administered to the subject is a therapeutically active amount. Thus, the invention also provides the nanoparticle described herein, the targeting peptide described herein, the pharmaceutical composition, or the cargo described herein may be used (or for use) in gene therapy.

A subject treated with the nanoparticle described herein, the targeting peptide described herein, the pharmaceutical composition described herein, or the cargo described herein may receive the nanoparticle described herein, the targeting peptide described herein, the pharmaceutical composition described herein, or the cargo described herein with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the nanoparticle described herein, the targeting peptide described herein, the pharmaceutical composition described herein, or the cargo described herein into the subject's body as described in more detail above (see "Pharmaceutical compositions"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the nanoparticle described herein, the targeting peptide described herein, the pharmaceutical composition described herein, or the cargo described herein that, when administered to a subject for treating a disease, is sufficient to effect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides use of the nanoparticle described herein, the targeting peptide described herein, the pharmaceutical composition described herein, or the cargo described herein in a method of diagnosing a disease and/or a disorder.

The invention provides the use of the nanoparticle described herein, the targeting peptide described herein, the pharmaceutical composition described herein, or the cargo described herein in the "in vitro" diagnosis of a disease.

The invention also provides the nanoparticle described herein, the targeting peptide described herein, the pharmaceutical composition described herein, or the cargo described herein for use in a method of diagnosis "in vivo" of disease.

The method may be used to diagnose a joint or cartilage disease or disorder. For example, MPS-1, rheumatoid arthritis, osteoarthritis, or Juvenile Idiopathic Arthritis (JIA).

The diagnostic and treatment methods described herein may be in vitro methods or in vivo methods.

The method of diagnosis may rely on the detection and/or quantification of the nanoparticle described herein,

the targeting peptide described herein, the pharmaceutical composition described herein, or the cargo described herein.

To facilitate detection and/or quantification of the nanoparticle described herein, the nanoparticle may be attached or bound to a functional portion. For example, the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the nanoparticle. For example, the signal may be measured by flow cytometry and/or fluorescence-activated cell sorting of the nanoparticle attached to a fluorescent probe.

The nanoparticle may be detected by being bound to a capture moiety, for example in a lateral flow assay. In this example, the functional portion is a protein, for example, an antibody specific for the capture moiety. The capture of the antibody attached to the nanoparticle may produce a visual signal (e.g. a band of a different colour).

The invention also provides a method that combines diagnosis of a disease with a treatment of the disease.

Cell therapy

The nanoparticle described herein, the targeting peptide described herein, the pharmaceutical composition described herein, or the cargo described herein may be used (or for use) in cell therapy.

The invention provides the use of the nanoparticle described herein, the targeting peptide described herein, the pharmaceutical composition described herein, or the cargo described herein in the production of cell therapy. The invention provides a method of cell therapy, comprising contacting the nanoparticle described herein, the targeting peptide described herein, the pharmaceutical composition described herein, or the cargo described herein with a cell. Preferably, cell therapy is ex-vivo cell therapy. The cell may be an animal cell, preferably mammal cell, such as human cell.

The cargo is preferably formulated as part of the nanoparticle described herein before or simultaneously to contacting the cell.

The invention also provides a cell obtainable by any methods described herein. For example, the cell may be suitable for use in cell therapy.

Vaccines

The products of the invention are particularly suitable for use in vaccine production. The nanoparticle described herein, the targeting peptide described herein, the pharmaceutical composition described herein, or the cargo described herein may be used to produce a vaccine, preferably an mRNA-based vaccine. For example, vaccines such as the BioNTech and Moderna mRNA vaccines against COVID-19.

The nanoparticle described herein or the pharmaceutical composition described herein may be used as a vaccine.

The nanoparticle described herein, the targeting peptide described herein, the pharmaceutical composition described herein, or the cargo described herein may be used in the therapeutic or prophylactic immunisation.

CRISPR delivery

The nanoparticles and targeting peptides are particularly suitable for use in delivery of CRISPR machinery to cells, for example in cell therapy or in vivo therapy.

The nanoparticle may comprise a cargo, which is a nucleic acid cargo (e.g. the nucleic acid cargo described herein). The nucleic acid cargo may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template may comprise or consist of a homology region for a dystrophin gene or a part of the dystrophin gene. The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cas9, Cpf1, or MAD7). The repair template (or editing template) may introduce at least one mutation (e.g. an insertion, deletion, and/or substitution) in the desired DNA region (i.e. target molecule). For example, the repair template may introduce a correct (i.e. functional) copy of the IL-1Ra, Interferon-β, IL-10, IDUA, or TGF-β gene or a portion thereof. The repair template may comprise a correct (i.e. functional) copy of the IL-1Ra, Interferon-β, IL-10, IDUA, or TGF-β gene or a portion thereof. The repair template (or editing template) may comprise at least 10, at least 20, at least 30, at least 40, at least 50, at least 100, at least 200, at least 300, at least 400, at least 500, at least 600, at least 700, at least 800, at least 900, at least 1000, at least 1500, at least 2000, at least 2500, at least 3000, at least 3500, at least 4000, at least 4500, at least 5000, at least 5500, at least 6000, at least 6500, at least 7000, at least 7500,

at least 8000, at least 8500, at least 9000, at least 9500, at least 10000, at least 11000, at least 12000, at least 13000, at least 14000, or at least 15000 base pairs.

Thus, the invention provides the nanoparticle for use in CRISPR system delivery to a cell. The invention provides a method of delivering the CRISPR system to a cell, comprising contacting the nanoparticle with a cell. Preferably, the cell is a chondrocyte.

Delivery methods may be in vivo or in vitro methods.

The cell may be an animal cell, preferably mammal cell, such as human cell.

The invention also provides a cell obtainable by the methods described herein. The cell is particularly suitable for use in cell therapy and/or in vivo therapy.

Uses of the products described herein may be *in vivo* or *in vitro* uses.

### 6. Methods for producing nanoparticles

The invention provides a method for producing (or forming) a nanoparticle described herein, wherein the method comprises:

(a) contacting a lipid component with a cargo and a targeting peptide comprising a chondrocyte targeting sequence to form a single contiguous aqueous volume; and
(b) forming the nanoparticle.

The invention provides a method for producing (or forming) a nanoparticle described herein, wherein the method comprises:

(a) contacting a lipid component with a cargo and a targeting peptide comprising a chondrocyte targeting sequence; and
(b) forming the nanoparticle.

The lipid component may be a liposome. The method may comprise the steps:

(a) contacting a liposome with a cargo and a targeting peptide comprising a chondrocyte targeting sequence; and
(b) forming the nanoparticle.

The method may further comprise a step of forming a liposome. The step of forming a liposome may be performed before the step of contacting a liposome with a cargo and a targeting peptide comprising a chondrocyte targeting sequence.

The step of forming the nanoparticle may be performed by shaking, pipetting or using a microfluidics device.

The step of forming the nanoparticle may rely on self-assembly. That is to say that the nanoparticles of the present invention do not require conjugation of a targeting sequence to a lipid component before encapsulating the cargo.

Step (a) of the method may further comprise contacting the lipid component (or liposome) with a peptide comprising a cargo-binding component. Alternatively, the targeting peptide used in step (a) of the method may further comprise a cargo-binding component. The cargo-binding component may facilitate self-assembly of the nanoparticle. Thus step (b) of the method may comprise forming the nanoparticle by condensing the cargo as a result of its interaction with the cargo-binding component e.g. the cargo-binding component may be a nucleic-acid binding cationic component and the cargo may be a nucleic acid (e.g. DNA).

In aqueous solutions, lipids can self-assemble into liposomes. This is driven by hydrophilic interactions between polar headgroups, and van der Waals interactions between hydrocarbon chains. The lipids are organised so that the headgroups are exposed to the aqueous interface, while the hydrophobic tails are shielded from the aqueous milieu, forming a bilayer. The inclusion of cationic, amphipathic lipids facilitates spontaneous electrostatic binding with negatively charged phosphate groups on nucleic acids.

The positively charged cargo-binding cationic component of the targeting peptide also facilitates electrostatic interactions with negatively charged phosphate backbone of the nucleic acid, condensing the nucleic acid cargo.

The positive charge on both the cationic lipid and cationic-binding component facilitate the self-assembly of the components into nanoparticles encapsulating a nucleic acid payload via electrostatic (intermolecular) interaction.

The method may further comprise a step of diluting the nanoparticle to a desired cargo concentration. The step of diluting the nanoparticle to a desired cargo concentration after the step of forming the nanoparticle. For example, if the cargo is a nucleic acid (e.g. DNA), the desired cargo concentration may be 0.01 ng/μL - 10,000 ng/μL, 0.1 ng/μL - 1000 ng/μL or 1 ng/μL - 100 ng/μL.

The step of forming the nanoparticle is facilitated by the targeting peptide which has cargo (e.g. nucleic acid)

condensing properties.

Each step of the methods described herein may be performed in vitro.

The invention also provides a method for producing (or forming) a library of nanoparticles (e.g. non-viral transfection complexes) described herein, wherein the method comprises:

(a) contacting a lipid component with a cargo and a targeting peptide comprising a chondrocyte targeting sequence to form a single contiguous aqueous volume; and
(b) forming a library of the nanoparticles (e.g. non-viral transfection complexes).

Preferably, the methods described herein produce (or form) nanoparticles or libraries of nanoparticles which are self-assembled. In a library of self-assembled nanoparticles (e.g. non-viral transfection complexes), the size of the particles may be lower than the size of particles which were produced by methods other than self-assembly methods (e.g. methods in which the lipid component is conjugated to a targeting sequence before encapsulating of a cargo). For example, the size of the self-assembled nanoparticles in a library may be lower by at least 5%, at least 10%, at least 15%, at least 20%, at least 25%, at least 30%, at least 35% or at least 40% than the size of particles which were produced by methods other than self-assembly methods.

In the library of nanoparticles (e.g. non-viral transfection complexes), the nanoparticles may be monodisperse or substantially monodisperse. The nanoparticles (e.g. the non-viral transfection complex) may have a polydispersity index (PDI) of less than 0.4, less than 0.3, less than 0.2, or less than 0.15. The nanoparticles (e.g. the non-viral transfection complex) may have a polydispersity index similar or equal to the polydispersity index of empty liposomes (i.e. liposomes not containing a cargo).

The invention provides a library of nanoparticles described herein. In the library of nanoparticles, the nanoparticle (e.g. the non-viral transfection complex) may have a particle size of less than 500 nm, for example less than 250 nm, less than 100 nm, less than 85, less than 75nm, less than 65 nm, less than 50 nm, or less than 40 nm. In the library of nanoparticles there will be some variation in particle size but the above criteria will be taken as met if at least 70%, at least 80% or at least 90% of the nanoparticles are of less than 500 nm, for example less than 250nm, less than 100 nm, less than 85 nm, less than 75nm, less than 65 nm, less than 50 nm or less than 40 nm. Preferably, in a library of nanoparticles, at least 80% of the nanoparticles are less than 500 nm, for example less than 250nm, less than 100 nm, less than 85 nm, less than 75nm, less than 65 nm, less than 50nm or less than 40 nm.

## 7. Cell transfection

The invention provides a cell transfection composition comprising the nanoparticle described herein.

The invention provides a method for transfecting a cell comprising:

(a) contacting a cell with the nanoparticle described herein; and
(b) transfecting the nanoparticle to the cell.

The cell may be an animal cell, such as mammal cell (e.g. a human cell), a fungal cell, a cell of a micro-organism (e.g. a prokaryotic cell or a eukaryotic cell), or a plant cell. The cell may be a human cell. The cell may be a C2C12 cell, a HEK293 cell, or an H9C2 cell.

The cell transfection composition may or may not comprise an agent selected from a photosensitizing agent and/or a radical initiator e.g. a photoinitiator. Preferably, this agent improves the function of the cargo (e.g. the nucleic acid cargo described herein) at a target site and/or protects the cargo (e.g. the nucleic acid cargo described herein) from metabolism and/or degradation.

The invention further provides a cell obtainable by the methods of the invention. Thus, the cell may comprise the nanoparticle of the invention.

The invention further provides a cell transfected with the nanoparticle described herein.

The step of contacting the cell with the nanoparticle may be performed in vivo. For example, the nanoparticle may be administered to an organism (e.g. a subject) in need thereof. The organism (e.g. the subject) may be an animal, such as mammal (e.g. a human), a fungus, a micro-organism, or a plant.

Each step of the methods described herein may be performed in vitro.

## BRIEF DESCRIPTION OF THE DRAWINGS

Figure 1 shows GFP expression and median fluorescence intensity (MFI) in primary human chondrocytes transfected

with closed linear DNA:lipid:peptide nanoparticles comprising different mass ratios of lipid and peptide components. Transfection efficiency was measured 48h post-transfection . n=3 in all experiments, error bars = S.D.

Figure 2 shows GFP expression and median fluorescence intensity (MFI) in primary human chondrocytes transfected with closed linear DNA:lipid:peptide nanoparticles comprising different mass ratios of lipid and peptide components. Transfection efficiency was measured 48h post-transfection . n=3 in all experiments, error bars = S.D.

Figure 3 shows luciferase expression in primary human chondrocytes transfected with mRNA:lipid:peptide nanoparticles at different N/P ratios driven by the mass of the peptide. Transfection efficiency was measured 4h post-transfection. n=3 in all experiments

Figure 4 shows the encapsulation efficacy of nanoparticles formulated with mRNA:lipid:peptide at N/P ratio 3.98 driven by the mass of peptide. n=3 in all experiments

Figure 5 shows biophysical characterization of nanoparticles formulated with DOTMA-DOPE 2:1 + 10% cholesterol and various peptides encapsulating closed linear DNA molecule. n=3 in all experiments

Figure 6 shows biophysical characterization of nanoparticles formulated with DOTMA-DOPE 2:1 + 10% cholesterol and various peptides encapsulating mRNA. n=3 in all experiments

Figure 7 provides details of exemplary targeting peptides.

Figures 8 shows chemical structure of DOTMA, DOPE, DMG-PEG, ALC-0315 and cholesterol.

Figure 9 shows IL-6 expression and transfection efficiency of closed linear DNA and plasmid DNA (pDNA) nanoparticles comprising C18/DOPE at a molar ratio of 2:1 with 10% cholesterol and a targeting peptide comprising a sequence of SEQ ID NO: 23. IL-6 expression was measured 24 h post-transfection. GFP expression was measured 48h post transfection. n=3 in all experiments.

Figure 10 shows IL-6 expression and transfection efficiency of closed linear DNA and plasmid DNA (pDNA) nanoparticles comprising ALC/DOPE at a molar ration of 2:1 with 10% cholesterol and a targeting peptide comprising a sequence of SEQ ID NO: 23. IL-6 expression was measured 24 h post-transfection. GFP expression was measured 48h post-transfection. n=3 in all experiments.

## EXAMPLES

### Example 1 - Liposome formulation

Liposomes were made using NanoAssemblr Ignite, a microfluidcs device (Precison Nanosystems, Vancouver Canada). The cationic lipid and phospholipid/cholesterol were mixed together at various molar ratios in ethanol and injected into the cartridge at a flow rate of 12mL/ min. They were then sonicated in a water bath for 20 minutes. The resulting liposomes were dialysed overnight in 10k Slide-A-Lyzer (Thermo Fisher) cassettes to remove any residual ethanol. Prior to use in experiments, liposomes were stored at 4°C.

### Example 2 - Transfection of primary human chondrocytes - GFP

Peptides identified via phage display with affinity toward primary human chondrocytes, and peptides identified in the literature with desirable chondrocyte binding characteristics were synthesised via solid-phase peptide synthetic chemistry in various conformations. A sixteen-lysine tail group was attached using standard synthesis methods (amsbio, UK). Sequences are shown in Table 2.

DOTMA, DOPE and cholesterol lipids were purchased from Avanti Polar Lipids, Alabaster, Alabama, USA.

Primary human chondrocytes were cultured in were cultured in Chondrocyte Growth Medium (Cat no: 411-500 Cell Applications, US).

Transfections in primary human chondrocyte cells were performed with a range of peptide: liposome: nucleic acid cargo mass ratios and N/P ratios. Transfections were performed in a 96-well plate at a density of 2000 cells per well seeded 5 days prior to transfection. Charge and mass ratio of components in the transfection complex varied; DNA and mRNA were maintained at a constant mass of 300 ng per well. The molar ratio of the cationic lipid C18 to the phospholipid DOPE was kept at 2:1 (DD 2:1), while cholesterol was added at a molar percentage of 10% of the total

lipid content. The mass ratio of closed linear DNA:lipid and mRNA:lipid was maintained at 1:3, while the chondrocyte-cell binding peptides were in some cases altered.

Transfection complexes were formed via electrostatic interaction by vigorous pipetting, before diluting in a final volume of 200 ul per well, with a DNA concentration of 1.5ng/ul. All dilutions were performed with OptiMEM reduced serum media. Controls included cells with no transfection complexes added (OptiMEM only), along with liposomes encapsulating nucleic acid cargo without peptide.

Transfection complexes were incubated for 30 min at RT before adding to cells. All conditions were performed in triplicate. Cells were incubated at 37°C for 4 hours, before replacing media with growth media. 48 h post-transfection, the cells were rinsed in PBS, before incubation in 0.05% Trypsin EDTA (Gibco) to detach the cells. 48 h post-transfection, cells were resuspended in PBS for analysis by flow cytometry using the Aligent Novocyte flow cytometer.

Figure 1 shows GFP expression and median fluorescence intensity (MFI) in primary human chondrocytes transfected with closed linear DNA:lipid:peptide nanoparticles comprising different mass ratios of lipid and peptide components. DOTMA-DOPE 2:1 with 10% cholesterol and peptide HC5 C-N showed highest GFP expression with 21% of the cells expressing GFP and MFI of 1E+06 indicating the highest transfection efficacy of these nanoparticles. DOTMA-DOPE 2:1 + 10% cholesterol with HC3 C-N peptide showed the lowest expression with 9% of the cells expressing GFP and MFI of 1E+05. Liposomes encapsulating closed linear DNA without a targeting ligand were used as controls. Nanoparticles formulated with DOTMA-DOPE 2:1 + 10% cholesterol and peptides HC3, HC4 and HC5 showed GFP expression between 12% and 20% of the cells expressing GFP.

Figure 2 shows GFP expression and median fluorescence intensity (MFI) in primary human chondrocytes transfected with closed linear DNA:lipid:peptide nanoparticles comprising different mass ratios of lipid and peptide components. C16-DOPE 2:1 with 10% cholesterol and peptide HC5 C-C showed highest GFP expression with 24% of the cells expressing GFP. Nanoparticles formulated with peptides which are not targeting the human chondrocyte cells achieved a GFP expression between 9.26% and 13.16% of the cells expressing GFP.

**Example 3** - **Transfection of primary human chondrocytes** - **Luciferase**

Peptides identified via phage display with affinity toward primary human chondrocytes, and peptides identified in the literature with desirable chondrocyte binding characteristics were synthesised via solid-phase peptide synthetic chemistry in various conformations. A sixteen-lysine tail group was attached using standard synthesis methods (amsbio, UK). Sequences are shown in Table 2.

DOTMA, DOPE and cholesterol lipids were purchased from Avanti Polar Lipids, Alabaster, Alabama, USA.

Primary human chondrocytes were cultured in were cultured in Chondrocyte Growth Medium (Cat no: 411-500 Cell Applications, US).

Transfections in primary human chondrocyte cells were performed with a range of peptide: liposome: nucleic acid cargo mass ratios and N/P ratios. Transfections were performed in a 96-well plate at a density of 2000 cells per well seeded 5 days prior to transfection. Charge and mass ratio of components in the transfection complex varied; DNA and mRNA were maintained at a constant mass of 300 ng per well. The molar ratio of the cationic lipid DOTMA to the phospholipid DOPE was kept at 2:1 (DD 2:1), while cholesterol was added at a molar percentage of 10% of the total lipid content. The mass ratio of closed linear DNA:lipid and mRNA:lipid was maintained at 1:3, while the chondrocyte-cell binding peptides were in some cases altered.

Transfection complexes were formed via electrostatic interaction by vigorous pipetting, before diluting in a final volume of 200 ul per well, with a DNA concentration of 1.5ng/ul. All dilutions were performed with OptiMEM reduced serum media. Controls included cells with no transfection complexes added (OptiMEM only), along with liposomes encapsulating nucleic acid cargo without peptide.

Transfection complexes were incubated for 30 min at RT before adding to cells. All conditions were performed in triplicate. Cells were incubated at 37°C for 4 hours, before replacing media with growth media. 48 h post-transfection for DNA and 4 h post-transfection for RNA, the cells were rinsed in PBS, before resuspension in Reporter Lysis buffer (Promega). Plates were incubated at 4°C for 20 minutes, before -80°C for 40 minutes. After thawing, luciferase activity was measured following injection of the luciferase assay substrate on the ClarioSTAR plus plate reader (BMG Labtech, Aylesbury, UK). Luciferase expression was normalised to protein content using Pierce BCA Protein Assay, with absorbance measured at 562nm. Luciferase activity was expressed as Relative Light Units per mg of protein (RLU/mg).

Figure 3 shows luciferase expression in primary human chondrocytes transfected with mRNA:lipid:peptide nanoparticles at different N/P ratios driven by the mass of the peptide. The next best performing were the nanoparticles formulated at N/P ratio of 3.48 and 7.48 with 3.2E+11 and respectively 3.3E+11. Nanoparticles were formulated with DOTMA-DOPE 2:1 + 10% cholesterol and the peptide HC5 C-N at N/P ratios between 3.48 and 8.48. The highest expression was achieved by the nanoparticles formulated at 4.48 N/P ratio with luciferase expression of 4E+11 RLU/mg protein. The formulations at N/P ratios 3.48, 5.48, 6.48, 7.48 and 8.48 showed luciferase expression ranging between 2.5E+11 RLU/mg protein and 3.5E+11 RLU/mg protein with the lowest expression of N/P ratio 8.48 (2.5E+11 RLU/mg

protein). Liposomes without targeting peptides were used as controls.

**Example 4- Ribogreen encapsulation efficiency assay**

Encapsulation efficiency (EE%) was measured using Quant-iT™ RiboGreen™ RNA Assay Kit (Invitrogen™, Thermo Fisher Scientific, Waltham, MA, USA). Briefly, mRNA was mixed with RiboGreen™ in a total volume of 100ul TE buffer (10 mM Tris-HCl, 1 mM EDTA, pH 7.5) per well of a 96-well plate and incubated for 10 minutes at RT in the absence of light. Lipid and peptide components were each diluted in a volume of 50ul TE buffer before mixing thoroughly with the mRNA-Ribogreen via pipette. Nanocomplexes were incubated for 30 minutes at RT in the absence of light. Encapsulation efficiency was measured by quantifying the fluorescence (λem = 520 nm, λex = 480 nm) of RNA encapsulated in nanoparticles vs. the fluorescence of naked mRNA complexed with Ribogreen™ reagent using a fluorimeter (ClarioStar, BMG Labtech).

Figure 4 shows the encapsulation efficacy of nanoparticles formulated with mRNA:lipid:peptide at N/P ratio 3.98 driven by the mass of peptide. The highest encapsulation efficacy was achieved by the nanoparticles formulated with the peptide HC5 L-N with 96.86% of mRNA encapsulated. All the other formulations showed encapsulation efficacy ranging from 87% to 96% of mRNA encapsulated. Liposomes encapsulating mRNA without targeting peptide and naked mRNA were used as controls.

**Example 5- Biophysical characterisation of closed linear DNA and mRNA nanoparticles**

The size in nm by Dynamic Light Scattering (DLS), zeta potential in mV by Electrophoretic Light Scattering (ELS), and concentration of particles per ml by Multi-Angle Dynamic Light Scattering (MADLS) of the closed linear DNA/mRNA-lipid-peptide nanoparticles was determined using the Zetasizer Ultra (Malvern). Complexes were prepared as before, except formulated in nuclease-free water rather than Opti-MEM. Samples containing 0.7 μg hpDNA were diluted in 1ml for analysis.

Figure 5 shows nanoparticles formulated with DOTMA-DOPE 2:1 + 10% cholesterol and various peptides encapsulating closed linear DNA. Nanoparticles were measured by dynamic light scattering (DLS) and showed sizes between 51.37nm to 100nm. The smallest nanoparticles were formulated with closed linear DNA, DOTMA-DOPE 2:1 + 10% cholesterol and peptide HC5 C-C with the average size of 51.37nm while the biggest nanoparticles were the nanoparticles formulated with closed linear DNA, DOTMA-DOPE 2:1 10% cholesterol and peptide HC5 C-N with the average sizes of 100nm. Polydispersity Index (PDI) was also measured using DLS. The nanocomplexes formulated with closed linear DNA, DOTMA-DOPE 2:1 + 10% cholesterol and peptide HC3 L-N were less polydisperse with a PDI of 0.2 while the nanocomplexes formulated with closed linear DNA, DOTMA-DOPE 2:1 10% cholesterol and peptide HC4 C-N were the most polydisperse with a PDI of 0.45 Figure 6 shows nanoparticles formulated with DOTMA-DOPE 2:1 + 10% cholesterol and various peptides encapsulating mRNA. Nanoparticles were measured by dynamic light scattering (DLS) and showed sizes between 36.65 nm to 78nm. The smallest nanoparticles were formulated with mRNA, DOTMA-DOPE 2:1 + 10% cholesterol and peptide HC4 L-N with the average size of 36.65nm while the largest nanoparticles were the nanoparticles formulated with mRNA, DOTMA-DOPE 2:1 10% cholesterol and peptide HC3 C-N with the average sizes of 78nm. Polydisperse Index (PDI) was also measured using DLS. The nanocomplexes formulated with mRNA, DOTMA-DOPE 2:1 + 10% cholesterol and peptide HC3 L-N were less polydisperse with the PDI of 0.13 while the nanocomplexes formulated with closed linear DNA, DOTMA-DOPE 2:1 10% cholesterol and peptide HC3 C-N were the most polydisperse with the PDI of 0.37.

**Example 6 - Immunogenicity and transfection efficiency of nanoparticles encapsulating closed linear DNA vs plasmid DNA**

Shown in Figure 9 is human interleukin (IL-6) expression and transfection efficiency in human chondrocytes after treatment with closed linear DNA or plasmid DNA (pDNA) encoding GFP in lipid-peptide nanoparticles. The lipid component used was DOTMA-DOPE 2:1 10% cholesterol. Closed linear DNA and pDNA nanoparticles performed similarly in terms of transfection efficiency, however, an ELISA assay showed that pDNA nanoparticles elicited higher IL-6 expression, indicating higher immunostimulatory properties as compared to closed linear DNA.

Shown in Figure 10 is human interleukin (IL-6) expression and transfection efficiency in human chondrocytes after treatment with closed linear DNA or plasmid DNA (pDNA) encoding GFP in lipid-peptide nanoparticles. The lipid component used was ALC-DOPE 10% cholesterol. Closed linear DNA and pDNA nanoparticles performed similarly in terms of transfection efficiency, however, an ELISA assay showed that pDNA nanoparticles elicited higher IL-6 expression, indicating higher immunostimulatory properties as compared to closed linear DNA.

Nanoparticles were formulated manually by pipetting following a mass ratios of 1:5 (cargo:liposomes) with a peptide-driven N/P ratio 6. Firstly, the components - cargos, liposomes and peptide (KKKKKKKKKKKKKKKKGARVRRCRLDPTSYLRTFWC; SEQ ID NO:23) were suspended in ultra pure water in volumes of 60ul, 60ul and 80ul, respectively. Components were mixed following the order - liposomes to peptide; cargos to liposomes+peptide complexes. Nanoparticles were allowed to incubate for 30 minutes at room temperature. All samples were diluted in low serum media before transferring onto cells. Human chondrocytes were transfected with 300ng of closed linear DNA or pDNA nanoparticles in 96-well plate format. The microplate was centrifuged at 12,000 rpm for 5 minutes and incubated at 37°C for 4 hours before replenishing full growth media. 24 hours post-transfection, the supernatant was collected and preserved at -20°C and a sandwich ELISA (R&D Systems) was used to measure IL-6 expression 24h hours post-transfection, while 48 hours post-transfection, the cells were trypsinised and prepared for analysis by flow cytometry.

ELISA method: In brief, Capture Antibody (Mouse Anti-Human IL-6) was plated on a 96-well plate at the required working concentration and left overnight at RT. The plate was washed with Wash Buffer (0.05% PBS-Tween-20) and then blocked with 3% BSA in PBS for a minimum of 1 hour. The plate was washed with Wash Buffer before adding samples or standards diluted in Reagent Diluent (1% BSA in PBS) for 2 hours at RT. The plate was washed again, before the Detection Antibody (Biotinylated Goat Anti-Human IL-6) was added at the recommended working concentration for a further 2 hours. After washing the plate, Streptavidin-HRPB was added for 20 minutes, protected from light. A final wash step was performed before adding the Substrate Solution (1:1 mixture of $H_2O_2$:Tetramethylbenzidine) and incubating for 20 minutes, protected from light. The assay was stopped by the addition of Stop Solution (2N $H_2SO_4$) to the wells. The plate was read using the Clariostar (BMG LabTech) at a wavelength of 540-570 nm. IL-6 concentration was determined using the interpolation software in GraphPad Prism (version 9.3.1) and normalized to the un-transfected conditions.

## Claims

**1.** A nanoparticle comprising:

(a) a nucleic acid cargo;
(b) a lipid component; and
(c) a targeting peptide comprising a chondrocyte targeting sequence and a nucleic acid-binding cationic component.

**2.** The nanoparticle of claim 1, wherein the nanoparticle is a non-viral transfection complex.

**3.** The nanoparticle of claim 1 or claim 2, wherein the nanoparticle is a self-assembled nanoparticle.

**4.** The nanoparticle of any one of claims 1-3, wherein the nucleic acid cargo is:

a. a closed linear DNA molecule;
b. a linear DNA molecule comprising one or more nuclease-resistant nucleotides;
c. a partially closed linear DNA molecule comprising a double-stranded DNA portion that is closed at a first end and open at a second end, wherein the partially closed linear DNA molecule comprises one or more nuclease-resistant nucleotides in an open end region adjacent to the second end; or
d. mRNA.

**5.** The nanoparticle of any one of claims 1-4, wherein the chondrocyte targeting sequence comprises:

(a) SEQ ID NO: 1 or a variant thereof comprising one or more conservative amino acid substitutions;
(b) SEQ ID NO: 1 or a variant thereof comprising one or more conservative amino acid substitutions;
(c) SEQ ID NO: 3 or a variant thereof comprising one or more conservative amino acid substitutions;
(d) SEQ ID NO: 4 or a variant thereof comprising one or more conservative amino acid substitutions; or
(e) SEQ ID NO: 5 or a variant thereof comprising one or more conservative amino acid substitutions.

**6.** The nanoparticle of any one of claims 1-5, wherein the chondrocyte targeting peptide comprises the sequence of any one of SEQ ID NO: 6-37, or a variant thereof comprising one or more conservative amino acid substitutions.

**7.** The nanoparticle of any one of claims 1-6, wherein the Nitrogen/Phosphate (N/P) ratio of the nanoparticle is between 3.0-9.0, optionally between 4.0-5.0.

**8.** A targeting peptide comprising:

(a) a chondrocyte targeting sequence; and
(b) a nucleic acid-binding cationic component.

**9.** The targeting peptide of claim 8, wherein the targeting peptide further comprises a linker, optionally wherein the linker is cleavable or non-cleavable.

**10.** A targeting peptide comprising the sequence of any one of SEQ ID NO: 6-37, or a variant thereof comprising one or more conservative amino acid substitutions.

**11.** A pharmaceutical composition comprising the nanoparticle of any one of claims 1-7, or the targeting peptide of any one of claims 8-10, and a pharmaceutically suitable carrier.

**12.** The nanoparticle of any one of claims 1-7, the targeting peptide of any one of claims 8-10, or the pharmaceutical composition of claim 11, for use in therapy.

**13.** The nanoparticle of any one of claims 1-7, the targeting peptide of any one of claims 8-10, or the pharmaceutical composition of claim 11, for use in the treatment of a joint disease or disorder.

**14.** A method for transfecting a cell comprising:

(a) contacting a cell with the nanoparticle of any one of claims 1-7; and
(b) transfecting the nanoparticle to the cell.

**15.** A method for producing the nanoparticle of any one of claims 1-7, wherein the method comprises:

(a) contacting the nucleic acid cargo with the lipid component and the targeting peptide comprising the chondrocyte targeting sequence and the nucleic acid-binding cationic component; and
(b) formulating the nanoparticle.

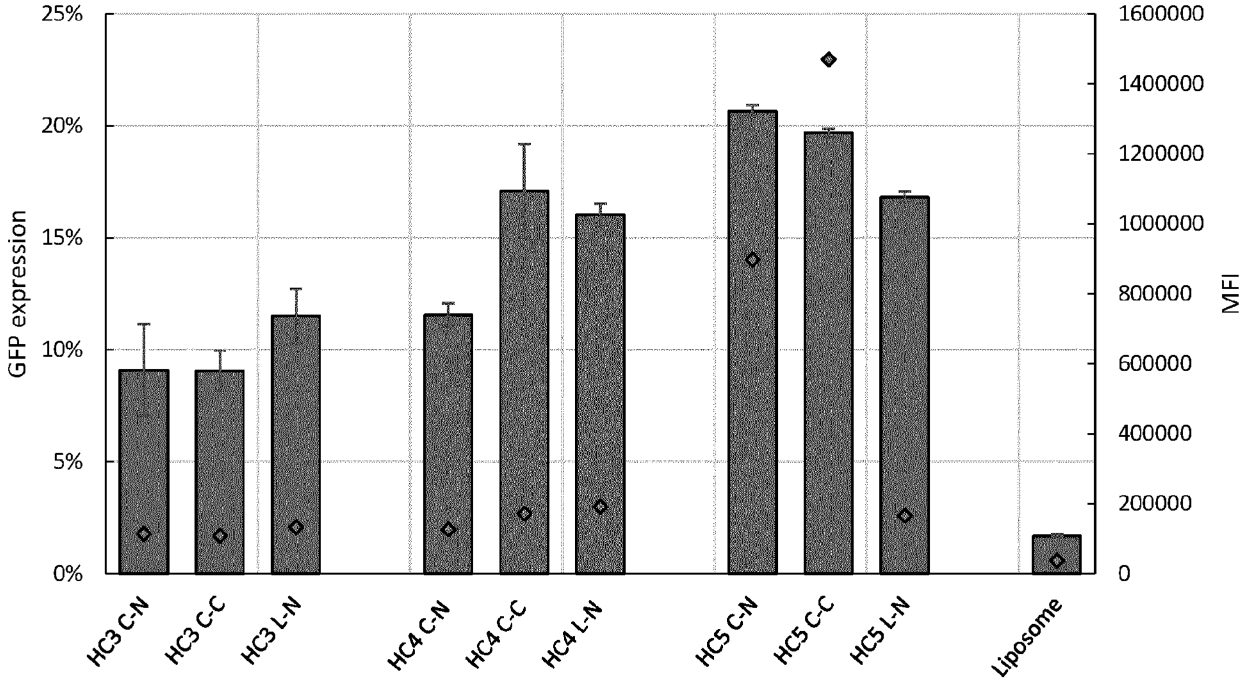
25%
20%
15%
10%
5%
0%
GFP expression
1600000
1400000
1200000
1000000
800000
600000
400000
200000
0
MFI
HC3 C-N
HC3 C-C
HC3 L-N
HC4 C-N
HC4 C-C
HC4 L-N
HC5 C-N
HC5 C-C
HC5 L-N
Liposome

Fig. 1

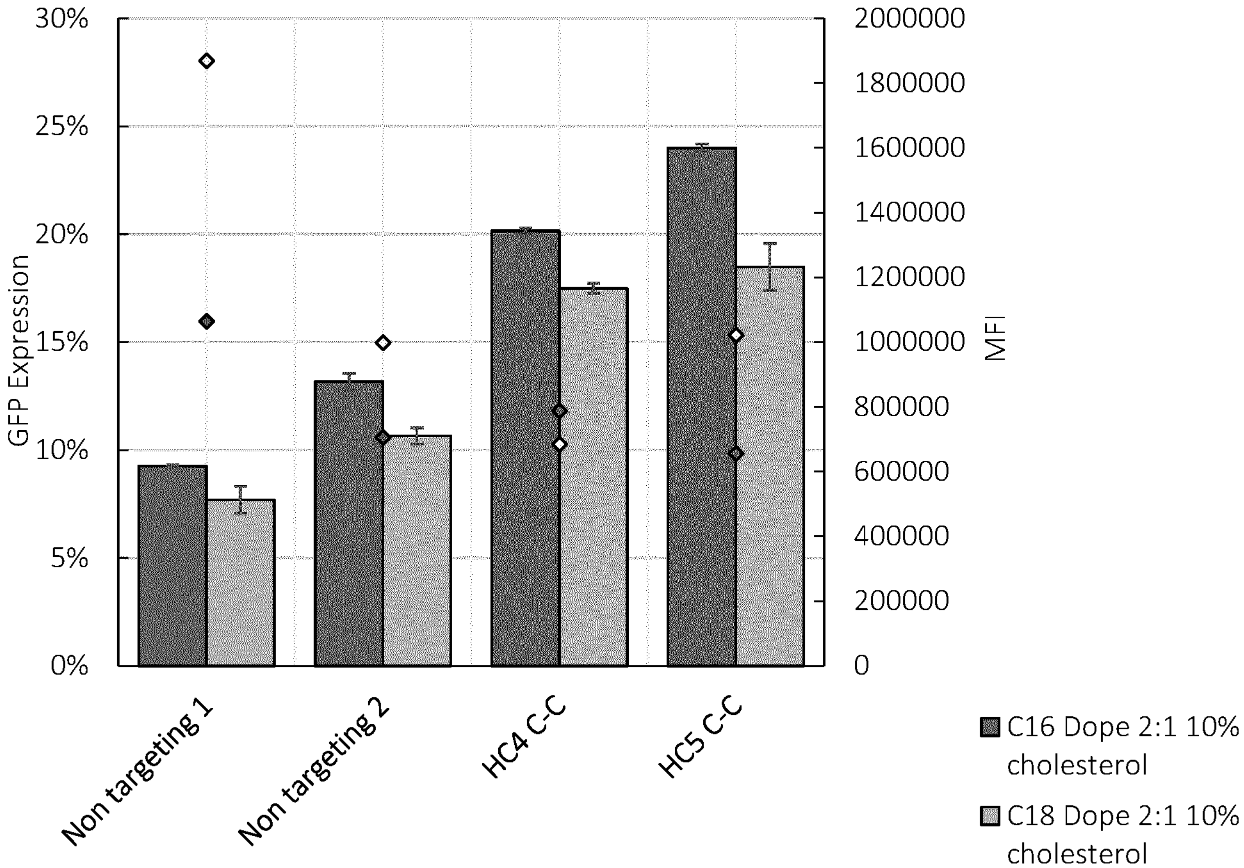
30%
25%
20%
15%
10%
5%
0%
GFP Expression
2000000
1800000
1600000
1400000
1200000
1000000
800000
600000
400000
200000
0
MFI
Non targeting 1
Non targeting 2
HC4 C-C
HC5 C-C
C16 Dope 2:1 10% cholesterol
C18 Dope 2:1 10% cholesterol

Fig. 2

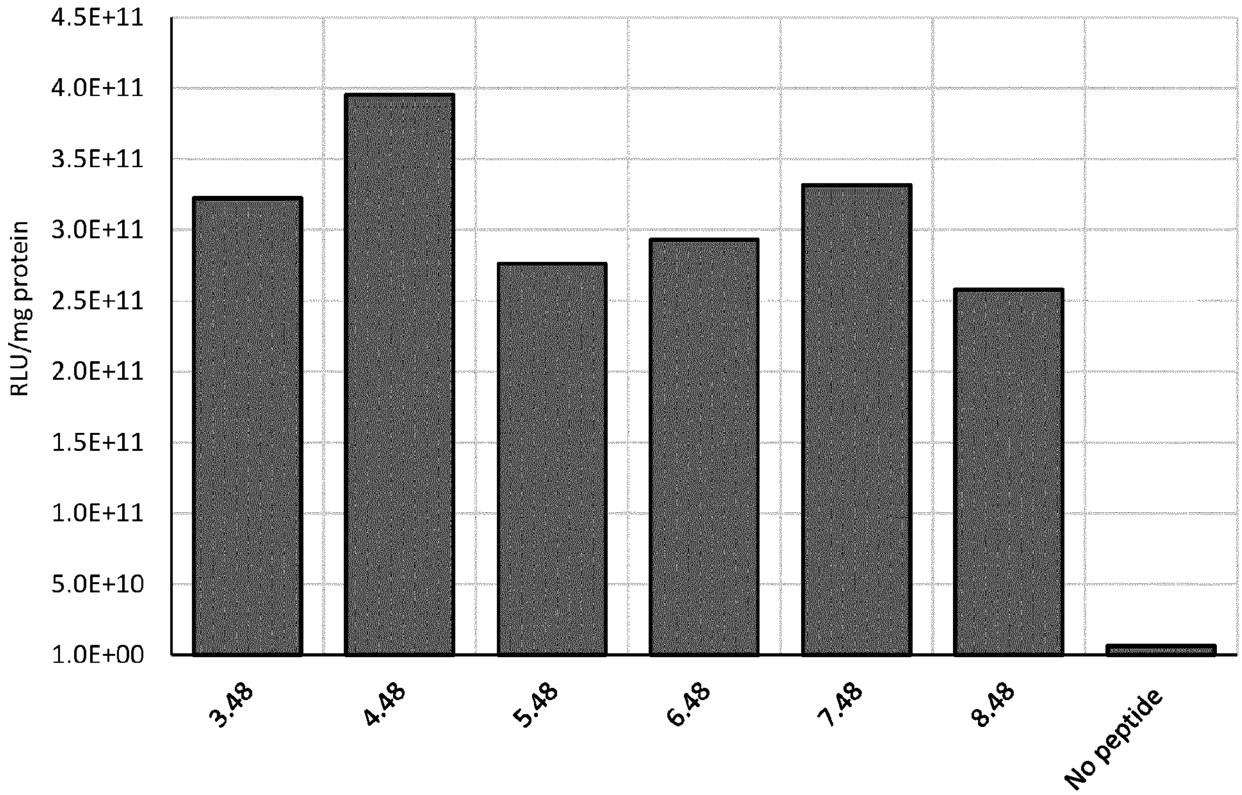
4.5E+11
4.0E+11
3.5E+11
3.0E+11
2.5E+11
2.0E+11
1.5E+11
1.0E+11
5.0E+10
1.0E+00
RLU/mg protein
3.48
4.48
5.48
6.48
7.48
8.48
No peptide

Fig. 3

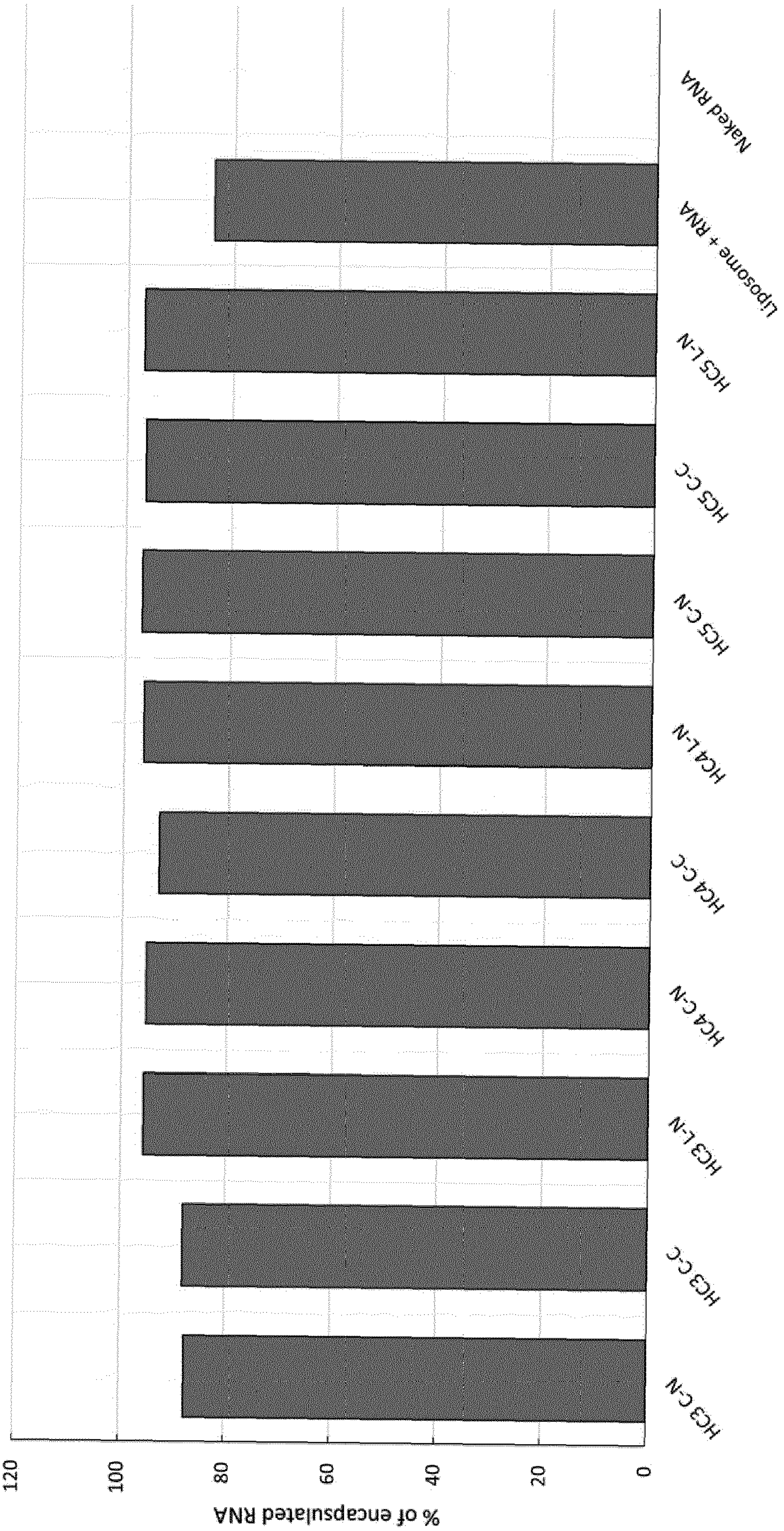
Naked RNA
Liposome + RNA
HC5 L-N
HC5 C-C
HC5 C-N
HC4 L-N
HC4 C-C
HC4 C-N
HC3 L-N
HC3 C-C
HC3 C-N
120
100
80
60
40
20
0
% of encapsulated RNA

Fig. 4

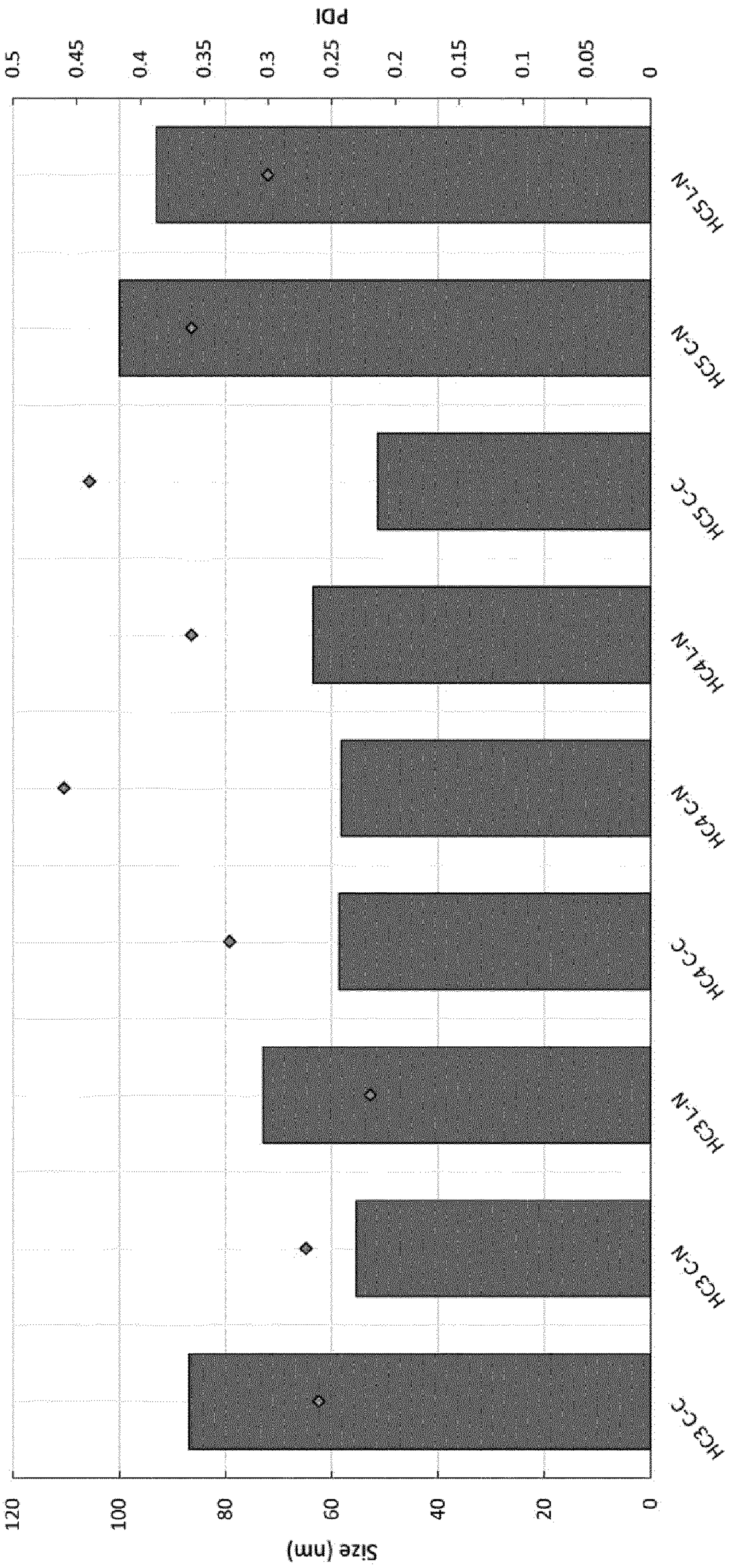
PDI
0.5
0.45
0.4
0.35
0.3
0.25
0.2
0.15
0.1
0.05
0
Size (nm)
120
100
80
60
40
20
0
HC5 L-N
HC5 C-N
HC5 C-C
HC4 L-N
HC4 C-N
HC4 C-C
HC3 L-N
HC3 C-N
HC3 C-C

Fig. 5

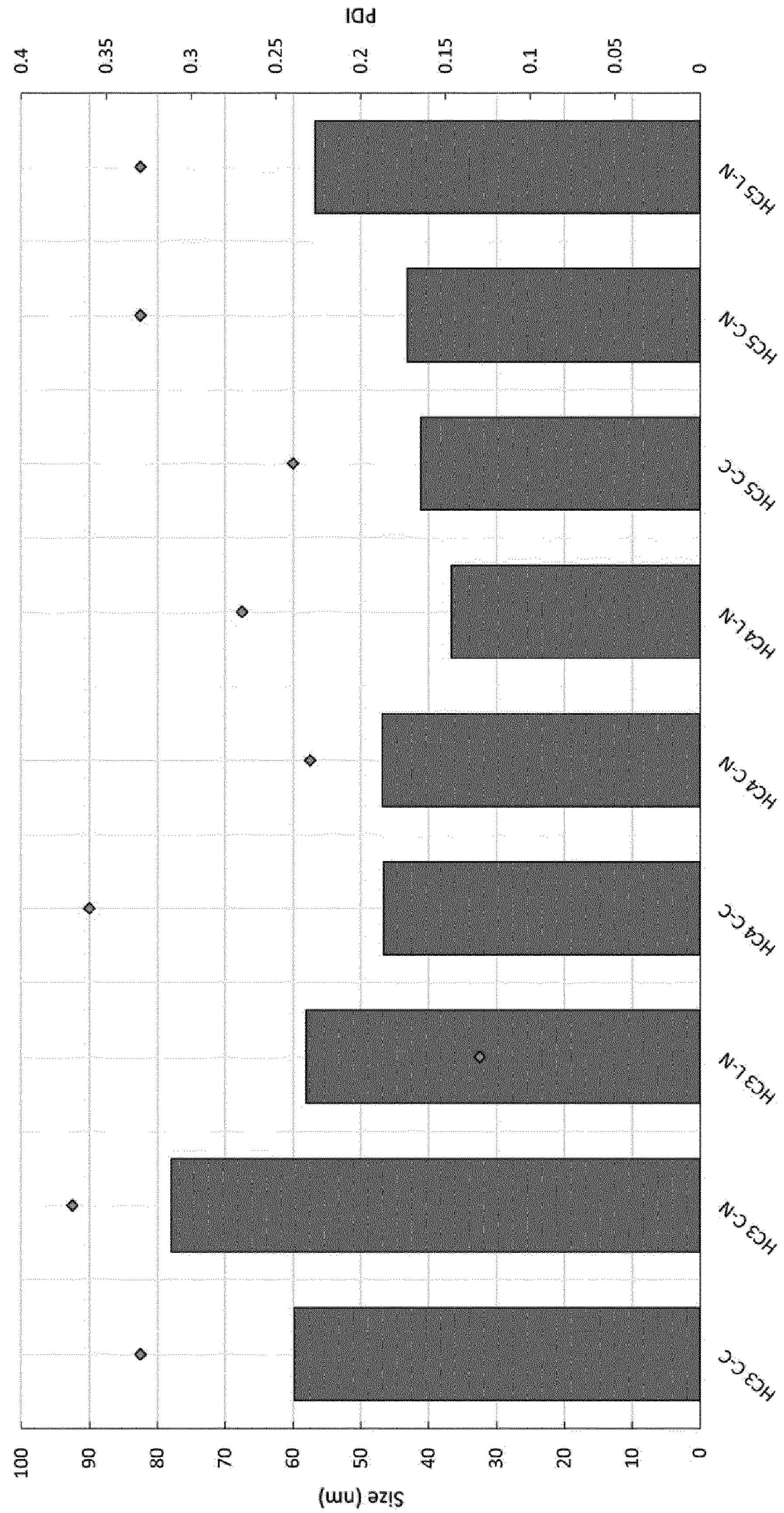
PDI
0.4
0.35
0.3
0.25
0.2
0.15
0.1
0.05
0
Size (nm)
100
90
80
70
60
50
40
30
20
10
0
HC3 C-C
HC3 C-N
HC3 L-N
HC4 C-C
HC4 C-N
HC4 L-N
HC5 C-C
HC5 C-N
HC5 L-N

Fig. 6

| Peptide Name | Peptide sequence | DNA binding domain | Targeting sequence |
|---|---|---|---|
| HC1 L-N | KKKKKKKKKKKKKKKKGASTLHQKL | K16 | STLHQKL |
| HC1 C-N | KKKKKKKKKKKKKKKKGACSTLHQKLC | K16 | STLHQKL |
| HC1 C-C | KKKKKKKKKKKKKKKKGARVRRCSTLHQKLC | K16 | STLHQKL |
| HC2 L-N | KKKKKKKKKKKKKKKKGASSLHQKL | K16 | SSLHQKL |
| HC2 C-N | KKKKKKKKKKKKKKKKGACSSLHQKLC | K16 | SSLHQKL |
| HC2 C-C | KKKKKKKKKKKKKKKKGARVRRCSSLHQKLC | K16 | SSLHQKL |
| HC3 C-N | KKKKKKKKKKKKKKKKGACRLDPTSYLRTFWC | K16 | RLDPTSYLRTFW |
| HC3 C-C | KKKKKKKKKKKKKKKKGARVRRCRLDPTSYLRTFWC | K16 | RLDPTSYLRTFW |
| HC3 L-N | KKKKKKKKKKKKKKKKGARLDPTSYLRTFW | K16 | RLDPTSYLRTFW |
| HC4 C-N | KKKKKKKKKKKKKKKKGACHDSQLEALIKFMC | K16 | HDSQLEALIKFM |
| HC4 C-C | KKKKKKKKKKKKKKKKRVRRGACHDSQLEALIKFMC | K16 | HDSQLEALIKFM |
| HC4 L-N | KKKKKKKKKKKKKKKKGAHDSQLEALIKFM | K16 | HDSQLEALIKFM |
| HC5 C-N | KKKKKKKKKKKKKKKKGACDWRVIIPPRPSAC | K16 | DWRVIIPPRPSA |
| HC5 C-C | KKKKKKKKKKKKKKKKRVRRCDWRVIIPPRPSAC | K16 | DWRVIIPPRPSA |
| HC5 L-N | KKKKKKKKKKKKKKKKGADWRVIIPPRPSA | K16 | DWRVIIPPRPSA |

Fig. 7

DOTMA

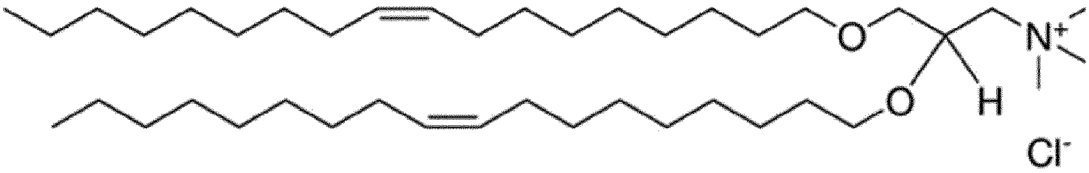

DMG-PEG

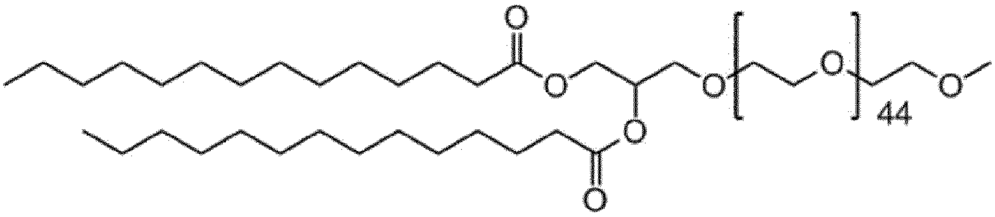

DOPE

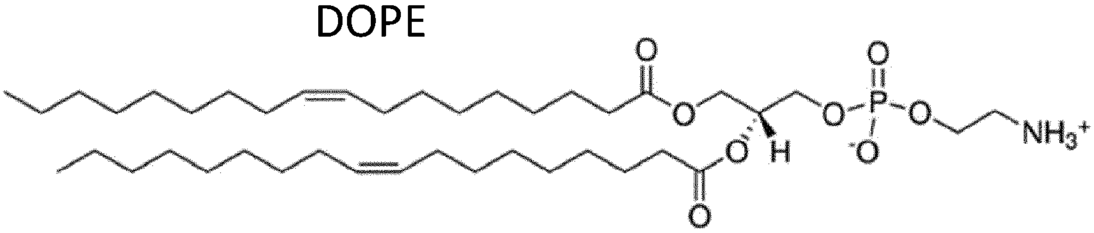

cholesterol

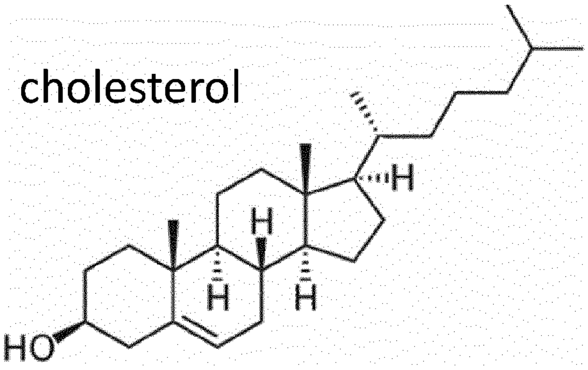

ALC-0315

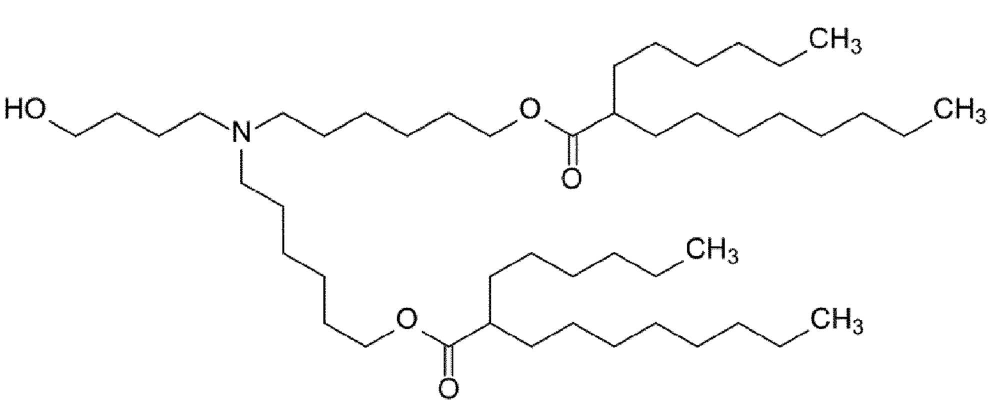

Fig. 8

Fig. 9

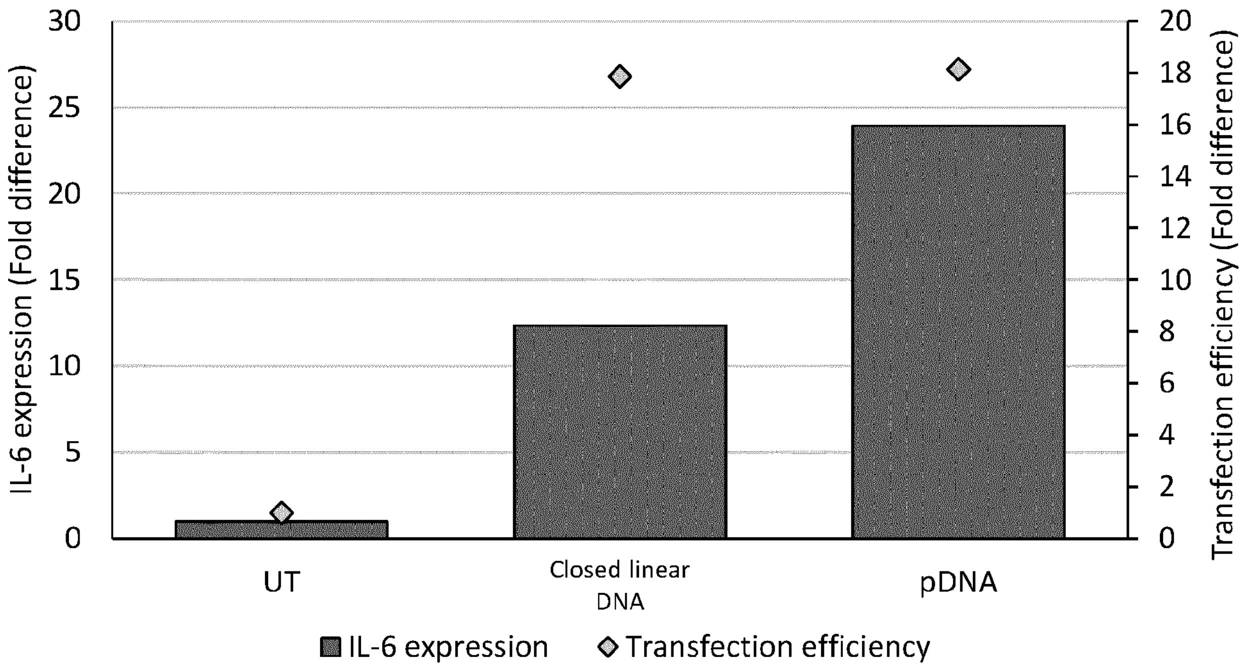
IL-6 expression (Fold difference)
Transfection efficiency (Fold difference)
0
5
10
15
20
25
30
0
2
4
6
8
10
12
14
16
18
20
UT
Closed linear DNA
pDNA
IL-6 expression
Transfection efficiency

Fig. 10

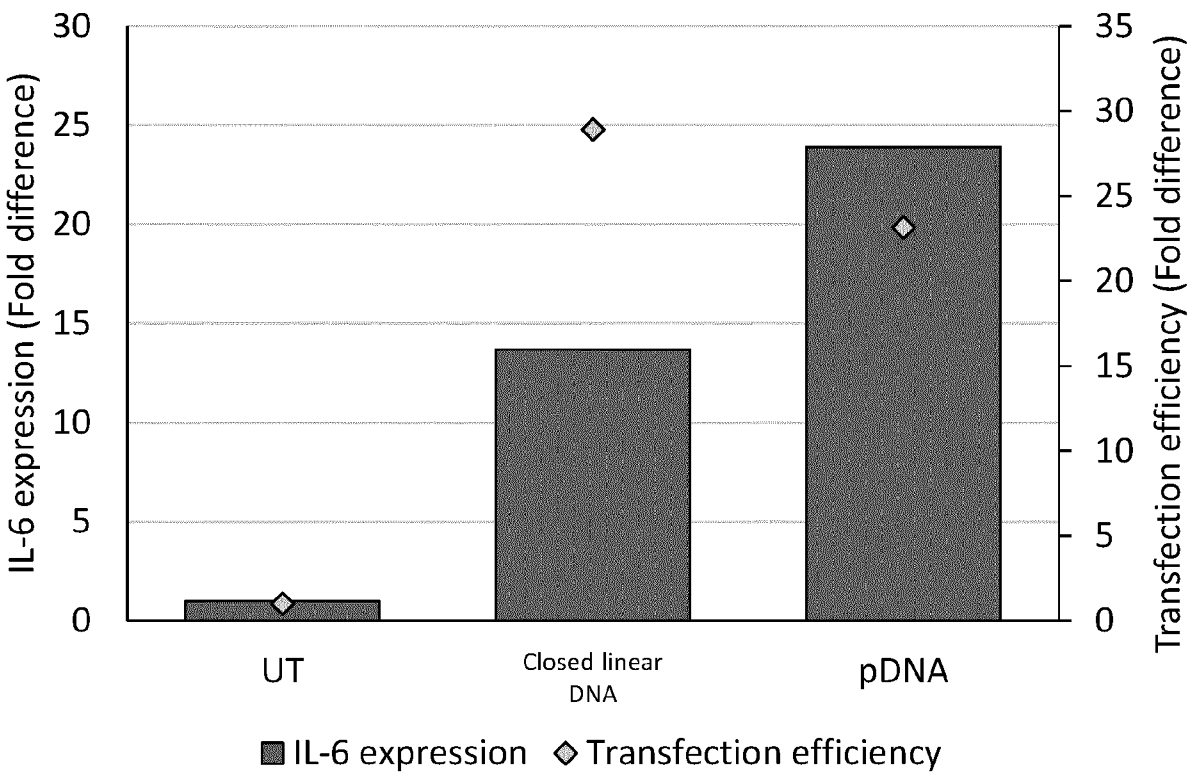
IL-6 expression (Fold difference)
Transfection efficiency (Fold difference)
0
5
10
15
20
25
30
0
5
10
15
20
25
30
35
UT
Closed linear DNA
pDNA
IL-6 expression
Transfection efficiency

Europäisches Patentamt
European Patent Office
Office européen des brevets

# EUROPEAN SEARCH REPORT

Application Number
EP 22 38 2731

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/133893 A1 (CHEN YUPENG [US] ET AL) 5 May 2022 (2022-05-05) * RN 2769756-83-2; page 30, paragraph 152 * | 1-5, 8-12,15 | INV. A61K47/66 A61K47/69 A61P19/02 |
| X | YANBIN PI ET AL: "Targeted delivery of non-viral vectors to cartilage invivo using a chondrocyte-homing peptide identified by phage display", BIOMATERIALS, ELSEVIER, AMSTERDAM, NL, vol. 32, no. 26, 5 May 2011 (2011-05-05), pages 6324-6332, XP028097288, ISSN: 0142-9612, DOI: 10.1016/J.BIOMATERIALS.2011.05.017 [retrieved on 2011-05-11] * CAS RN 1258783-03-7 (SEQ: DWRVIIPPRPSA - seq ID 5 of current application) * | 8,9, 11-13 | |
| X | OUYANG ZHENGXIAO ET AL: "Targeted delivery of hesperetin to cartilage attenuates osteoarthritis by bimodal imaging with Gd2(CO3)3@PDA nanoparticles via TLR-2/NF-[kappa]B/Akt signaling", BIOMATERIALS, vol. 205, 18 March 2019 (2019-03-18), pages 50-63, XP085644763, ISSN: 0142-9612, DOI: 10.1016/J.BIOMATERIALS.2019.03.018 * CAS RN 1258783-03-7 (SEQ: DWRVIIPPRPSA - seq ID 5 of current application) * | 8,9, 11-13 | TECHNICAL FIELDS SEARCHED (IPC): A61K |
| X | CN 101 891 803 A (UNIV PEKING 3RD HOSPITAL) 24 November 2010 (2010-11-24) * CAS RN 1258783-03-7 (SEQ: DWRVIIPPRPSA - seq ID 5 of current application); claim 1 * | 8,9, 11-13 | |

-/--

The present search report has been drawn up for all claims

3

| Place of search | Date of completion of the search | Examiner |
|---|---|---|
| The Hague | 12 January 2023 | Langer, Miren |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 38 2731

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HE KONGFANG ET AL: "Intra-articular Injection of Lornoxicam and MicroRNA-140 Co-loaded Cationic Liposomes Enhanced the Therapeutic Treatment of Experimental Osteoarthritis", AAPS PHARMSCITECH, SPRINGER INTERNATIONAL PUBLISHING, CHAM, vol. 23, no. 1, 3 December 2021 (2021-12-03), XP037716232, DOI: 10.1208/S12249-021-02149-W [retrieved on 2021-12-03] * abstract * ----- | 1-15 | |
| Y | YOUNAS AYESHA ET AL: "Novel approaches of the nanotechnology-based drug delivery systems for knee joint injuries: A review", INTERNATIONAL JOURNAL OF PHARMACEUTICS, ELSEVIER, NL, vol. 608, 25 August 2021 (2021-08-25), XP086820551, ISSN: 0378-5173, DOI: 10.1016/J.IJPHARM.2021.121051 [retrieved on 2021-08-25] * page 6; table 3 * * the whole document * ----- | 1-15 | TECHNICAL FIELDS SEARCHED (IPC) |
| Y | CN 109 966 506 A (2ND PEOPLES HOSPITAL SHENZHEN) 5 July 2019 (2019-07-05) * RN 1258783-03-7, (SEQ: DWRVIIPPRPSA - seq ID 5 of current application); page 2 * ----- | 1-15 | |
| | -/-- | | |

The present search report has been drawn up for all claims

| Place of search | Date of completion of the search | Examiner |
|---|---|---|
| The Hague | 12 January 2023 | Langer, Miren |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 22 38 2731

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CRYSTAL S.F. CHEUNG ET AL: "Identification of chondrocyte-binding peptides by phage display : IDENTIFICATION OF CHONDROCYTE-BINDING PEPTIDES", JOURNAL OF ORTHOPAEDIC RESEARCH, vol. 31, no. 7, 1 July 2013 (2013-07-01), pages 1053-1058, XP055636815, US ISSN: 0736-0266, DOI: 10.1002/jor.22325 CAS RN 1612883-39-2, CAS RN 1612883-40-5: SEQ ID 3 and 4 of current application SEQ RLDPTSYLRTFW, HDSQLEALIKFM; * abstract * ----- | 1-15 | |
| A | PI Y ET AL: "Intra-articular delivery of anti-Hif-2[alpha] siRNA by chondrocyte-homing nanoparticles to prevent cartilage degeneration in arthritic mice", GENE THERAPY, NATURE PUBLISHING GROUP, LONDON, GB, vol. 22, no. 6, 16 April 2015 (2015-04-16), pages 439-448, XP037770997, ISSN: 0969-7128, DOI: 10.1038/GT.2015.16 [retrieved on 2015-04-16] ----- | 1-15 | TECHNICAL FIELDS SEARCHED (IPC) |
| A | BOTTINI MASSIMO ET AL: "Nanodrugs to target articular cartilage: An emerging platform for osteoarthritis therapy", NANOMEDICINE: NANOTECHNOLOGY, BIOLOGY, AND MEDICINE, vol. 12, no. 2, 1 January 2016 (2016-01-01), pages 255-268, XP029459502, ISSN: 1549-9634, DOI: 10.1016/J.NANO.2015.09.013 ----- | 1-15 | |

The present search report has been drawn up for all claims

| Place of search | Date of completion of the search | Examiner |
|---|---|---|
| The Hague | 12 January 2023 | Langer, Miren |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

# ANNEX TO THE EUROPEAN SEARCH REPORT ON EUROPEAN PATENT APPLICATION NO. EP 22 38 2731

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2023

| Patent document cited in search report | Publication date | Patent family member(s) | Publication date |
|---|---|---|---|
| US 2022133893 A1 | 05-05-2022 | NONE | |
| CN 101891803 A | 24-11-2010 | NONE | |
| CN 109966506 A | 05-07-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02072616 A2

### Non-patent literature cited in the description


- **DREIER.** Hypertrophic differentiation of chondrocytes in osteoarthritis: the developmental aspect of degenerative joint disorders. *Arthritis Research & Therapy,* 2010, vol. 12 (5), 1-11
- **TSENG et al.** Dual Role of Chondrocytes in Rheumatoid Arthritis: The Chicken and the Egg. *International Journal of Molecular Sciences,* 2020, vol. 21 (3), 1071
- **HAMPE et al.** Mucopolysaccharidosis Type I: A Review of the Natural History and Molecular Pathology. *Cells,* 2020, vol. 9 (8), 1838
- **CUCCHIARINI et al.** New trends in articular cartilage repair. *Journal of experimental orthopaedics,* 2015, vol. 2 (1), 1-8
- **EVANS et al.** Gene therapeutic approaches - transfer in vivo. *Advanced drug delivery reviews,* 2006, vol. 58 (2), 243-258
- **FELGNER et al.** Lipofection: a highly efficient, lipid-mediated DNA-transfection procedure. *Proceedings of the National Academy of Sciences,* 1987, vol. 84.21, 7413-7417